# EUROPEAN PATENT SPECIFICATION

(11) **EP 1 016 381 B1**
(45) Date of publication and mention of the grant of the patent: **10.12.2003**
(21) Application number: 98600018.0
(22) Date of filing: 23.11.1998
(51) Int. Cl.: A61B 17/64

(54) **External fixation system with telescopic pin or arm holding unit**
Äusserliche Befestigung mit teleskopischem Halter für einen Stift oder einen Arm
Fixateur externe avec monture télescopique pour une broche ou pour un bras

(43) Date of publication of application: 05.07.2000
(73) Proprietor: Kourtis, Hraklis, 41222 Larisa (GR); Kourtis, Lambros, 41222 Larisa (GR); Konstantinos, Malizos, Ioannina (GR)
(72) Inventor: Kourtis, Hraklis, 41222 Larisa (GR); Kourtis, Lambros, 41222 Larisa (GR); Konstantinos, Malizos, Ioannina (GR)
(74) Representative: Panagiotidou, Effimia

(56) References cited:
- WO-A-91/11149
- WO-A-92/02185
- WO-A-98/12975

## Description

The invention belongs to the field of Orthopedic Surgery, Traumatology and Distraction Osteogenesis and consists of a mechanism used in an external fixation system, with a wide range of application in all therapeutic surgical practices, which today use systems of external fixation. Thus, the invention may be used in cases of bone fracture restoration and in cases of correction of cranium, jaw, limb bones, etc. dysplasias, which require distraction osteogenesis.

The term Orthopedics is used to encompass the practices of diagnosis, conservative and surgical therapy, prevention and correction of affections of the musculoskeletal system as well as of the peripheral vessels and nerves surrounding it.

The term Traumatology is used to encompass the practice of prevention, collection, reception of the wounded, life support functions, and the healing of the wounded anatomical parts using all appropriate means and methods.

Orthopaedics and Traumatology are adjacent and widely overlapping fields of knowledge with regard to their field and range of application on the musculoskeletal system and its supporting peripheral vessels and nerves.

The term Distraction Osteogenesis is used to define the practice of bones elongation and their corresponding limbs or the transfer of osteal parts, in order to bridge osteal gaps.
Modem state-of-the-art techniques in the setting of bone fractures using orthopaedics and traumatology and in the correction of dysplasias using distraction osteogenesis include the use of systems of both internal and external fixation.

The systems of external fixation are used wherever a temporary practice of immobilisation is required, in order to promote the healing process in broken bones and the adjoining tissues. They are also used in the temporary immobilisation of joints.

In contrast to the technique of internal fixation where broken bones are held in place by artificial implants, placed under the tissues and totally covered by them, the technique of external fixation uses systems which are placed outside the human body and are connected to the broken bones with special pins applied wedged or screwed on the bones, with the help of various mechanisms.

The systems of external fixation, thanks to the bracing mechanisms they consist of, comprise a multifunctional and flexible system of bone bracing, put in place under anaesthesia, just like the systems of internal fixation. Over the systems of internal fixation, they display, first of all, the serious advantage of giving the surgeon the ability to intervene at any time during the porosis of the fracture they brace and to alter, if he deems it necessary, the arrangement and biomechanical qualities of the occasional system, which is something that cannot be done with the systems of internal fixation, where once the surgeon places the system is then deprived of any means of altering its arrangement or its biomechanical qualities.

Thus, the use of systems of external fixation enables the system to become more resilient or less resilient, to correct the position of the bones should it prove unsatisfactory, to exercise contraction or distraction, as well as to increase the 'rigidity' of the system-bone system at any time the surgeon deems it necessary, whether during the operation or at some point later in time. In contrast, the use of systems of internal fixation only allows the increase of the 'rigidity' of the system-bone system, and that only at the point of its placement in the patient's body and absolutely at no time later on.
A further advantage of the systems of external fixation is the fact that if during the time of tissue healing ensue conditions requiring rearrangement of the fixation, this can be done at any time and without encumbrance to the patient or his wound. In contrast, any rearrangement of the systems of internal fixation requires a new open operation on the patient.
Another advantage displayed by the systems of external fixation over the systems of internal fixation is that their use does not require placement of implants in an open wound which had originally been infected, thus dramatically decreasing the possibility of developing inflammation, a lurking danger when systems of internal fixation are used.

A further advantage of the systems of external fixation over the systems of internal fixation is that with their use further damage to the soft tissues is avoided, as external fixation consists of placing pins far from the injured tissues, while the use of systems of internal fixation inflict further damage to the soft tissues, since at the time of their placement they come into contact with part of the surface of the damaged tissues.

With regard to the state-of-the-art techniques proposed to day for systems of external fixation, appropriate bracing mechanisms are used, which are either based on circular frames and carry clamps that are placed on the bone to be set or distracted or rest on a connecting rod and carry pins, which are placed on the bone to be set or distracted.

More particularly, most holding mechanisms of pins which rest on connecting rods display the disadvantage that the placing of the pins must necessarily be set at the manufacturer's predesignated and preset particular holdings of the holding mechanisms. Thus, after the placement of one of the pins in the body of the patient, the placement of the next pins must necessarily be set at the positions indicated by the manufacturer. This, however, might not be feasible on account of anatomical or pathological factors in the particular position, such as a nerve, an inflammation, etc. In addition, the pin may be wrongly placed, that is, having a wrong inclination or being in the wrong position during surgery. To avoid such an incidence, the surgeon is forced to bend the pin in order to align it with the rest of the pins so as to manage, later, to apply on them the holding mechanism. As a result, the biomechanical strength of the whole system decreases and there is a risk of a pin failure or even of a stultification of the whole system leading to a reinstalling of a new one, with catastrophic consequences to the patient. Also, the duration of the surgical operation is extended, which requires a lot more time of narcosis.

Another disadvantage of the systems which carry holding mechanisms with manufacturer's predesignated and preset positions for the holding of pins is that the mandatory placement of the said pins in these particular preset positions results in the decrease of the general spatial flexibility of the system, making it necessary, in order to succeed in their proper placement, to need the use of a special auxiliary manufacturer's template which will indicate to the surgeon the proper position of placing the pins on the patient's body. This disadvantage renders these systems extremely unwieldy, not to mention the increase in the duration of the operation and the extra distress to the patient. Finally, there incurs an increase in the final production cost of the system.

Certain other mechanisms which support pins resting on connecting rods display the further disadvantage of holding the pins in places which are always close to the rod and in a fixed and preset by the manufacturer distance from the rod and, as a consequence, the bone parts to be restored are exposed to bending and shearing forces with an orientation perpendicular to the level defined by the pins, thus creating unfavourable conditions for the porosis of the fracture. Moreover, the risk of a failure, either of the pin or the mechanism itself, becomes greater.

Finally, another serious disadvantage displayed by most holding mechanisms of pins in use today in systems of external fixation with an extending arm is that in cases of distraction or bone healing where it is preferable to have the axis of the bone to be set parallel to that of the rod, this cannot be achieved as most mechanisms are so designed that the position of the rod is dictated by the positions of the pins. For exactly the same reason, neither can the placement of the rod in a different orientation from the axis of the bone to be set be achieved, when preferable, in order to avoid varus, valgus, etc.
Because of all the above reasons, the use of most holding mechanisms of pins renders the placement of the whole system inconvenient for the surgeon, sometimes inflicting disastrous harm on the patient.

However, there exist certain systems which allow for a way of distancing from the holding rod by using an extending arm on which rest the holding mechanisms of the pins. Nevertheless, such mechanisms display the very serious disadvantage of using friction couplings instead of rigid ones, which results in making the system very weak from a biomechanical point of view. Moreover, the use of fixed extending arms in these mechanisms makes the whole system heavier, more complex and having a higher production cost.

This is the case for instance, with WO 9111149 A, which exhibits the features which are listed in the preamble of claim 1, where the friction couplings between telescopic arm (17) and opening (37) and between pin holding mechanism (19) and telescopic arm (39) are vulnerable to chronic alternate loads, resulting thus disastrous consequences for the bone healing process. That is, the key - keyway coupling, used in this invention, in combination with the radial toothed surface reassures that a solid joint fixation is obtained.
Moreover, in WO 91 11149 A, numbers (24), (32), (33), (41), (44) represent five screws that have to be tightened in order for the fixation of the pins, while in the introduced device, only 3 screws are needed to be tightened, reassuring thus that the procedure is shorter. In addition, WO 9111149 A suggests a degree of freedom resulting the rotation of part (34) around axis of outlet (38), while in the introduced device this same degree of freedom is offered much closer to the pin. Consequently, translation of the pin is minimum when rotated, while in WO 9111149 A, rotation leads to translation of the pin. So, these two inventions have fundamental differences in their concepts.

The present invention refers to a telescopic unit of orientating and immobilizing of one pin or even of a second pin or even an extending arm and to a system which carries such units, which, like any other external fixation system, is put in place while the patient is under anaesthesia. The present system provides the possibility of transposition of the unit along the rod, rotation of the unit around the rod, rotation of the telescopic mechanism of the telescopic unit, inside its clamp, axial transposition of the telescopic mechanism of the telescopic unit inside and outside its holding clamp and rotation of the holding mechanism of the pin. For the more correct use of the system and the telescopic units it consists of, the surgeon places in the beginning the pins on the points of the bone to be restored he considers suitable. Having loosened initially all the tightening parts of the clamping mechanisms, he places and hooks around each one of the pins the special holding jaw available in each telescopic unit. Thanks to the novelty of the special holding jaw, the pin can be hooked and unhooked with a single move. Also, thanks to the elastic agent used in almost all the positions of the rigid couplings of each telescopic unit, the initial places where the surgeon sets the parts of the telescopic unit remain temporarily fixed. Then the necessary moves are made by the surgeon in order to select, from the wide range of choices provided by the telescopic mechanism of each telescopic unit, the most suitable final position of the telescopic unit, without however completely immobilising it yet. Following that, the special holding jaws of the rest of the telescopic units are placed, in the same way, on the rest of the pins and the most suitable place of the rest of the units is chosen by the surgeon. Then, the surgeon decides on the position of the rod in relation to the bone and immediately afterwards tightens, in any order he prefers in each unit, the screw of the holding jaws of the pin, the immobilising screw of the telescopic mechanism and the screw of the clamp of each unit, thus completely immobilising all the units on the holding rod and, consequently, the whole system. Each unit, as well as all the mechanisms that make up each unit, is capable of being placed and fixed on the rod according to the position and the sequence of the user's choice. The system with these units is used for the healing of fractures or osteal deformities through independent pins. Each independent pin is put on each telescopic unit according to the orientation, position and sequence of the user's choice and is fixed on the parts of the bones to be restored. The telescopic units on the rod constitute the external fixation system. However the pins are placed, the telescopic units of the system, thanks to their extreme flexibility and the independence of transposition provided to them by the telescopic mechanism they contain, can be fitted on the pins and effect their complete immobilisation. Thus, the bending of pins, required in many cases when using other systems, is avoided and, as a result, the biomechanical endurance of the system is maintained at its maximum.

An additional advantage of the present invention and, more particularly, of the independent movement of the units constituting it and, consequently, of the independent placing of each pin, or of each pair of pins held by a pin clamp when the units hold pairs of pins, is that they display an amazing spatial flexibility, requiring no special auxiliary template for their correct positioning from the manufacturer indicating to the surgeon the correct placing of the pins. Thus, the present invention is an extremely manageable system, with less final production cost, one requiring much less time of surgical operation that the previous ones and which, by shortening the duration of the surgical operation and anaesthesia, minimises the risks faced by the patient.

Additionally, in contrast to other holding mechanisms of pins, the units of the present invention have the advantage that they do not limit the pins to a place near the rod nor to a preset and predesignated by the manufacturer position from the rod, but they have the ability to move and increase or decrease the position of the pins from the rod, either by themselves, or with the use of the extending arm, thanks to the telescopic holding mechanism used by the present invention. This means that it is possible to hold osteal parts far from the rod, with pins each of which defines a different bracing plane with the bone, thus providing complete immobilisation of the bone parts in the position chosen by the user and the avoidance of the parts being displaced on account of the application of forces not axial to the bone. As a result, and an additional advantage, the bone parts to be restored are protected by the random additional bending and shearing forces and so the risk of a failure of the pin or the mechanism itself is minimised.

Moreover, in contrast to most of the modem holding mechanisms of pins, an additional advantage, due to the versatility of the position rod-pin displayed by the present system and the units constituting it, is that it is possible to position the rod in any desirable orientation and distance in relation to the bone, either parallel or at angle. The placing of the system in parallel orientation in relation to the bone is often preferred, as it causes increased elasticity of the whole system, while protecting the bone fragments from oscillations in other directions, which are catastrophic, as it has been clinically established that this elasticity accelerates the porosis of the fracture. At other times, in contrast, the placing of the rod is sought to be in another orientation so that during the elongation the peripheral section of the bone be 'trasnsported' to varus, valgus or parallel transposition in relation to the central section. Thanks to the versatility of the distance rod-pin, offered by the present system and the units constituting it, this choice is allowed and expedited without the addition of further extensions to the original system.

The present invention also displays the advantage that, through an alternative way of application, in the end of the telescopic mechanism, there can be adapted and 'locked' either a similar telescopic mechanism or an extending arm so as to make possible the holding of two adjacently- or consecutively-placed rods.

Another advantage of the present system and the units constituting it particularly by comparison with WO 91 11149 A is that, in contrast to certain systems which while using an extending rod to distance the pins from the rod, nevertheless use friction couplings instead, thus rendering the whole system weak from an biomechanical point of view, the telescopic units constituting the present system use, for the use of an extending arm, rigid couplings which add to the endurance of the system.

Another advantage of the present invention is that in case the initial placing of the telescopic units is deemed inefficient, for some reason, it is possible afterwards for one or more telescopic units to be placed on the rod, after the placement of the system on the patient has been completed, without being forced to remove and re-place the already placed bracing units.

Another advantage of the present invention is that it makes it possible to maximise the immobilisation of the bone to be restored by using a second rod which, after being placed in the same way as the first rod, can then be connected to it by using either one or more extending arms or a second telescopic mechanism, or a combination of the afore-mentioned accessories.
Finally, one more advantage of the present invention is that the use of elastic agents in the accessories of its mechanisms allows the surgeon the possibility to achieve a temporary immobilisation of these accessories and, consequently, of all the units constituting the system, until he has decided on the suitable and desirable final position of each unit so as to proceed to their complete immobilisation.

For all the above-mentioned reasons, the advantages of the present invention make the surgical operation short and the placement of the telescopic units and of the whole system they constitute extremely easy for the surgeon.

The invention is described below with the help of a non-restrictive example and with reference to the attached drawings, which display one form of execution of the subject matter of the present invention.
Figure 1 displays an indicative form of application of the present invention and represents an external fixation system, consisting of a rod and six telescopic units of holding a pin, and four telescopic units with an extending arm, in combination with a second rod, placed on a bone.
Figure 2 is a drawing in perspective view showing one telescopic unit of holding a pin or even a second pin with cross-section of the clamp.

Figure 3 displays in detail the components constituting a telescopic unit.

Figure 4 shows in detail a view of the whole mechanism of the present invention of a telescopic unit of holding a pin, with a partial cross-section of the lower half and the front of the upper half.

Figure 5 shows in perspective a drawing of the whole mechanism of the present invention of a telescopic unit of holding a pin, in an alternative way of application, with a joint, for the facilitation of the subsequent placement of an additional mechanism on the rod.

Figure 6 shows a cross-section of the telescopic unit of holding a pin, along the plane I-II of figure 4.

Figure 7 shows the back side view of the telescopic unit of holding a pin with a cross-section of a fracture on the plane III-IV of figure 4.

Figure 8 shows a cross-section of the telescopic unit of holding a pin, with an extending arm.

Figure 9 shows an alternative way of application of the telescopic unit, with a taper key and with a holding mechanism and a second pin.

Figure 10 shows an alternative way of application of the telescopic unit, without straining rings.
Figure 11 shows an indicative form of application of the telescopic unit and represents an external fixation system, consisting of a rod and four telescopic mechanisms of holding a pin, placed on a bone.

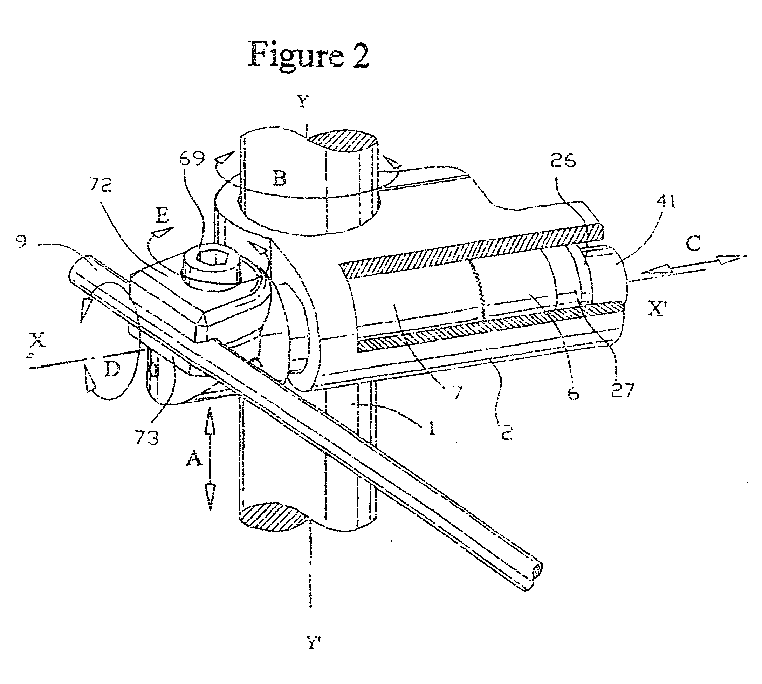

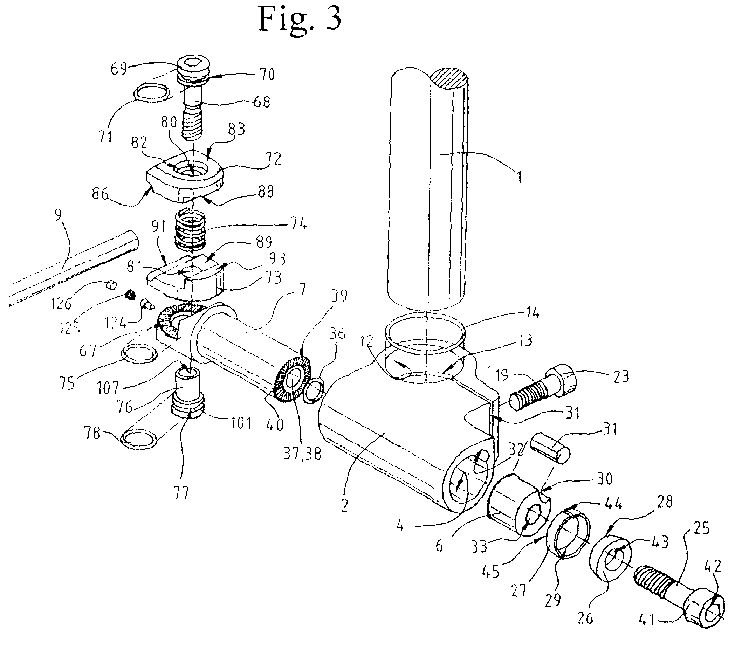

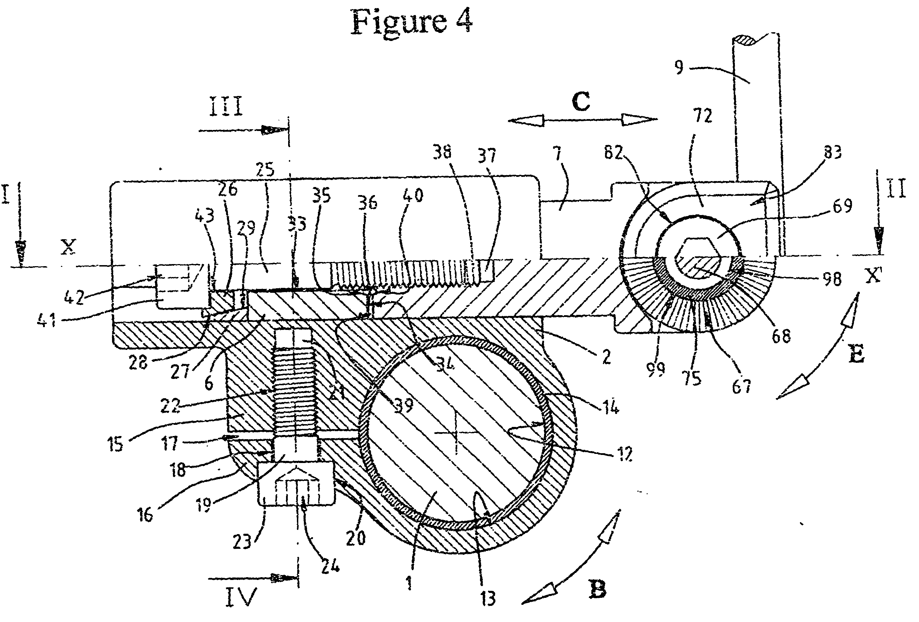

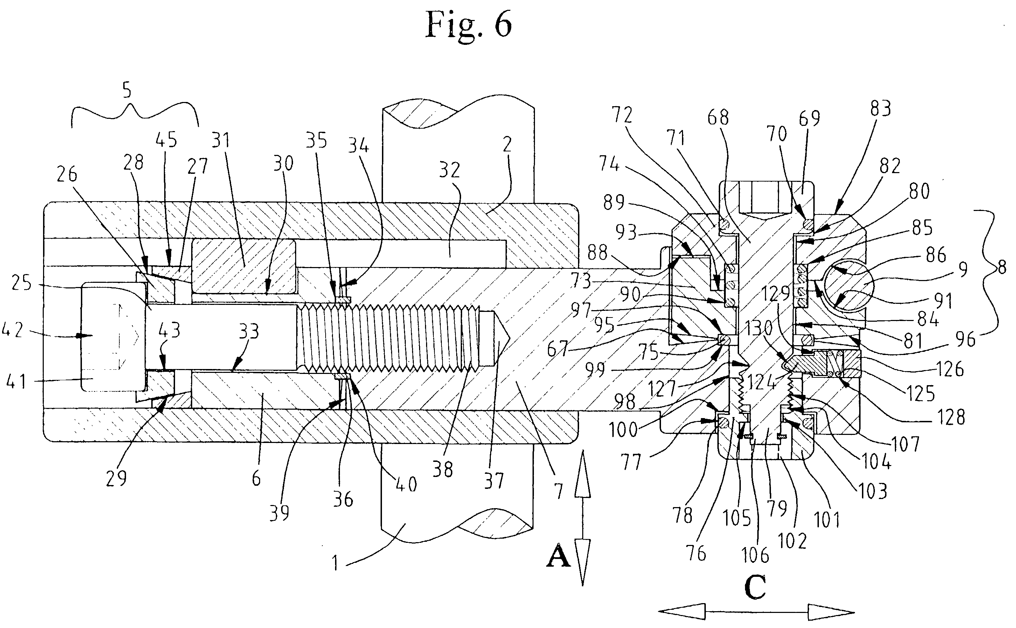

Along and around the rod (1) on the Y-Y' axis, as shown in figure 2, a clamp is placed, which has the ability to move along the Y-Y' axis along the rod (1) according to arrow A and to rotate around the Y-Y' axis according to arrow B. The clamp (2) comprises a mechanism of axial and rotating immobilisation along and around the rod (1). The clamp (2) shows a through hole (4) whose X-X' axis, as shown in figure 3, is in a plane perpendicular to the Y-Y' axis of the rod (1). Inside the through hole (4) there is a rotation regulator (6), as shown in figures 2 and 3, which has the ability to move telescopically along the X-X' axis according to arrow C of figures 2 and 4 and when axially immobilised by the immobilisation mechanism (5), it immobilises rotatively and axially the telescopic arm (7), as shown in figure 6, to which it is adjoined. The telescopic arm (7), which has the ability to move rotatively around the X-X' axis according to arrow D of figure 2 and axially along the X-X' axis according to arrow C of figure 2, carries at its end an orientating and holding mechanism (8) of a pin (9) or a pair of pins (9,10) or even an extending arm (11).

The figures show a way of application of the present invention on a rod (1), solid with a circular cross-section, but, of course, the present invention can also be applied to other kinds of cross-sectional rods like, for example, to a rod with a polygonal cross-section, etc., as well as to a rod that is not solid but, for example, tubular or even with telescopic elements, or with joints or elastic agents, with a damper, etc.
Along the rod (1) at the Y-Y' axis of figure 3, a clamp (2) is placed, by means of a through hole (12) of the circular cross-section located in one part of the clamp, of which one way of operation is shown in figures 2, 3 and 4 and which allows the transposition of the clamp (2) along the rod (1) on the Y-Y' axis according to arrow A and the rotation of the clamp (2) around the Y-Y'axis according to arrow B. The through hole (12) of the circular cross-section carries an internal groove (13) of an elastic ring like, for example, that of an O-type ring (14), according to figures 3 and 4, which facilitates the user by temporarily preventing any undesirable sliding of the clamp (2) along the rod (1) along the Y-Y' axis according to arrow A and any undesirable rotation of the clamp around the Y-Y' axis according to arrow B, until the final position of the exact placement of the clamp has been chosen by the user, using the axial and rotary motion fixation mechanism.

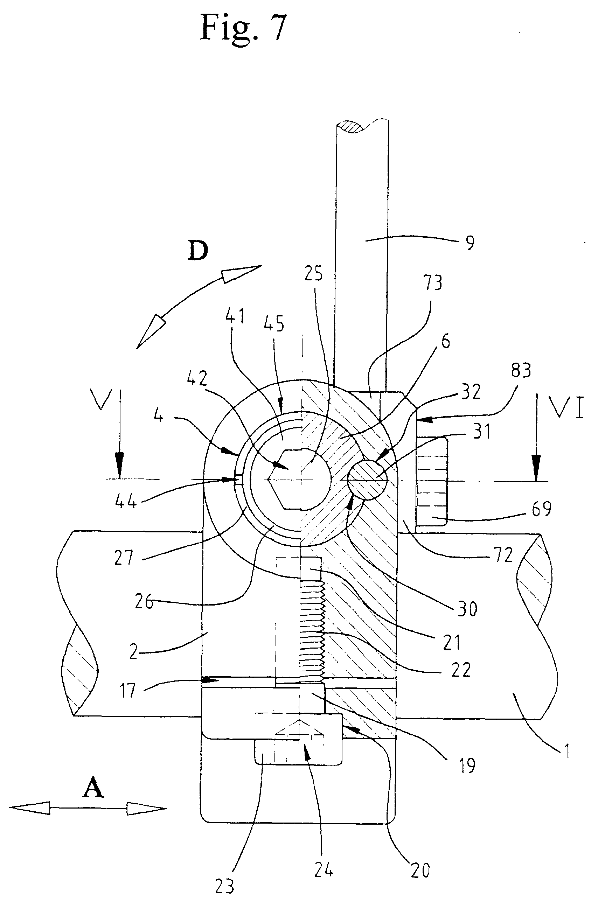

The axial and rotary motion fixation mechanism, as shown in figures 2, 3, 4 and 7, includes the rod (1) with one jaw, the upper (15) and lower (16) part of which, as shown in figures 4 and 7, are divided by a through slit (17). On the lower part of the jaw (16), as shown in figure 4, there is a hole (18) for a screw (19) and a recess for the head (23) of the screw (19) at the point of its positioning, while on the upper part of the jaw (15), as shown in figure 4, there is a hole (21 ) for a screw (19) on the same axis with the hole (18) for the screw (19) of the lower part of the jaw (16), as shown in figure 4, which carries an internal thread (22). The screw (19) carries a head (23) with a socket or a grip (24), as shown in figures 4 and 7, suitable for the screwing and unscrewing tool (such as a hexagonal screw key, or a special key for the screwing of a screw with internal or external socket, etc.), or even for an early screwing by hand. The screwing of the screw (19) results in the tightening of the two parts of the jaw, of the upper (15) and the lower (16), achieving the complete immobilization and preventing the clamp (2) from sliding along the rod (1) according to arrow A, as shown in figure 2, and around the rod (1) according to arrow B, as shown in figures 2 and 4.

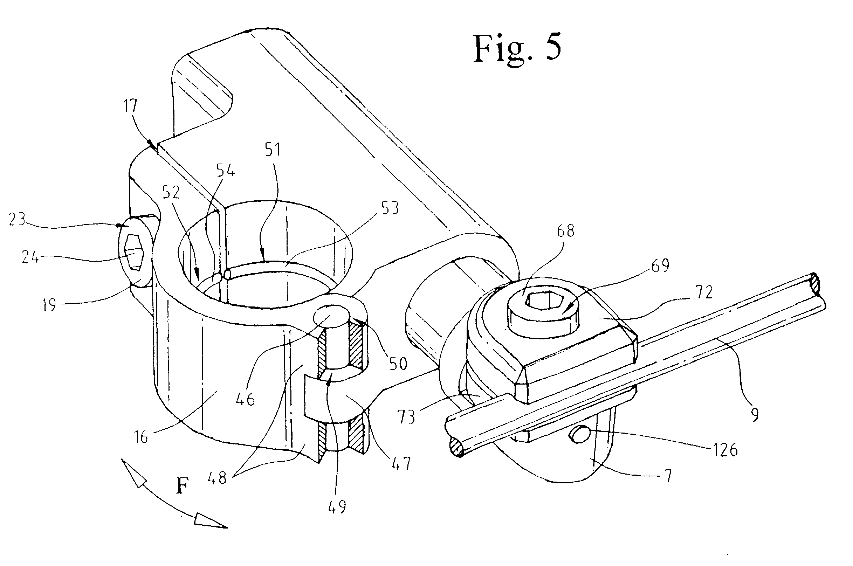

The axial and rotary motion fixation mechanism, as shown in figures 2 and 4, including the rod (1) with a jaw and consisting of an upper (15) and a lower (16) part, is only one way of application of the present invention. Another way of application, shown in figure 5, is for the axial and rotary motion fixation mechanism (3) to comprise two jaws, the upper (15) and the lower (16), which constitute two independent components, each of which carries in its antidiametrical to the through slit (17) end a joint part, (47) for the upper jaw and (48) for the lower jaw, respectively. The joint parts (47) and (48) carry a hole in their centers, (49) and (50) respectively. Through the holes (49) and (50), which are coaxial, passes a pivot (46), which is placed and fixed inside the joint parts (47) and (48) by any means known to those versed in the art (such as impaction, screwing, etc.). The pivot (46) is located at a point which is antidiametrical to the through slit (17), so as to make possible the partial rotation of the lower (48) jaw around the pivot (46), according to arrow F. This variation in the application way of the present invention provides the user with the possibility, during the surgical operation, to place a new part of the holding unit of a pin or even a second pin or even an extending arm between two already positioned units, without having to remove and re-place them. In this case, the upper (47) and lower (48) jaw each carry an internal configuration recess, (51) and (52) respectively, of an elastic body such as a part of an elastic O-ring type ring, (53) and (54) respectively, placed and fixed inside the recesses (51 ) and (52), as shown in figure 5. The elastic bodies (53) and (54) facilitate the user in preventing temporarily the clamp (2) from any undesirable sliding along the rod (1) on the Y-Y' axis according to arrow A and around the rod (1) around the Y-Y' axis until the final exact positioning of the clamp has been chosen by the user through the axial and rotary motion fixation mechanism (3).

As is also shown in figure 7, the clamp (2) is crossed by a through hole (4) on the X-X' axis, the wall of which, in its whole length or in part of it, is traversed by a key way (32). As shown in figure 6, inside the through hole (4) there is a telescopic arm (7), which in one end, which extends outside the through hole (4), carries, an orientating and holding mechanism (8) of one pin (9) or a pair of pins (9,10) or even an extending arm (11), while the other, which is inside the through hole (4), as shown in figures 2, 3 and 6, adjoins a rotation regulator (6), and furthermore the rotation regulator (6) adjoins an axial immobilisation mechanism (5) which, completely immobilised axially and rotatively, completely immobilises in turn axially and rotatively the telescopic arm (7).
The axial immobilisation mechanism (5), as shown in figure 6, consists of a screw (25) and two straining rings, an internal one (26) and an external one (27). The internal straining ring (26) has an external conicity (28) which continues along its whole length and has a hole (43) for the passing of the screw (25). The external strained ring (27) has an internal conicity (29) which continues along its whole length, similar to the external conicity (28) of the internal ring (26), in order to achieve a complete mesh of at least part of the external conical surface (28) of the internal ring (26) with at least part of the internal conical surface (29) of the external ring (27) through the coaxial positioning of the internal straining ring (26) inside the external straining ring (27). The internal conicity (29) continues along the whole length of the external straining ring (27), which is open and carries a through slit (44) at a point in its perimeter, as shown in figures 3 and 7. The screw (25) carries a head (41) with a socket (42), as shown in figures 3, 4, 6 and 7, suitable for the screwing and unscrewing tool (such as a hexagonal screw key, or a special key for the screwing of a screw with internal or external socket, etc.).

The rotation regulator (6), as shown in figures 3, 6 and 7, is of a cylindrical shape and carries in its center a through hole (33). In its frontal surface, which, adjoined to the telescopic arm (7), completely immobilizes it rotatively, carries radial toothed face (34). In the internal perimeter of the radial toothed face (34) there is a recess (35) at the end of the hole (33) for an elastic ring, such as, for example, of an O-ring type ring or any other elastic agents (36), as shown in figures 4 and 6. At a part of the cylindrical surface of the rotation regulator (6), as shown in figures 3 and 6 and in cross-section in figure 7 with V-VI orientation, there is a key way (30) of a semicircular cross-section, which holds a key (31 ) of a cylindrical shape. The key (31), part of its curved surface fitted inside the key way (30) of the rotation regulator (6), is fixed inside it by any means known to those versed in the art (such as screwing, seizing, wedging, etc.), so that it becomes an integral and unified body of the rotation regulator (6), while the rest of it, the part which is not fitted inside the key way (30) of the rotation regulator (6), remains uncovered.

The rotation regulator (6), as well as the key (31 ), constituting after the fixing by any means known to those versed in the art (such as screwing, seizing, wedging, etc.) a new integral body, adjoin and slide along their curved surface, along the through hole (4) and the key way (32) of the clamp (2), as shown in figures 3, 6 and 7. Thus, the part of the rotation regulator (6), with the exception of the part of the key way (30) covered by the key (31), adjoins and slides along the X-X' axis of the through hole (4) of the clamp (2), with its curved surface. The part of the cylindrical surface of the key (31 ) which remains uncovered and is not inside the key way (30) of the rotation regulator (6), adjoins and slides inside and along the length of the key way (32), which traverses part of the wall of the through hole (4) and the clamp (2). However, the insertion of part of the key (31 ) into the key way (32) prevents the rotative movement of the whole new integral component constituted by the rotation regulator (6) together with the key (31 ) around the X-X' axis of the through hole (4) of the clamp (2).

The cylindrical shape of the key (31), as shown in the figures of the present invention, is only indicative of how it can be applied. In this light, the shape of the key may not be cylindrical but rectangular, disc-shaped, sphenoidal, etc. Correspondingly, the part of the key way (30) of the rotation regulator (6), which holds one part of the key (31), may not be of a cylindrical shape but of a shape corresponding to that of the part of the key (31) which adjoins the surface of the key way (30). Correspondingly, the key way (32) which holds the other part of the key (31) can also not be semicircular but to have a cross-section corresponding to that of the part of the key (31) sliding on its surface. This correspondence between the cross-sections of the part of the key way (30) which holds one part of the key (31) with the part of the key (31) which adjoins its surface, as well as the correspondence of the key way (32) holding the other part of the key (31) to the part of the key (31) which slides on its surface, results in the complete rotary motion fixation of the rotation regulator (6), at the same time providing the possibility of its axial movement along the hole (4).

Moreover, that in the drawings of the present invention is shown only a key (31) and a key way (30) traversing the surface of the rotation regulator (6) and a key way (32) traversing part of the wall of the through hole (4) of the clamp (2), is only indicative of how it can be applied. Thus, the number of the keys (31) and the number of the key ways (30) inside which the keys (31) are adjoined and slide may be larger, but always equal, so that inside each key way (32) is affixed and slides one key (31), thus ensuring a more complete stabilization of the rotation regulator (6) on the wall of the through hole (4) of the clamp (2).

The application way of the key (31) at a point of the curved surface of the rotation regulator (6), as it is described above and shown in figures 3, 6 and 7 of the present invention, is only indicative of its application way, as it is possible for the rotation regulator (6) and the key (31) to constitute not two separate components connected to one another, but one part made integral from the very beginning, with a cross-section similar to that which results from the subsequent joining of the two parts, of the rotation regulator (6) and the key (31), or from the joining of the rotation regulator (6) with more keys (31).

The rotation regulator (6) is adjoined to a telescopic arm (7), which is cylindrical and carries in its center a blind hole (37) for the insertion of a screw (25), with an internal thread (38), as shown in figures 3, 4 and 6. The blind hole (37) is coaxial with the through hole (33) of the screw (25) of the rotation regulator (6), with the through hole (43) of the internal straining ring, as shown in figures 3, 4 and 6. The telescopic arm (7) carries in its end which extends outside the through hole (4) an orientation and holding mechanism (8) of one pin (9) or a pair of pins (9,10) or even an extending arm (11). In its other end, which is inside the through hole (4) of the clamp (2) and is adjoined to the rotation regulator (6), it carries a radial toothed face (39), perimetrically of the hole (37), with an equal number of teeth and corresponding geometry to that carried by the radial toothed face (34) of the rotation regulator (6). The teeth of both the radial toothed face (34) of the rotation regulator (6) and the radial toothed face (39) of the telescopic arm (7) are so made that the teeth of each toothed face are inserted and fit completely inside the teeth of the opposite radial toothed face along the full length of their surfaces. In the internal perimeter of the radial toothed face (39), the telescopic arm (7) carries a recess (40) at the end of the blind hole (37) for an elastic agent such as, for example, an O-ring type ring (36), according to figures 3, 4 and 6.

The elastic agents such as, for example, the O-ring type ring or any other elastic agents (36), as shown in figures 3, 4 and 6, which on one side are adjoined to the surface of the recess (40) of the hole (37) of the radial toothed face (39) of the telescopic arm (7) and on the other side are adjoined to the recess (35) of the hole (33) of the radial toothed face (34) of the rotation regulator (6), prevent the meshing of the two radial toothed faces (39) and (34) of the telescopic arm (7) and the rotation regulator (6), respectively.

The early screwing of the screw (25) in the internal thread (38) of the telescopic arm (7) aims at the drawing closer of the radial toothed face (39) of the telescopic arm (7) and the radial toothed face (34) of the rotation regulator (6). During the procedure of drawing closer these two parts (6) and (7) through screwing, the elastic O-ring type ring or any other elastic agents (36), which is located between these two parts, as on one side it is adjoined to the surface of the recess (35) of the hole (33) of the radial toothed face (34) of the rotation regulator (6) and on the other side it is adjoined to the surface of the recess (40) of the hole (37) of the radial toothed face (39) of the telescopic arm (7), is twisted, preventing temporarily the complete meshing of the radial toothed face (39) of the telescopic arm (7) with the radial toothed face (34) of the rotation regulator (6). Once the user has selected a suitable angle for the final position of the telescopic arm (7), in relation to the rotation regulator (6), along and around the X-X' axis, he intensifies the tightening of the screw (25), thus achieving the complete meshing of the radial toothed face (39) of the telescopic arm (7) with the radial toothed face (34) of the rotation regulation (6). At the same time, the existence of the key (31 ), which prevents the rotation of the rotation regulator (6) around the X-X' axis, after the complete meshing of the radial toothed face (34) of the rotation regulator (6) with the radial toothed face (39) of the telescopic arm (7), makes also possible the prevention of the rotation of the telescopic arm (7) around the X-X' axis.

As shown in figures 3, 6 and 7, the rings of the axial immobilization mechanism (5) are positioned coaxially, the internal one (26) inside the external one (27), so that the two conical surfaces of the two straining rings are in contact. The external conical surface (28) of the internal ring (26) adjoins the internal conical surface (29) of the external ring (27). The farther screwing of the screw (25) inside the internal thread (38) of the telescopic arm (7) forces the internal straining ring (26), where the head (41) of the screw (25) is adjoined to and presses on, to penetrate even deeper inside the external straining ring (27) and due to the reverse conical shape of the two surfaces (28) and (29), and thanks to the through slit (44) located in the perimeter of the external ring (27), there occurs an increase of the external perimeter of the external curved surface (45) of the external ring (27). The increase of the external perimeter of the external ring (27) results in the external curved surface (45) of the external ring (27) pressing on the internal wall of the through hole (4) of the clamp (2) , thus achieving a complete axial and rotary motion fixation of the two straining rings of the axial immobilization mechanism (5), the internal (26) and the external (27), along and around the X-X' axis.

At the same time, the complete meshing of the radial toothed face (39) of the telescopic arm (7) with the radial toothed face (34) of the rotation regulator (6), combined with the existence of the key (31 ), part of which is located inside the key way (32) immobilizing rotatively the rotation regulator (6), with the pressure exercised simultaneously by the external curved surface (45) of the external (27) ring on the internal wall of the through hole (4) of the clamp (2) and with the complete straining of the screw (25), produces, due to the generated friction forces, a complete axial and rotary motion fixation, that is, a 'blocking' along and around the X-X' axis not only of each part separately but also in relation to the rest of the parts and, more particularly, with the internal (26) straining ring, the external (27) straining ring, the rotation regulator (6), the telescopic arm (7) and the screw (25), and not only in relation to each other, but also in relation to the clamp (2).

The existence of the through slit (44) at a point of the perimeter of the external straining ring (27), as described above and shown in figures 3, 4, 6 and 7, is only one way of application of the present invention. Another way of application is one where there is no through slit in the perimeter of the external straining ring (27). Still another way of application is one where there is not only one pair of straining rings but more pairs, placed in succession and adjoined to one another by pair.

The cylindrical shape of the rotation regulator (6) and the through hole (4) of the clamp (2), with the cross-section they are shown to have in the drawings of the present invention, is only an indicative way of application. Thus, the shape of the rotation regulator (6), as well as the shape of the through hole (4) of the clamp (2), may not necessarily be cylindrical but polygonal or asymmetrical, etc., but the cross-section of the rotation regulator (6) and the through hole (4) must always be corresponding. In this case, the immobilization of the rotation regulator (6) inside the through hole (4) of the clamp (2), by the axial immobilization mechanism (5), is achieved even without the existence of the key (31 ) and without the existence of the key way (30), thanks to the correspondence between the cross-section of the rotation regulator (6) and the non-circular cross-section of the through hole (4). This correspondence between the cross-sections of the rotation regulator (6) and the through hole (4) ensures a complete angular immobilization, that is, the 'blocking' of the rotation regulator (6) inside the through hole (4) of the clamp (2), thanks to the rigid couplings.

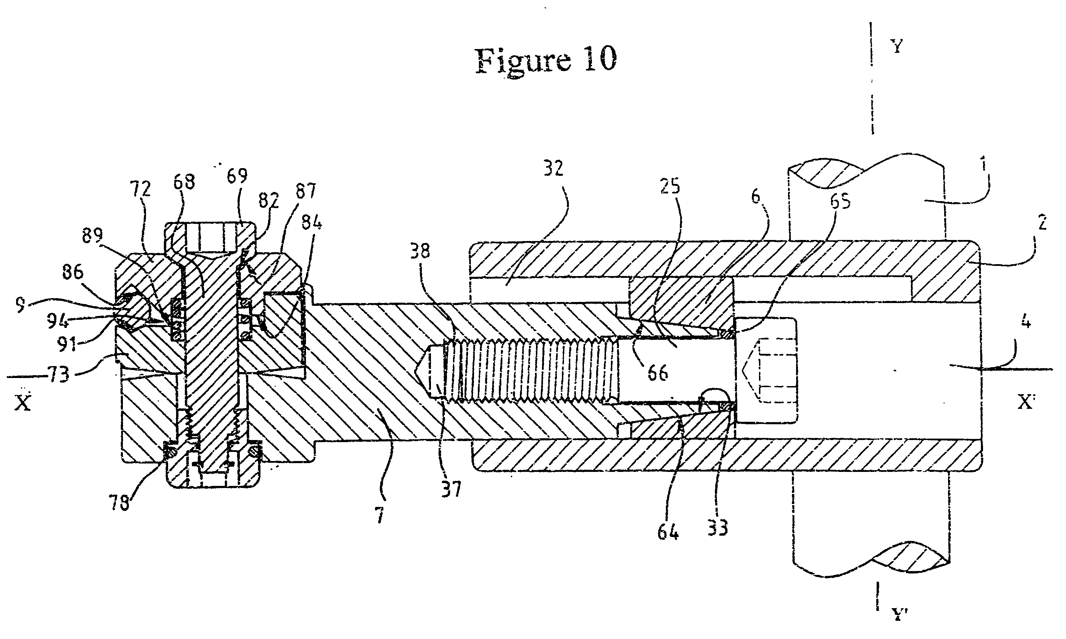

The way of the complete axial and rotary motion fixation along the X-X' axis of the axial immobilization mechanism (5) of the rotation regulator (6) and the telescopic arm (7), as described above and shown in figures 3, 6 and 7, is not the only way of 'blocking' these parts. Another way of 'blocking' of axial and rotary motion fixation, of the axial immobilization mechanism (5), the rotation regulator (6) and the telescopic arm (7), along and around the X-X', is shown in figure 10.

As shown in figure 10, the rotation regulator (6), constituting with the key (31) as shown in figure 3, a single part, either manufactured as one from the beginning or resulting from the union of the two parts by any means known to those versed in the art, is externally cylindrically shaped and carries in its center a through hole (33) with conicity (66) and along the length of its surface is traversed by a longitudinal through slit (63).

The telescopic arm (7) is cylindrically shaped and carries in its center a blind hole (37), with an internal thread (38), which is coaxial with the through hole (33) for the insertion of the screw (25) of the rotation regulator (6) as shown in figure 10. The part of the telescopic arm (7), which is inside the through hole (4) of the clamp (2), carries an external conical end (64), corresponding to the internal conicity of the through hole (33) of the rotation regulator (6). The external conical end (64) of the telescopic arm (7) is inserted and adjoined to the corresponding conicity surface (66) of the through hole (33) of the rotation regulator (6). At the top of the conical end (64) of the telescopic arm (7) there is an elastic agent (65) such as, for example, a spring, an elastic ring, etc., to prevent a early self-blocking of the telescopic arm (7), inside the through hole (33) of the rotation regulator (6), until the final axial and rotary position of these two parts has been chosen by the user. The tightening of the screw (25) initially causes a twist of the elastic agent (65) until the external conical end (64) of the telescopic arm (7) comes into full contact with the corresponding internal conicity surface (66) of the through hole (33) of the rotation regulator (6). The tightening of the screw (25) results in the conical end (64) of the telescopic arm (7) being inserted even deeper inside the internal conicity of the through hole (33) of the rotation regulator (6), and due to the reverse conical form of the two surfaces (64) and (66) and thanks to the through slit (63) which exists and traverses the whole length of the rotation regulator (6), there occurs an increase of the external diameter of the rotation regulator (6). The result of the increase of the external diameter of the rotation regulator (6) is that the rotation regulator (6) presses on the internal wall of the through hole (4) of the clamp (2) and achieves a complete axial and rotary motion fixation along and around the X-X' axis, not only of the rotation regulator (6), inside the through hole (4), but also of the telescopic arm (7), inside the rotation regulator (6), both in relation to each other and in relation to the clamp (2).

At the same time, the existence of the key (31) prevents the rotation of the rotation regulator (6) around the X-X' axis and due to the pressure exercised by the conical surface (64) of the telescopic arm (7) upon the conical surface (66) of the rotation regulator (6) because of the tightening of the screw (25), results as well in the complete rotary motion fixation of the telescopic arm (7) around the X-X' axis.

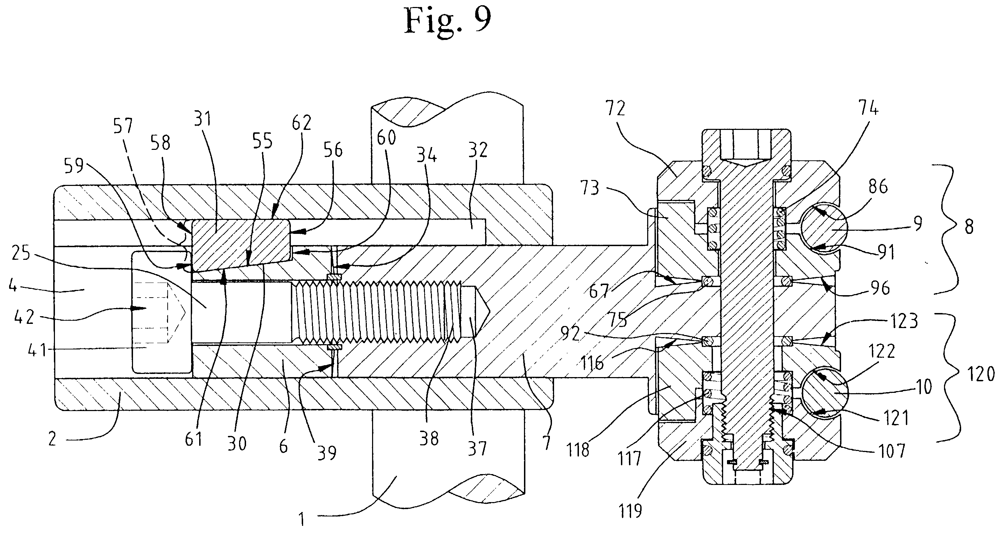

Furthermore, according to another application way of the present invention, as shown in figure 9, the key way (30) of the rotation regulator (6), which holds one part of the key (31), has a rectangular cross-section and displays an inclined surface (55), in such a way that the depth (59) of the insertion of the key way (30) is bigger than the depth of the end (60) of the key way (30). Moreover, according to this application way, the key way (32) of the through hole (4) of the clamp (2), which holds the other part of the key (31), is flat and also displays a rectangular cross-section. Correspondingly, the key (31) has a wedged shape, with one of its sides inclined (61), an inclination corresponding to the inclination displayed by the surface (55) of the key way (30) and its other side flat (62) and with uneven bases, (58) and (56), as shown in figure 9. In addition, the larger (58) of its two bases is greater than or equal to the opening (57) created by the key way (30) of the rotation regulator (6) and the key way (32) of the through hole (4) of the clamp (2), while the smaller (56) of its two bases is smaller than the opening (57) created by the key way (30) of the rotation regulator (6) and the key way (32) of the through hole (4) of the clamp (2).

The key (31) is placed, in its part which displays an inclined surface (61), inside the corresponding inclined surface (55) of the key way (30) of the rotation regulator (6) and, in its part which displays a flat surface (62), inside the flat surface of the key way (32) of the through hole (4) of the clamp (2), as shown in figure 9. At the same time, the larger (58) of its two bases is adjoined to the pressure surface of the head (41) of the screw (25), which tightens the rotation regulator (6) onto the telescopic arm (7), as shown in figure 9, while the smaller (56) of the two bases of the key (31 ) is inside the opening formed between the key way (30) of the rotation regulator (6) and the key way (32) of the wall of the through hole (4). Thus, the tightening of the screw (25) inserts the key (31), with the smaller (56) of its two bases first, inside the opening (57) formed by the key way (30) of the rotation regulator (6) and the key way (32) of the wall of the through hole (4). The tightening of the screw (25) causes the complete wedging of the key (31), inside the opening (57), which due to the friction forces generated causes an axial immobilisation of each and every part both separately and in relation to the rest, that is, of the rotation regulator (6) and the telescopic arm (7) in relation to the clamp (2). Moreover, the key (31), due to its form, also causes the rotary motion fixation of each and every part both separately and in relation to the rest, that is, of the rotation regulator (6) and the telescopic arm (7). This way of application of the present invention renders unnecessary the existence of the two straining rings, the internal (26) and the external (27) of the axial immobilisation mechanism (5).
In all the application ways of the present invention, the insertion of the axial immobilisation mechanism (5), the rotation regulator (6) and the telescopic arm (7), inside the through hole (4), of the clamp (2) is possible through both entrances of the through hole (4), as shown in figures 2, 3, 4, 5, 6, 7, 8, 9 and 10.

The telescopic arm (7) carries on its end which extends outside the through hole (4) a radial toothed face (67), an orientating and holding mechanism (8) of a pin (9) or two pins (9,10) or even an extending arm (11), as shown in figures 2, 3, 4, 6, 8 and 11.

As shown in figure 3, the radial toothed face (67) is located at a plane perpendicular to the plane of the radial toothed face (39) carried by the telescopic arm (7) in its other end which is inside the through hole (4) of the clamp (2).

The orientating and holding mechanism (8) of a pin (9) or even a second pin (10) or even an extending arm (11), as shown in figures 3 and 6, consists of a screw (68), two jaws, the upper (72) and the lower (73), a spring (74) positioned between the two jaws, an elastic agent (75), a tightening nut (76) and an unscrewing limiter (79). The orientating and holding mechanism (8) of a pin (9) or even a second pin (10) or even an extending arm (11), as shown in figures 3 and 6, may also carry a temporary stabilisation mechanism of a pin (9).

As shown in figures 3 and 6, the screw (68) carries a thread (107) and in a part of its head (69) it carries a perimetric groove (70), inside which is placed an elastic ring, e.g., an O-ring (71) type ring and in a part of its body before the thread it carries a perimetric groove (127). The tightening nut (76) carries a perimetric groove (77), inside which is placed an elastic ring, e.g., an O-ring (78) type ring, and the temporary stabilisation mechanism consists of a nib (124), an elastic pressure means (125) such as a spring, an elastic sphere, etc., and a stopper (126), which are placed in a hole (128) of the telescopic arm (7).

As shown in figures 2, 3 and 6, the two jaws, the upper (72) and the lower (73), in a part of their surface, carry a through hole (80), the upper jaw, and a through hole (81), the lower jaw. The upper jaw (72), on its upper side (83), at the entrance of the through hole (80), carries a recess (82) for the head (69) of the screw (68). During the placing of the screw (68), inside the hole (80), the elastic ring (71) hinders the free rotation of the screw (68). The upper jaw (72), on its lower side (84), carries a recess (85) in the hole (80), for the holding of the spring (74) and a holding groove (86) of a pin (9), and a recess (88).

The lower jaw (73), on its upper side (89), carries a recess (90) in the hole (81), for the holding of the spring (74). Also, the lower jaw (73), on its upper side (89), carries a holding groove (91) of a pin (9), of the same cross-section with, and opposite of, the holding groove (86) of the pin (9), carried by the upper jaw (72) on its lower side (84). It also carries an projection (93), corresponding to the recess (88) carried by the upper jaw (72) on its lower side (84), as shown in figures 2, 3 and 6. The height of the projection (93) and the depth of the recess (88) are such that, between the lower side (84) of the upper jaw (72) and the upper side (89) of the lower jaw (73), a gap (94) is created. As shown in figure 10, it is clear that the recess (88) and the projection (93) may be reversed, on the lower and the upper j aw, respectively.

As shown in figures 2, 3 and 6, the lower jaw (73), on its lower side (95), carries a radial toothed face (96), perimetrically of the hole (81), with an equal number of teeth and corresponding geometry to the radial toothed face (67), carried by the telescopic arm (7). The teeth of both the radial toothed face (96) of the lower jaw (73) and the radial toothed face (67) of the telescopic arm (7) are so made that the teeth of each toothed face are inserted and fit completely inside the teeth of the opposite radial toothed face, along the full length of their surfaces.

The hole (81) of the lower jaw (73) carries a recess (97) for the placing of an elastic body such as, for example, an O-ring type ring , inside which the elastic body (75) is inserted, which hinders an early meshing of the two frontal toothed face surfaces (67) and (96).

As shown in figure 6, through the center of the radial toothed face (67) of the telescopic arm (7) there traverses a through hole (98), on the upper part of which there is a recess (99) for the placing of an elastic ring (75), while on its lower part there is a recess (100) for the head (101) of the tightening nut (76).

The head (101) of the nut (76), as shown in figure 6, on its lower side, carries a socket or grip (102), suitable for the screwing and unscrewing tool (such as an hexagonal key, a key special for the screwing a head with an internal or external socket, etc), or even for early screwing by hand. In the center of the tightening nut (76) there is a through hole (103), which traverses the tightening nut (76) along its full length. From a point of the through hole (103) traversing the tightening nut (76), starts an internal thread (104), which extends to the end of the through hole (103). At the internal starting point of the thread (104), of the through hole (103), of the tightening nut (76), there is a narrowing point (105), which prevents the passing of the unscrewing limiter (79) and consequently the exit of the screw (68) from the tightening nut (76). From the head (101) of the tightening nut (76), passes an unscrewing limiter (79) such as, for example, a safety, a small screw, etc., which is placed at the end (106) of the screw (68) in a suitable way such as, for example, by screwing or gluing, etc. The unscrewing limiter (79) prevents the screw (68) from getting completely unscrewed from the tightening nut (76) but, of course, the application of the present invention remains the same even without the use of the unscrewing limiter (79).

The head (101) of the tightening nut (76), at a point of its curved surface, which is inserted in the recess (100), of the through hole (98), of the telescopic arm (7), carries a perimetric groove (77), inside which is placed an elastic ring (78), which prevents the free rotation of the tightening nut (76).

The spring (74), which is between the two jaws (72) and (73), achieves their distancing from each other, thus making possible the easy placement of the pin (9) between the grooves (86) and (91) of the upper and lower jaw, respectively.

The nib (124), as shown in figures 3 and 6, carries a widening point (129), while in the end with which it enters the hole (128) it carries a conical or wedged configuration ( 130), with which it enters the groove (127) when the screw (68) is in the proper place. The elastic agent (125) exercises permanent pressure on the nib (124), which tends to move towards the screw (68), while the stopper (126), firmly fixed from the beginning by any means known to those versed in the art (such as, for example, screwing, wedging, etc.), prevents the exit of the elastic agent (125) and the nib (124) from the hole (128).

As soon as the user places the pin (9) between the two jaws, upper (72) and lower (73), and presses on the screw (68) to hold the pin (9), the elastic agent is twisted, allowing the screw (68) to slide axially, letting the groove (127) meet the nib (124), thus ensuring the temporary axial immobilisation of the screw and consequently of the whole orientating and holding mechanism (8) of a pin (9) or even an extending arm (11).

The user places the pin on the bone to be fixed. Then, he adjusts the pin (9) between the groove (91) of the lower jaw (73) and the opposite groove (86) of the upper jaw (72) and has the choice of tightening the assembly either by rotating the screw (68) or the tightening nut (76), at will, depending on which of the two tightening parts he has easiest access at the particular stage of the operation. In case there is a temporary stabilisation mechanism of a pin (9) on the unit - since its operation is possible even without it - the temporary stabilisation of the pin (9) is achieved by simply putting pressure on the screw (68).

The tightening of the screw (68), through the thread (107) inside the thread (104) of the tightening nut (76), or the reverse, that is, the tightening of the thread (104) of the tightening nut (76) around the thread (107) of the screw (68) - whichever of the two parts the user selects to tighten, depending on which one is more suitable, is the same - achieves the coiling of the spring (74) and consequently the approaching and immobilisation of the two jaws, the upper (72) and the lower (73) and therefore the tightening of the pin (9) between the groove (86) carried by the upper jaw (72) on its lower side (84) and the corresponding groove (91) carried by the lower jaw (73) on its upper side (89).
At the same time, the elastic ring (75), as shown in figures 3 and 6, which on its one side is adjoined to the surface of the recess (97) of the hole (81) of the lower side (95) of the lower jaw (73) and on its other side it is adjoined to the surface of the recess (99) of the hole (98) of the radial toothed face (67) of the telescopic arm (7), with the early screwing of the screw (68), is twisted, preventing temporarily a complete meshing of the radial toothed face (67) of the telescopic arm (7) with the radial toothed face (96) of the lower jaw (73).

As soon as the user has selected the proper angle of the final position of the orientating and holding mechanism (8) of a pin (9) in relation to the telescopic arm (7), he intensifies the tightening of the screw (68), thus achieving a complete meshing of the radial toothed face (67) of the telescopic arm (7) with the radial toothed face (96) of the lower jaw (73) and, at the same time, due to the pressure exercised by the tightening assembly of the screw (68) and the tightening nut (76), the two jaws, (72) and (73), are tightened, both between themselves and on the telescopic arm (7), thus achieving a complete immobilisation of the pin (9) in relation to the telescopic arm (7) and therefore with the clamp (2).

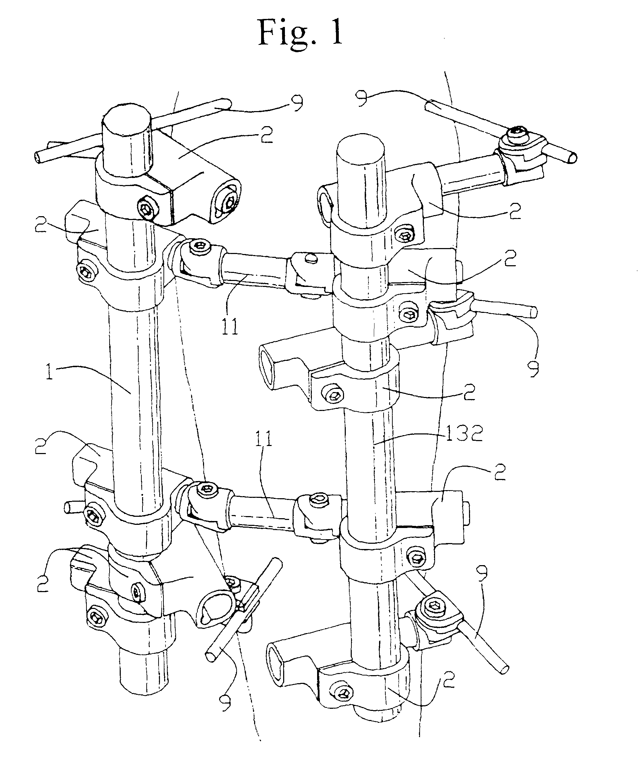

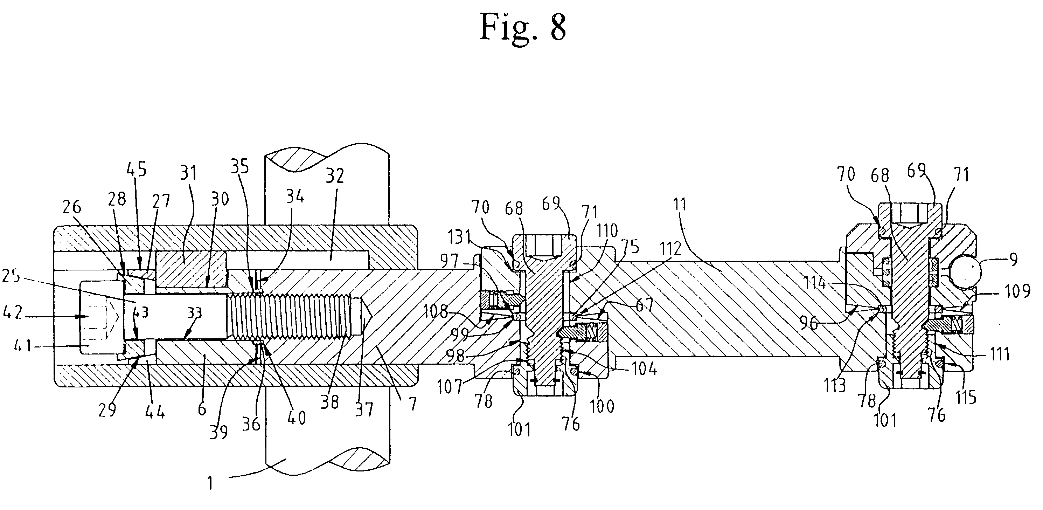

Another way of application of the present invention, which is shown in figures 1 and 8, is the case where the user can extend the length of the telescopic arm (7), using towards this end the extending arm (11) component. As shown in figure 8, the extending arm (11) component carries in its two ends, surfaces (108) and (109) with an toothed face surface. The surfaces (108) and (109) are at such a plane that the axis of the arm is within the plane of the surfaces. These two surfaces with the radial toothed face, that is, (108) and (109), as shown in figure 8, have opposite orientation, but in other application ways of the present invention they may have the same orientation or any other orientation. The two radial toothed faces, (108) and (109), carry in their centre through holes, (110) and (111) respectively, which at one point and in their internal perimeter each carries a recess (112), for the elastic ring (75), and a recess (113), for the elastic ring (114), respectively. On the other end of the through holes (110) and (111) there are the recesses (131) and (115) respectively, for the placing of the heads (101) of the tightening nuts (76). As shown in figure 8, through the through hole (110) passes a screw (68), which carries a thread (107) and which at a section of its head (69) carries a perimetric groove (70), inside which is placed an elastic ring (71). The placement of the rest of the components, that is, the tightening nut, (76) with the perimetric groove (77) inside which is placed the elastic ring (78), which passes through the through hole (98) of the telescopic arm (7) and the unscrewing limiter (79), according to the application way described above and shown in figure 6, remains the same.

The elastic ring (75), as shown in figure 8, which on its one side is adjoined to the surface of the recess (99) of the hole (98) of the telescopic arm (7) and on its other side it is adjoined to the surface of the recess (112) of the hole (110) of the radial toothed face (108) of the extending arm (11) component, prevents the meshing of the two radial toothed face surfaces (67) of the telescopic arm (7) with the radial toothed face (108) of the extending arm (11) component.

The early screwing of the screw (68) in the tightening nut (76) aims at the approaching of the radial toothed face (67) of the telescopic arm (7) to the radial toothed face (108) of the arm extension (11) component. During the procedure of the approaching of these two components, (7) and (11), through screwing, the elastic ring (75), which is located between these two parts, as on its one side it is adjoined on the surface of the recess (99) of the hole of the telescopic arm (7) and on its other side it is adjoined to the surface of the recess (112) of the hole (110) of the extending arm (11) component, is twisted, preventing temporarily a complete meshing of the radial toothed face (67) of the telescopic arm (7) with the radial toothed face (108) of the extending arm (11) component.

Then, the immobilisation of the pin (9), in relation to the extending arm (11) component, is done in exactly the same way the pin (9) is immobilised in relation to the telescopic arm (7), through the use of the holding mechanism (8), placed on the radial toothed face (109) of the extending (11) arm.

Figure 9 shows an application way of the present invention, different from that shown in figures 2, 3, 4, 5, 6 and 7. According to this application way, the telescopic arm (7), in its end which extends outside the through hole (4) and carries an orientating and holding mechanism (8) of a pin (9) or even an extending (11) arm, can carry a second pair of orientating and holding jaws (120) of a second pin (10). In this case, the two pairs, (8) and (120), of orientating and holding jaws of two pins (9) and (10), are identical and consist of the same parts. However, the end of the telescopic arm (7), which extends outside the through hole (4), is so shaped that it also accommodates the placement of the second pair of the orientating and holding jaws (120) of a second pin (10). Thus, the end of the telescopic arm (7) carries also a second radial toothed face (116) at a plane also perpendicular to the plane of the radial toothed face (39) carried by the telescopic arm (7) in its other end which is positioned inside the through hole (4) of the tightening clamp (2) and at a plane parallel to the first radial toothed face (67). On the second radial toothed face (116), a second orientating and holding mechanism (120) of the second pin (10) is placed, in the same way the first orientating and holding mechanism (8) of the first pin (9) was placed on the first radial toothed face (67) and is shown in figures 2, 3, 4 and 6.

Thus, as shown in figure 9, the tightening of the screw (68) inside the tightening nut (76) causes the coiling of the spring (74) of the first mechanism (8) and the coiling of the spring (117) of the second mechanism (120) and consequently the approach and the immobilisation of the two jaws, the upper (72) and the lower (73), of the first mechanism (8), and at the same time the approach and the immobilisation of the two jaws, the upper (118) and the lower (119) of the second mechanism (120). It also causes the tightening of both the first pin (9), between the grooves (86) and (91) of the first mechanism (8), and the second pin (10), between the grooves (121) and (122) of the second mechanism (120).

At the same time, the early screwing of the screw (68), inside the tightening nut (76), causes the approach of both the first radial toothed face (67) of the telescopic arm (7) to the radial toothed face (96) of the lower jaw (73) of the first mechanism (8) and of the second radial toothed face (116) of the telescopic arm (7) to the radial toothed face (123) of the upper jaw (118) of the second mechanism (120).
As shown in figure 9, during the procedure of approximating all these parts, that is, the telescopic arm (7), the two jaws, the upper (72) and the lower (73) of the first mechanism (8) and the two jaws, the upper (118) and the lower ( 119), of the second mechanism (120) through screwing, the first elastic ring (75), which is halfway between the telescopic arm (7) and the first mechanism (8), and the second elastic ring (92), which is halfway between the telescopic arm (7) and the second mechanism (120), are twisted, preventing temporarily the complete meshing of both the radial toothed face (67) of the telescopic arm (7) with the radial toothed face (96) of the lower jaw (73) of the first mechanism (8), and the radial toothed face (116) of the telescopic arm (7) with the radial toothed faced (123) of the upper jaw (118) of the second mechanism (120).

As shown in figure 9, once the user has chosen the proper angle of the final position of the first pin (9) and the first mechanism (8), in relation to the telescopic arm (7), and of the second pin (10) and the second mechanism (120), in relation to the telescopic arm (7); he intensifies the tightening of the screw (68). Due to the pressure exercised by the tightening assembly of the screw (68) and the tightening nut (76), the two jaws, the upper (72) and the lower (73) of the first mechanism (8) are completely tightened to each other and to the telescopic arm (7), through the radial toothed face (96) of the lower jaw (73) and through the radial toothed face (67). At the same time, the two jaws, the upper (118) and the lower (119), of the second mechanism (120) are tightened to each other and to the telescopic arm (7), through the radial toothed face (123) of the upper jaw (118) and through the radial toothed face ( 116). As a result of the tightening of the four jaws to the telescopic arm (7), there is achieved the complete immobilisation of both the first pin (9) and of the second pin (10) in relation to the telescopic arm (7) and consequently in relation to the clamp (2).

Also, as shown in figures 1, 3 and 9, on the end of the telescopic arm (7), which extends outside the through hole (4) and on which are fitted two orientating mechanisms of a pin, can be fitted, on one face (67) of the telescopic arm (7), one telescopic orientating and holding mechanism (8) of a pin (9), and on the other face (116) of the telescopic arm (7), an extending arm (11), or the reverse.

As shown in figure 10, in all its ways of application of the present invention, the groove (86) of the pin (9), carried by the upper jaw (72) in its lower side (84) and the corresponding groove (91 ) of the pin (9) carried by the lower jaw (73) in its upper side (89), may be of a cylindrical or a triangular cross-section. The same is also true for the grooves (121) and (122) of the jaws (118) and (119), respectively, of the second orientating and holding mechanism (120). This variation offers the user the possibility of placing pins of various diameters.
As shown in figure 10, in this case, the pressure surface (87) of the head (69) of the screw (68) may be a part of a sphere, as shown in figure 10, and the surface of the recess (82) of the head of the screw (68) has a corresponding shape. This application way of the invention results in the achievement of a uniform load on the pressure surface (87) of the head (69) of the screw (68) in whatever angle the upper jaw (72) can take in relation to the lower one (73), due to the different diameters of the pins which can be used.

The tightening of the assembly may be achieved either by rotating the screw (68) or by rotating the tightening nut (76), as shown in figures 3, 4, 6, 7, 9 and 10 of the present invention, at the user's discretion, and this is achieved thanks to the elastic rings (71) and (78) on both the head (69) of the screw (68) inside the perimetric groove (70) and the head (101) of the tightening nut (76) inside the perimetric groove (77), respectively.

To achieve the full facilitation of the user, but also the maximum service to the patient, during this placement, all the edges of each unit and of the system must be properly chamfered.

The joining of the holding mechanism of a pin (9) or two pins (9,10) or even an extending arm (11), as described above, offers a wide range of possibilities to the user, with regard to the number of mechanisms and the combination of the parts which he will select to place on the rod, in order to achieve, in his opinion, the best stabilization of the bone to be restored.

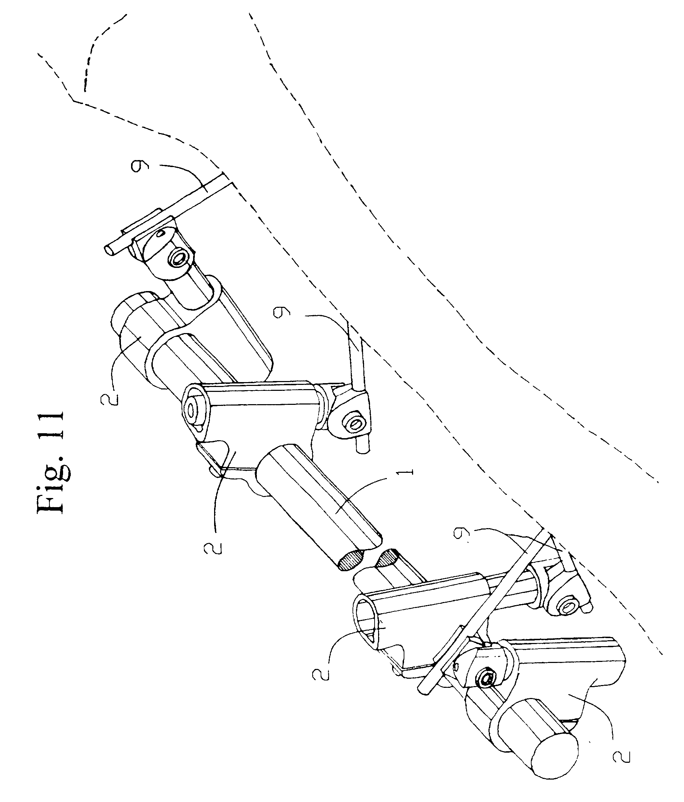

Thus, as shown in figure 1, the user may choose to position one or two rods (1), which he connects together either through extending arms (11) or through their direct connection, through the telescopic arms (7) of the holding mechanisms (8) of a pin (9). He may also choose to simultaneously use holding mechanisms (8) of a pin (9) or even holding mechanisms (120) of two pins (9,10) or even holding mechanisms (120) of an extending arm (11), or even orientating and holding mechanisms (8) of a pin (9) and an extending arm (11) or, as shown in the indicative application way of figure 11, only four telescopic units, each carrying one pin, all four of which are placed on one rod (1).

## Claims

1. A telescopic orientating and holding unit for a pin (9) or for a pair of pins (8,10) or for an extending arm (11), said unit being mountable on a rod (1), said unit such that said pin or pair of pins or extending arm is capable of five degrees of freedom of spatial movement relative to the rod (1), A, B, C, D, E where A means translation along the rod axis, B means rotation about the rod axis, C means translation perpendicular to the rod axis, along the axis of a telescopic arm (7), D means rotation about the telescopic arm axis, which is perpendicular to the rod axis, E means rotation about the axis of a screw (68), which is perpendicular to the telescopic arm axis and according to the fact that it consists of:
a. a clamp (2) for placement along and around the rod (1), which has an axial and rotary fixation mechanism (15,16,19) for axial fixation along (A) and rotary fixation around (B) the rod (1), and having a through hole or opening (4) whose axis is perpendicular to the rod (1) axis when the unit is mounted on the rod.
b. a telescopic mechanism, consisting of an axial immobilization mechanism (5) and a rotation regulator (6) both located inside through hole or opening (4) of the clamp (2), and telescopic arm (7) connected to the axial immobilization mechanism (5) for immobilization of the telescopic arm (7) along (C), the axis of the opening (4), and connected to the rotation regulator (6) for immobilization of the telescopic arm about (D) the axis of the opening (4).
c. an orientating and holding mechanism (8) for the pin (9) or the extending arm (11) and optionally, when the orientating and holding mechanism (8) is for the pin (9), a second orientating and holding mechanism (120) for a second pin (10), said mechanism (8) or mechanisms (8, 120) being mounted on the telescopic arm (7) in such a way as to be rotatable about axis (E) which is perpendicular to the axis of the telescopic arm (7); said orientating and holding mechanism (8) or mechanisms (8, 120) can securely be immobilized, when final position is determined, using a rigid coupling (96, 97),
**characterized in that**:
there is a key (31) - keyway (32) rigid coupling between opening (4) and rotation regulator (6) and telescopic arm (7) is connected to the rotation regulator (6) and the axial immobilization mechanism via a pair of radial toothed faces (34,39) and a screw (25), to offer rotational and axial immobilization of the telescopic arm (7) and consequently of the orientating and holding mechanism (8); and each orientating and holding mechanism (8,120) is mounted to the telescopic arm (7) via a pair of radial toothed faces (96,97) and the screw (68) which defines rotation (E) axis to offer immobilization against rotation (E) of said orientating and holding mechanism (8,120) relative to said telescopic arm (7) and consequently securely fix the pin (9) or pair of pins (9,10) or extending arm (11)

2. An external fixation system, comprising two or more telescopic orientating and holding units according to claim 1 positioned on a rod (1) which optionally is connected to a second rod (132) achieved through coupling between telescopic arms (7) mounted on the two rods.

3. A telescopic orientating and holding unit for a pin (9) or a pair of pins (9,10) or an extending arm (11), according to claim 1, which is **characterized by** the fact that the clamp (2), is traversed by a through hole or opening (4) and by the fact that, through a through hole or opening (12) of a circular cross-section located at a point on its body, has the ability to slide along the rod (1) on the Y-Y' axis according to arrow A and to rotate around the rod (1), on the Y-Y' axis according to arrow B.

4. An external fixation system according to claim 2, which is **characterized by** the fact that, both the through hole or opening (12) and the rod (1) may be of a circular cross-section or of a non-circular one such as, for example, of a polygonal one, etc., which however must be such that it will allow the sliding of the clamp (2) along the rod (1) on the Y-Y' axis according to arrow A and the rotation around rod (1) around the Y-Y' axis according to arrow B, while it is also **characterized by** the fact that the rod (1) may be solid but also not solid; it may, for example, be tubular or even with telescopic elements, or joints or elastic agents or a damper, etc., or a combination of these.

5. A telescopic orientating and holding unit according to claim 1 or 3 or an external fixation system according to claim 4, which is **characterised by** the fact that the clamp (2) consists of an axial and rotary motion fixation mechanism (3) along and around the rod (1) which, comprises a jaw, the upper (15) and the lower (16) part of which are divided by a through slit (17).

6. A telescopic orientating and holding unit or an external fixation system according to claim 5, which is **characterized by** the fact that on the lower part (16) of the jaw of the axial and rotary motion fixation mechanism (3) of the clamp (2), there is a hole (18) for the holding of a screw, inside which a screw (19) is placed, while on the upper part of the jaw (15) of the axial and rotary motion fixation mechanism (3) of the clamp (2), there is a hole (21) for the holding of the screw (19), carrying an internal thread (22) and located on the same axis with the hole (18) for the holding of the screw (19) of the lower part of the jaw (16), as well as by the fact that the screwing of the screw (19) causes a mechanical tightening of the two part of the jaw, the upper (15) and the lower (16), resulting in the complete immobilisation and the prevention of the sliding of the clamp (2) along the rod (1), according to arrow A, and around the rod (1) according to arrow B.

7. A telescopic orientating and holding unit according to claim 1 or 3 or an external fixation system according to claim 4, which is **characterised by** the fact that in order for the user to be able to position, during the surgical operation, a new telescopic unit between two already placed units, without having to remove and re-install them, the axial and rotary motion fixation mechanism (3) of the clamp (2), may consist of two independent jaws, the upper (15) and the lower (16), which comprises two independent components, each of which carries in its end which is diametrically opposite of the slit (17) a joint part, (47) for the upper and (48) for the lower jaw, respectively, with a central hole in the centre of each part, (49) and (50) respectively, which (49) and (50) are coaxial to one another, while through their centre passes a pivot (46) which is fixed between the joint parts (47) and (48) by any means known to those versed in the art (such as, for example, by wedging, screwing, etc.), thus forming a joint which allows the rotation of the lower (16) jaw around the pivot (46), according to arrow F, as well as by the fact that the screwing of the screw (19) causes a mechanical tightening of the two parts of the jaw, the upper (15) and the lower (16), resulting in the complete immobilisation and the prevention of the sliding of the clamp (2) along the rod (1), according to arrow A, and around the rod (1), according to arrow B.

8. A telescopic orientating and holding unit or an external fixation system according to claims 5, 6 or 7, which is **characterised by** the fact that the upper (15) and lower (16) parts of the jaw of the tightening clamp (2), as well as the two independent jaws, of the tightening clamp (2), carry at a point of their internal surface a friction elastic agent (14) or (53) and (54), which facilitates the user, causing a temporary prevention of an undesirable sliding of the tightening clamp (2) along the rod (1) on the Y-Y' axis according to arrow A, along the axis of the rod, and an undesirable rotation of the tightening clamp (2) around the Y-Y' axis, around the axis of the rod, according to arrow B, until the final and precise position of the permanent positioning of the clamp (2) has been selected by the user through the use of the axial and rotary motion fixation mechanism (3).

9. A telescopic orientating and holding unit for a pin (9) or a pair of pins (9, 10) or an extending arm (11), according to claim 1, which is **characterised by** the fact that, the telescopic mechanism, consisting of an axial immobilisation mechanism (5), a rotation regulator (6) and a telescopic arm (7), is located and moving inside a through hole or opening (4) which traverses the clamp (2), the wall of which, either at its full length or in part of it, is traversed by a key way (32), and by the fact that, the insertion of the whole telescopic mechanism inside the through hole or opening (4) of the clamp (2) is possible through both of the two entrances of the through hole or opening (4).

10. A telescopic orientating and holding unit for a pin (9) or a pair of pins (9, 10) or an extending arm (11), according to claims 1 and 9, which is **characterised by** the fact that the axial immobilisation mechanism (5), consists of a screw (25) and two straining rings, an internal one (26) and an external one (27) and by the fact that the pair of the internal (26) and external (27) straining rings may not be one but more pairs, arranged in sequence, axially and adjoined by pair.

11. A telescopic orientating and holding unit for a pin (9) or a pair of pins (9, 10) or an extending arm (11), according to claim 10, which is **characterised by** the fact that the two straining rings, the internal (26) and the external (27) of the axial immobilisation mechanism (5), carry, on its part, the internal straining ring (26) an external conicity (28) which continues throughout its length and has a hole (43) for the passing of the screw (25) while, on its part, the external straining ring (27) an internal conicity (29) which continues throughout its whole length, corresponding to the external conicity (28) of the internal ring (26), thus achieving a complete contact of at least part of the external conical surface (28) of the internal ring (26) with at least part of the internal conical surface (29) of the external ring (27) through the coaxial placement of the internal straining ring (26) inside the external straining ring (27), while also, the external straining ring (27) may be open at a point of its perimeter, carrying a slit (44).

12. A telescopic orientating and holding unit for a pin (9) or a pair of pins (9, 10) or an extending arm (11), according to claims 10 or 11, which is **characterised by** the fact that, when the screw (25) is screwed inside the internal thread (38) of the telescopic arm (7), its head (41) presses on the internal straining ring (26) forcing it to enter inside the external straining ring (27), and because of the reverse conical shape of the two surfaces, (28) and (29), and thanks to the through slit (44), there occurs an increase in the external diameter of the external ring (27), thus causing the external curved surface (45) of the external ring (27) to press on the internal wall of the through hole (4) of the clamp (2) and to achieve a complete axial immobilisation along the axis (X-X') of the two straining rings of the axial immobilisation mechanism (5) and consequently of the rotation regulator (6) to which it is adjoined, and next of the telescopic arm (7) , in relation to the clamp (2).

13. A telescopic orientating and holding unit for a pin (9) or a pair of pins (9, 10) or an extending arm (11), according to claim 12, which is **characterised by** the fact that the rotation regulator (6), is of a cylindrical shape and carries in its centre a through hole (33), coaxial with the through hole (43) of the internal straining ring (26), and by the fact that on its one surface it is adjoined to the axial immobilisation mechanism (5) while on its other frontal surface, carries a radial toothed face (34), in the internal perimeter of which there is a recess (35) in the hole (33), for the holding of an elastic agent (36), which facilitates the user, causing a temporary prevention of the complete meshing of the two frontal toothed face surfaces, (39) and (34), of the telescopic arm (7) and the rotation regulator (6), respectively, until the user has chosen the final position of their exact definite positioning.

14. A telescopic orientating and holding unit for a pin (9) or a pair of pins (9,10) or an extending arm (11), according to claims 1, 9 or 13, which is **characterised by** the fact that the rotation regulator (6) carries on a part of the curved surface a key way (30), which holds a key (31), which when adjoined, in part of its surface, to the inside of the key way (30), is fixed inside it by any means known to those versed in the art (such as screwing, gluing, wedging, etc.) so as to become an integral part of and a new unified body with the rotation regulator (6), thus preventing the rotation of the rotation regulator (6) around the axis (X-X') of the through hole or opening of the clamp (2).

15. A telescopic orientating and holding unit for a pin (9) or a pair of pins (9, 10) or an extending arm (11), according to claims 1, 9, 13 or 14, which is **characterised by** the fact that the rotation regulator (6), together with the key (31), constituting a new unified body, are adjoined and capable of sliding along their surfaces, along the through hole or opening (4) and the key way (32), which traverses part or the whole length of the wall of the through opening or hole (4) of the clamp (2), as well as that there may be more than one keys (31) as well as key ways (32) and key ways (30), but their number is always equal so that inside each key way (32) a key (31) is adjoined and slides and inside each key way (30) a key (31) is fixed.

16. A telescopic orientating and holding unit for a pin (9) or a pair of pins (9, 10) or an extending arm (11), according to claims 1, 9, 13, 14, or 15 which is **characterised by** the fact that the key way (30) of the rotation regulator (6), may have a cross-section of any, shape, such as circular, square, etc., always similar to the cross-section of the part of the key (31) which is inserted inside it and that, respectively, the key way (32) of the of the clamp (2), which holds the other part of the key (31), may have a cross-section of any shape, such as circular, square, etc., always similar to the cross-section of the part of the key (31) sliding on its surface, so that the insertion of part of the key (31) inside the key (32) way always prevents any rotative movement of the entire new unified component, consisting of the rotation regulator (6) together with the key (31) around the axis (X-X') of the through opening or hole (4) of the clamp (2).

17. A telescopic orientating and holding unit for a pin (9) or a pair of pins (9, 10) or an extending arm (11), according to claims 1, 9, 13, 14, 15 or 16, which is **characterised by** the fact that the rotation regulator (6) and the key (31 ), may constitute from the beginning a unified component, with a cross-section similar to the one which results from the union of the two parts, of the rotation regulator (6) and the key (31), or similar to the one resulting from the union of the rotation regulator (6) with more keys (31), or similar to the particular cross-section of the gap created by the through opening or hole (4) and the key way (32).

18. A telescopic orientating and holding unit for a pin (9) or a pair of pins (9, 10) or an extending arm (11), according to claims 1, 9, 13 or 14, which is **characterised by** the fact that, the cross-section of the rotation regulator (6), as well as the cross-section of the through opening or hole (4) of the clamp (2), may not be circular but polygonal, or non-symmetrical, etc., and that the cross-sections of the rotation regulator (6) and the through opening or hole (4) will always correspond to each other.

19. A telescopic orientating and holding unit for a pin (9) or a pair of pins (9, 10) or an extending arm (11), according to claims 1, 9 or 13, which is **characterised by** the fact that, the key way (30) of the rotation regulator (6), which holds one part of the key (31), may have a trapezoidal cross-section with an inclined bottom surface (55), in such a way that the insertion depth (59) of the key way (30) is bigger than the end depth (60) of the key way (30).

20. A telescopic orientating and holding unit for a pin (9) or a pair of pins (9, 10) or an extending arm (11), according to claims 1, 9, 13 or 19, which is **characterised by** the fact that, the key (31) is of a sphenoidal shape, with one of its sides inclined (61), an inclination corresponding to the inclination presented by the bottom surface (55) of the key way (30), and its other side flat (62), and that its bases, (58) and (56) are unequal, the larger (58) of which is larger or equal to the cross-section of the opening (57) created by the key way (30) of the rotation regulator (6) and the key way (32) of the through hole or opening (4) of the clamp (2), while the smaller (56) of which is smaller than the cross-section of the opening (57).

21. A telescopic orientating and holding unit for a pin (9) or a pair of pins (9, 10) or an extending arm (11), according to claims 13, 14, 15, 16, 17, 18, 19 or 20, which is **characterised by** the fact that, the screwing of the screw (25) inside the internal thread (38) of the telescopic arm (7) results, in the first stage, in the approaching of the radial toothed face (39) to the radial toothed face (34), and that the elastic agent (36), which is located between these two components, is twisted, thus temporarily preventing the complete meshing of the two radial toothed faces, as well as that once the user has chosen the proper angle of the final position of the telescopic arm (7), in relation to the rotation regulator (6) and to the clamp (2), he intensifies the tightening of the screw (25), causing a complete meshing of the radial toothed face (39) with the radial toothed face (34), while at the same time, thanks to the existence of the key (31 ), the complete meshing of the radial toothed face (34) with the radial toothed face (39) results in the prevention of the rotation around the axis (X-X') of the axial immobilisation mechanism (5), the rotation regulator (6) and the telescopic arm (7) with which it is meshed, in relation to the clamp (2).

22. A telescopic orientating and holding unit for a pin (9) or a pair of pins (9, 10) or an extending arm (11), according to claim 20, which is **characterised by** the fact that, the screwing of the screw (25) inserts the key (31 ), with the smaller (56) of its two bases first, inside the opening (57), formed by the key way (30) of the rotation regulator (6) and the key way (32) of the through opening (4), and that the straining of the screw (25) causes the complete wedging of the key (31) inside the opening (57) and the key (31), due to the friction forces which develop and its shape, causes an axial and rotary motion fixation of each component individually and, also, in relation to the rest, that is, to the rotation regulator (6) and the telescopic arm (7), in relation to the clamp (2).

23. A telescopic orientating and holding unit for a pin (9) or a pair of pins (9, 10) or an extending arm (11), according to claims 1 or 9, which is **characterised by** the fact that, the rotation regulator (6) may be shaped cylindrically externally, carrying in its centre an opening of a through hole (33), with conicity (66), and traversed along the full length of its surface by a longitudinal through slit (63), as well as that the telescopic arm (7) is of a cylindrical shape and carries in its centre a blind hole (37) for the insertion of the screw (25) with an internal thread (38), coaxial with the through hole (33) of the rotation regulator (6), and that the telescopic arm (7) has an external conical end (64), corresponding to the one with the internal conicity (66) of the through hole (33) of the rotation regulator (6), as well as that at the top of the conical end (64), there may be placed an elastic agent (65) such as, for example, a spring, an elastic ring, etc.

24. A telescopic orientating and holding unit for a pin (9) or a pair of pins (9, 10) or an extending arm (11), according to claim 13, which is **characterised by** the fact that, the tightening of the screw (25) causes, successively, the twisting of the elastic agent (65), the full fitting of the conical surfaces (64) and (66), the progressive insertion of the conical end (64) inside the internally conical hole (33) and because of the reverse conicity of the two surfaces (64) and (66) and thanks to the through slit (63), corresponding to the slit (44) of the ring (27) , there occurs an increase in the external diameter of the rotation regulator (6), causing its external curved surface to press the internal wall of the through hole (4) and, combined with the existence of the key (31) in the rotation regulator (6), there occurs a complete axial and rotary motion fixation along and around the axis (X-X') of the rotation regulator (6) and the telescopic arm (7), with which it is meshed, both inter se and in relation to the clamp (2).

25. A telescopic orientating and holding unit for a pin (9) or a pair of pins (9, 10) or an extending arm (11), according to claims 1, 9, 13, 21, 22 or 24, which is **characterised by** the fact that, the telescopic arm (7), on its one end, which extends outside the through hole or opening (4), carries a radial toothed face (67) and an orientating and holding mechanism (8) of a pin (9) or even a second pin (10) or even an extending arm (11), while on the other end, which is inside the through hole or opening (4), is adjoined to a rotation regulator (6).

26. A telescopic orientating and holding unit for a pin (9) or a pair of pins (9, 10) or an extending arm (11), according to claims 1, 13, 21, 22, 24 or 25, which is **characterised by** the fact that, the telescopic arm (7) has a cylindrical shape and carries in its centre a blind hole (37) for the screw (25) insertion with an internal thread (38) and that the blind hole (37) is coaxial with the through hole (33) of the rotation regulator (6).

27. A telescopic orientating and holding unit for a pin (9) or a pair of pins (9, 10) or an extending arm (11), according to claims 1, 9, 21, 22, 25 or 26, which is **characterised by** the fact that, the end of the telescopic arm (7), which is adjoined to the rotation regulator (6), carries a radial toothed face (39), perimetrically of the hole (37), with an equal number of teeth and corresponding geometry to those carried by the radial toothed face (34) of the rotation regulator (6), and that the internal perimeter of the radial toothed face (39) carries a recess (40) in the blind hole (37) to hold an elastic agent (36), which on its one side is adjoined to the surface of the recess (40) of the hole (37) of the radial toothed face (39) of the telescopic arm (7), and on its other side it is adjoined to the surface of the recess (35) in the hole (33) of the radial toothed face (34) of the rotation regulator (6).

28. A telescopic orientating and holding unit for a pin (9) or a pair of pins (9, 10) or an extending arm (11), according to claims 1, 9, 13, 14, 21, 22, 25, 26 or 27, which is **characterised by** the fact that, the teeth of both the radial toothed face (34) of the rotation regulator (6) and the radial toothed face (39) of the telescopic arm (7) are so made that the teeth of each toothed face are inserted and fully adjoined between the teeth of the opposite radial toothed face along the full length of their surface, thus preventing the rotative movement of the telescopic arm (7), in relation to the regulator (6) and the clamp (2).

29. A telescopic orientating and holding unit for a pin (9) or a pair of pins (9, 10) or an extending arm (11), according to claims 1, 9 and 25, which is **characterised by** the fact that the orientating and holding mechanism (8) of a pin (9) or even an extending arm (11), consists of a screw (68), two jaws, the upper (72) and the lower (73), a spring (74), placed between the two jaws, an elastic ring (75) and a tightening nut (76), and that the orientating and holding mechanism, may carry a temporary stabilisation mechanism of pin (9).

30. A telescopic orientating and holding unit for a pin (9) or a pair of pins (9, 10) or an extending arm (11), according to claim 29 which is **characterised by** the fact that, the radial toothed face (67) of the telescopic arm (7) is on a plane which is at an angle with the plane of the radial toothed face (39) carried by the telescopic arm (7) in its other end, the one inside the through hole or opening (4) of the clamp (2), and that under the radial toothed face (67) there is a hole (128) with a narrowing point for the holding of the temporary stabilisation mechanism.

31. A telescopic orientating and holding unit for a pin (9) or a pair of pins (9, 10) or an extending arm (11), according to claims 1 or 30, which is **characterised by** the fact that, the screw (68) passes through a through hole (98) which traverses the centre of the radial toothed face (67) of the telescopic arm (7) and carries a thread (107), and on a part of its head (69) it may carry a perimetric groove (70), inside which an elastic ring (71) is placed, while on a part of its body before the thread it may carry a perimetric groove (127), for the temporary stabilisation of the screw (68), while on the end of this it may carry an unscrewing limiter (79) such as, for example, a safety, a small screw, etc., which is placed on the end (106) of the screw (68), in a suitable way such as, for example, screwing or gluing, etc.

32. A telescopic orientating and holding unit for a pin (9) or a pair of pins (9,10) or an extending arm (11), according to claims 30 or 31, which is **characterised by** the fact that, the upper jaw (72) carries on a part of its surface a through hole (80), at the opening point of which it may carry a recess (82) for the head (69) of the screw (68), and that in its lower side (84) it carries a holding groove (86) for a pin (9) and a recess (88) and may also carry a recess (85) in the hole (80), to hold the spring (74), through the use of which there occurs a distancing of the two jaws, (72) and (73) so as to facilitate the placing of the pin (9) between the holding groove (86) and a groove (91) of the lower jaw.

33. A telescopic orientating and holding unit for a pin (9) or a pair of pins (9,10) or an extending arm (11), according to claims 31 or 32, which is **characterised by** the fact that, the lower jaw (73) carries on its upper side (89) a groove (91) of a pin (9), opposite the groove (86) of the upper jaw (72) and at a point on its surface a through hole (81 ), and that on its upper side (89) it may carry a recess (90) in the hole (81), to hold the spring (74), while on its lower side it may carry a recess (97), inside which is placed the elastic body (75) through which is prevented temporarily the complete meshing of the two radial toothed faces, (67) and (96), while during the straining of the screw the (68) elastic body (75) is twisted in order to achieve the complete contact of the two radial toothed faces.

34. A telescopic orientating and holding unit for a pin (9) or a pair of pins (9, 10) or an extending arm (11), according to claims 30, 31, 32 or 33, which is **characterised by** the fact that, the lower jaw (73) carries on its upper side (89) a projection (93), corresponding to the recess (88) carried by the upper jaw (72) on its lower side (84), and that the height of the projection (93) and the depth of the recess (88) may be such between the lower side (84) of the upper jaw (72) and the upper side (89) of the lower jaw (73) that a gap (94) is created, as well as that the recess (88) and the projection (93) may be in reverse positions, on the lower and the upper jaw, respectively.

35. A telescopic orientating and holding unit for a pin (9) or a pair of pins (9, 10) or an extending arm (11), according to claims 30, 31, 32, 33 or 34, which is **characterised by** the fact that, the lower jaw (73) carries on its lower side (95) a radial toothed face (96), perimetrically of the hole (81), with an equal number of teeth and corresponding geometry to the radial toothed face (67) carried by the telescopic arm (7), and that the teeth of both the radial toothed face (96) of the lower jaw (73 ) and the radial toothed face (67) of the telescopic arm (7) are so made as to have the teeth of each toothed face inserted in and completely adjoined between the teeth of the opposite radial toothed face, along the full length of their surfaces.

36. A telescopic orientating and holding unit for a pin (9) or a pair of pins (9, 10) or an extending arm (11), according to claims 30 or 35, which is **characterised by** the fact that, the tightening nut (76) carries a head (101) which, on its lower side, carries a socket or grip (102), suitable for the screwing and unscrewing tool or even for an early tightening by hand, as well as a perimetric groove (77), inside which is placed an elastic ring (78), thanks to which is achieved a temporary hindrance of the free rotation of the tightening nut (76), due to the friction generated between the elastic ring (78) and the internal wall of the surface of the recess (100) of the hole (98), and that at the centre of the tightening nut (76) there is a through hole (103) which traverses the tightening nut (76) along its full length, while from a point of the through hole (103) starts an internal thread (104) which extends to the end of the through hole (103) and the diameter of which is shorter than the diameter of the unscrewing limiter (79) so as to prevent its passing, and consequently the passing of the screw (68) outside the nut (76).

37. A telescopic orientating and holding unit for a pin (9) or a pair of pins (9, 10) or an extending arm (11), according to claim 29, which is **characterised by** the fact that, the temporary stabilisation mechanism consists of a nib (124), which may carry a widening part (129), while on its end which is inserted inside the hole (128) it carries a conical or spenoidal or any other kind of curved configuration (130), corresponding to that of a groove (127) which it enters when the screw (68) is in a proper position, of an elastic pressure means (125) such as a spring, an elastic sphere, etc., which exercises permanent pressure on the nib (124), which tends to slide towards the screw, and of a stopper (126), in a fixed position from the very beginning by any means known to those versed in the art (such as, for example, screwing, wedging, etc.), and that the nib (124), the elastic pressure means (125) and the stopper (126) are placed in the hole (128) of the telescopic arm (7).

38. A telescopic orientating and holding unit for a pin (9) or a pair of pins (9, 10) or an extending arm (11), according to claim 37, which is **characterised by** the fact that, once the user has implanted the pin (9) in the bone to be restored, in order to place it inside the telescopic unit, he adjusts it between the groove (91) and the opposite groove (86), and that if inside the system has also been placed a temporary stabilisation mechanism of a pin (9), the user may achieve, at this stage, a temporary immobilisation of the pin (9), simply through applying pressure on the screw (68), so the nib (124) is inserted inside the groove (127) carried by the screw (68), while the unhooking of the pin (9) from the holding mechanism (8) is achieved by applying a reverse force.

39. A telescopic orientating and holding unit for a pin (9) or a pair of pins (9, 10) or an extending arm (11), according to claims 36, 37 or 38, which is **characterised by** the fact that, in order to achieve the holding of the pin (9) by the telescopic unit, the user selects and tightens either the screw (68) inside the tightening nut (76) or the reverse - as the unit, thanks to the elastic rings carried by the nut (76) and the screw (68), allows the user the choice of selecting to screw only the one of the two components to which he has access, without at the same time being forced to immobilise the other one as well, achieving the same result - thus causing the twisting of the spring (74) and therefore the approaching of the two jaws, the upper (72) and the lower (73), as well as that once the user has selected the proper angle of the final position of the mechanism (8), in relation to the telescopic arm (7) and the clamp (2), he intensifies the tightening of the screw (68) onto the tightening nut (76), thus progressively causing the complete meshing of the two radial toothed faces, (67) and (96), and at the same time, the full tightening of the two jaws (71) and (73), not only around the pin (9) and between themselves but also against the telescopic arm (7), thus achieving the complete immobilisation of the pin (9), in relation to the telescopic arm (7) and therefore to the clamp (2).

40. A telescopic orientating and holding unit for a pin (9) or a pair of pins (9, 10) or an extending arm (11), according to claims 1, 9, 25, 29, 30, 31, 32, 33, 34, 35, 36 or 37, which is **characterised by** the fact that, the length of the telescopic arm (7) may be extended by adjusting on it the extending arm (11) component, which carries on both of its ends surfaces, (108) and (109), with a radial toothed face, on such a plane that the axis of the arm is contained within the plane of the surfaces, which may have reverse orientation with regard to each other, similar orientation or any other kind.

41. A telescopic orientating and holding unit for a pin (9) or a pair of pins (9, 10) or an extending arm (11), according to claim 40, which is **characterised by** the fact that, the two radial toothed faces, (108) and (109), of the extending arm (11) carry in their centre through holes, (110) and (111) respectively, and a recess each, (112) and (113), for two elastic rings, (75) and (114) respectively, and that at the exit of the through holes, (110) and (111), there is a recess, (131) and (115) respectively, to hold the heads, (101) and (101), of two tightening nuts, (76) and (76), as well as that the use of the two elastic rings, (75) and (114), until the user fully strains two screws (68), temporarily prevent the complete meshing of the two radial toothed face surfaces, (67) and (108), as well as of the two radial surfaces (109) and (96).

42. A telescopic orientating and holding unit for a pin (9) or a pair of pins (9, 10) or an extending arm (11), according to claims 40 or 41, which is **characterised by** the fact that, to fit the extending (11) arm to the telescopic arm (7), the radial toothed face (108) of the extending (11) arm meshes with the radial toothed face (67) of the telescopic arm (7), using in the same way the same parts used for the fitting of the orientating an holding mechanism (8) of a pin (9) onto the telescopic arm (7), that is, a screw (68) with a thread (107) and a perimetric groove (70), an elastic ring (71), a tightening nut (76), with an elastic ring (78) and a temporary stabilisation mechanism.

43. A telescopic orientating and holding unit for a pin (9) or a pair of pins (9, 10) or an extending arm (11), according to claims 40, 41 or 42, which is **characterised by** the fact that, when the extending (11) arm is fitted on the telescopic arm (7), the orientating and holding mechanism (8) of a pin (9) is fitted on the second radial toothed face (109) of the extending (11) arm, as well as that during the use of the extending (11) arm, the immobilisation of the pin (9), in relation to the extending arm (11) component, and consequently of the clamp (2), is achieved through the use of the holding mechanism (8), in exactly the same way the pin (9) is immobilised when the extending (11) arm is not used, and also the immobilisation of the extending (11) arm, in relation to the telescopic arm (7) and consequently to the clamp (2), is achieved in exactly the same way the orientating and holding mechanism (8) is immobilised in relation to the telescopic arm (7).

44. A telescopic orientating and holding unit for a pin (9) or a pair of pins (9, 10) or an extending arm (11), according to claims 1, 9, 25, 29, 30, 31, 32, 33, 34, 35, 36, 37, 40, 41, 42 and 43, which is **characterised by** the fact that, the telescopic arm (7), in its end which extends outside the through hole or opening (4) and carries the orientating and holding mechanism of a pin (9) or even an extending arm (11), may carry a second radial toothed face ( 116) on a plane parallel to the first radial toothed face (67) so that it holds an identical pair of orientating and holding jaws (120) of a second pin (10), consisting of the same components with the first pair of orientating and holding jaws (8) of a pin (9).

45. A telescopic orientating and holding unit for a pin (9) or a pair of pins (9, 10) or an extending arm (11), according to claim 44, which is **characterised by** the fact that, the placing of the second identical orientation and holding pair of jaws (120) of a second pin (10) is achieved on the second radial toothed face ( 116) in exactly the same way in which the first orientating and holding pair of jaws (8) of the first pin (9) is placed on the first radial toothed face (67).

46. A telescopic orientating and holding unit for a pin (9) or a pair of pins (9,10) or an extending arm (11), according to claims 44 or 45, which is **characterised by** the fact that, in order to achieve the holding of the two pins, (9) and (10), by the two holding mechanisms, (8) and (120) respectively, once the user has implanted the two pins, (9) and (10), in the bone to be restored, in order to place them in the telescopic unit, he positions each of them between the grooves (91 ) and (86) carried by the first and (121) and (122) carried by the second holding mechanism, and that the user selects and tightens either the screw (68) inside the tightening nut (76) or, in reverse, the tightening nut (76) around the screw (68) - as the unit, thanks to the elastic rings contained in the screw (68) and the nut (76) allows the user to select to screw that part of the two to which he has access, without at the same time being forced to also immobilise the other, achieving the same result - thus causing the simultaneous twisting of the two springs (74) and (117), carried by the two holding mechanisms, (8) and (120) respectively, and therefore the approaching of the two jaws, the upper (72) and the lower (73), of the first mechanism (8) and the simultaneous approaching of the two jaws, the upper (118) and the lower (119) of the second mechanism (120), not only between themselves but also in relation to the telescopic arm (7), thus tightening not only the first pin (9) between the grooves (86) and (91) of the first mechanism (8) but also the second pin (10) between the grooves (121) and (122) of the second mechanism (120).

47. A telescopic orientating and holding unit for a pin (9) or a pair of pins (9, 10) or an extending arm (11), according to claims 44, 45 or 46, which is **characterised by** the fact that, in order to achieve the complete holding of the two pins (9) and (10) by the two holding mechanisms (8) and (120), as the user intensifies the screwing of the screw (68) inside the tightening nut (76), the two elastic rings (75) and (92), which are located on either side of the telescopic arm (7) and are placed between it and the two holding mechanisms, (8) and (120) respectively, are twisted, temporarily preventing the complete meshing of the two pairs of radial toothed faces, (67) and (96), of the first mechanism (8) , and of (116) and (123) of the second mechanism (120), as well as that once the user has selected the proper angle of the final position of the two mechanisms (8) and (120) in relation to the telescopic arm (7), he intensifies the tightening of the screw (68) inside the nut (76) and through the pressure exercised by the tightening assembly of the screw (68) to the nut (76) of the first pair of jaws (72) and (73) is tightened, and at the same time so does the second pair of jaws, (118) and ( 119), not only against each other but also against the telescopic arm (7), causing a complete meshing of the two pairs of the radial toothed faces (96) and (67), on the one hand, and the (123) and (116) on the other. As a result of the tightening of the four jaws against the telescopic arm (7), there occurs a complete immobilisation not only of the two pairs of jaws but also of the two pins, (9) and (10), in relation to the telescopic arm (7) and the clamp (2).

48. A telescopic orientating and holding unit for a pin (9) or a pair of pins (9, 10) or an extending arm (11), according to claims 1, 44 or 45, which is **characterised by** the fact that, on the end of the telescopic arm (7), which extends outside the through hole (4) and to which are adjoined the two orientating mechanisms of a pin, may be fitted and tightened in the same way, on one side (67) of the telescopic arm (7), an orientating and holding mechanism (8) of a pin (9), and on the other side (116) of the telescopic arm (7) an extending (11) arm, or the reverse.

49. A telescopic orientating and holding unit for a pin (9) or a pair of pins (9, 10) or an extending arm (11), according to claims 32, 33, 34, 35, 36 or 39, 40, 41, 42, 43, 44, 45, 46, 47, 48, which is **characterised by** the fact that, in all application ways of the present invention, the grooves (86) and (91) may be of a circular or triangular cross-section, either one or several or all of them, as such a variation allow the user to place pins of various diameters, and that in case even one of them is of a triangular cross-section, the pressure surface (87) of the head (69) of the screw (68) may be part of a sphere and the surface of the recess (82) must have a corresponding shape so as to achieve a uniform load of the pressure surface (87) of the head (69) of the screw (68), in any angular position possible for the upper jaw (72) in relation to the lower one (73).

50. An external fixation system, according to claim 2, which is **characterised by** the fact that, in the case the system also comprises a second rod (132), this also carries two or more telescopic orientating and holding unit (8) for a pin (9) or a pair of pins (9, 10) or an extending arm (11), and may be connected to the first rod (1) through the direct coupling of the radial toothed faces of two or more telescopic arms (7) by pairs, that is, one of the first rod (1) with one, at a time, of the second rod (132), thus maximising the stability of the system.

51. An external fixation system, according to claims 2 or 50, which is **characterised by** the fact that, for the direct connection of each pair of the telescopic arms (7), that is, one of the first rod (1) with one of the second rod (132), the radial toothed face (67), carried by each telescopic arm (7) on its end, is tightened through a screw (68), directly against the radial toothed face (67) carried by the other telescopic arm (7), also causing the twisting of an elastic agent (75), which might be intervening between the two radial toothed faces (67) of the two telescopic arms (7), adjoined on its side to the surface of a recess (99) in a hole (98) which carries the radial toothed face (67) of each telescopic arm (7).

52. An external fixation system, according to claims 2 or 50, which is **characterised by** the fact that, the system may comprise a second rod (132), which may also carry two or more telescopic orientating and holding unit for a pin (9) or a pair of pins (9, 10) or an extending arm (11), the connection of which to the first rod (1) can be achieved through an extending (11) arm, carrying one or more telescopic arms (7), one extending end of which is connected to the telescopic arm (7) and the other extending end is connected to the telescopic arm (7) of another telescopic unit placed on the second rod (132), thus maximising the stability of the system.

53. An external fixation system, according to claims 2, 50 or 52, which is **characterised by** the fact that, the connection of the two rods may be achieved by using a telescopic arm (7) which carries in its end, besides the first radial toothed face (67), a second radial toothed face (116), attaching an orientating and holding mechanism (8) of a pin (9) to one radial toothed face and an extending arm (11) to the other radial toothed face, in a similar way to the one used when placing on the end of the telescopic arm (7) two identical orientating and holding mechanisms (8) of a pin (9).

54. An external fixation system, according to claims 2, 50 or 53, which is **characterised by** the fact that, in case on the end of the telescopic arm (7) is attached a mechanism (8) and an extending arm (11), the end of the extending (11) arm may carry either a holding mechanism (8) with a pin (9) or it may be adjoined to the end of a telescopic arm (7), a telescopic unit of a second rod (132), thus causing, in this way as well, the connection of the two rods, so maximising the stability of the system.

55. An external fixation system, according to claims 2 or 50, 51, 52, 53, 54, which is **characterised by** the fact that, in case the system has only one rod (1), there can be attached to it two or more telescopic units, which hold one pin (9) each, or there can be attached two or more units holding an extension (11), which in turn holds a holding mechanism (8) of a pin (9), or there can be attached two or more units, each holding two pins, (9) and (10), or there can be attached two or more holding units of a pin (9) and an extension which, in turn, holds a holding mechanism of yet one more pin, thus making the system more flexible and adjustable to the needs of the user.

## Patentansprüche

1. Eine teleskopische Ausrichtungs- und Halteeinheit für einen Stift (9) oder ein Stiftpaar oder einen Verlängerungsarm, wobei besagte Einheit auf eine Stange (1) montiert werden kann, und besagte Einheit wie jener Stift oder jenes Stiftpaar oder jener Verlängerungsarm über einen räumlichen 5-gradigen Bewegungsspielraum im Verhältnis zur Stange (1) in Richtung der Pfeile A, B, C, D, E verfügt, wobei A eine Verschiebung entlang der Stangenachse bedeutet, B eine Rotation um die Stangenachse bedeutet, C eine teleskopische Verschiebung senkrecht zur Stangenachse entlang der Achse des Teleskoparms bedeutet, D eine Rotation um die Achse des Teleskoparms, die senkrecht zur Stangenachse steht, bedeutet und E eine Rotation um die Schraubenachse, die senkrecht zur Achse des Teleskoparms steht, bedeutet, und aufgrund der Tatsache, dass die Einheit aus den folgenden Teilen besteht:
a. einer Klemme (2) für die Platzierung entlang und um die Stange ( 1 ) mit einem Axial- und Rotationsfixierungsmechanismus (15, 16, 19) für eine Axialfixierung entlang der Achse (A) und eine Fixierung der Rotation (B) um die Stange (1), welche eine durchgehende Loch-Öffnung (4) aufweist, deren Achse senkrecht zur Achse der Stange ( 1 ) steht, wenn die Einheit auf der Stange montiert ist.
b. einem Teleskopmechanismus, bestehend aus einem axialen Immobilisierungsmechanismus (5) und einem Rotationsregler (6), die sich beide innerhalb eines durchgehenden Lochs oder Öffnung (4) der Klemme (2) befinden, und einem Teleskoparm (7), der mit dem axialen Immobilisierungsmechanismus (5) verbunden ist, zum Zweck der Immobilisierung des Teleskoparms (7) in Richtung (C), der Achse der Öffnung (4), sowie für eine Immobilisierung des Teleskoparms um (D), die Achse der Öffnung (4), mit dem Rotationsregler (6) verbunden ist.
c. einem Ausrichtungs- und Haltemechanismus (8) für den Stift (9) oder den Verlängerungsarm (11) und optional einem zweiten Ausrichtungs- und Haltemechanismus (120) für einen zweiten Stift (10), wobei besagter Mechanismus (8) oder besagte Mechanismen (8, 120) so auf den Teleskoparm (7) montiert wird/werden, dass er/sie um die Achse (E) gedreht werden kann/können, welche senkrecht zur Achse des Teleskoparms (7) verläuft; besagte(r) Ausrichtungs- und Haltemechanismus (8) oder -mechanismen (8, 120) kann/können mittels einer festen Kupplung sicher immobilisiert werden, wenn ihre Endposition erreicht wurde (96, 97), die (Einheit) sich durch folgendes auszeichnet:
Zwischen der Öffnung (4) und dem Rotationsregler (6) befindet sich eine feste Keil (31) - Keilnut (32) - Kupplung, und der Teleskoparm (7) ist mit dem Rotationsregler (6) und dem axialen Immobilisierungsmechanismus mittels eines Paars an radial gezahnten Flächen (34, 39) und einer Schraube (25) verbunden, wodurch eine Immobilisierung der Drehung und der axialen Verschiebung des Teleskoparms (7) und folglich des Ausrichtungs- und Haltemechanismus (8) möglich ist; und jeder Ausrichtungs- und Haltemechanismus (8, 120) ist auf dem Teleskoparm (7) mittels eines Paars an radial gezahnten Flächen (96, 97) und einer Schraube (68) befestigt, wodurch eine Immobilisierung gegen Rotation (E) des besagten Ausrichtungs- und Haltemechanismus (8, 120) bezüglich des besagten Teleskoparms (7) und folglich eine sichere Fixierung des Stifts (9) oder Stiftpaars (9, 10) oder des Verlängerungsarms (11) möglich ist.

2. Ein externes Fixierungssystem, bestehend aus zwei oder mehreren teleskopischen Ausrichtungs- und Halteeinheiten gemäß Anspruch 1, die auf einer Stange (1) positioniert sind, die optional mit einer zweiten Stange (132) verbunden ist, was durch eine Verkupplung zwischen den Teleskoparmen (7), die zu verschiedenen Einheiten gehören, erreicht wird.

3. Eine teleskopische Ausrichtungs- und Halteeinheit für einen Stift (9) oder ein Stiftpaar (9, 10) oder einen Verlängerungsarm (11) gemäß Anspruch 1, die sich durch die Tatsache auszeichnet, dass quer durch die Klemme (2) ein durchgehendes Loch oder eine Öffnung (4) verläuft, sowie durch die Tatsache, dass sie, durch ein durchgehendes Loch oder eine Öffnung (12) eines runden Querschnitts an einem Punkt ihres Körpers, gemäß Pfeil A in Y-Y-Richtung entlang der Stange (1) verschoben oder gemäß Pfeil B auf der Y-Y-Achse um die Stange (1) gedreht werden kann.

4. Eine teleskopische Ausrichtungs- und Halteeinheit für einen Stift (9) oder ein Stiftpaar (9, 10) oder einen Verlängerungsarm (11) gemäß Anspruch 2, die sich durch die Tatsache auszeichnet, dass sowohl das durchgehende Loch oder Öffnung (12) als auch die Stange (1) einen kreisförmigen oder einen nicht-kreisförmigen Querschnitt, wie z.B. einen polygonalen Querschnitt, haben können, der jedoch derartig sein muss, dass er ein Gleiten der Klemme (2) entlang der Stange auf der Y-Y-Achse gemäß Pfeil A und ein Drehen um die Stange ( 1 ) um die Y-Y-Achse gemäß Pfeil B erlaubt, wobei sie sich auch durch die Tatsache auszeichnet, dass die Stange ( 1 ) fest oder nicht fest sein kann. Sie kann z.B. ein Rohr sein oder teleskopische Elemente haben, oder Gelenke oder elastische Medien oder einen Dämpfer etc. oder eine Kombination aus all diesem.

5. Eine teleskopische Ausrichtungs- und Halteeinheit für einen Stift (9) oder ein Stiftpaar (9, 10) oder einen Verlängerungsarm (11) gemäß Anspruch 1, 3 und 4, die sich durch die Tatsache auszeichnet, dass die Klemme (2) aus einem Mechanismus zur Fixierung von Axial- und Rotationsbewegungen (3) entlang der und um die Stange (1) besteht, welcher eine Backe umfasst, deren oberes (15) und unteres (16) Teil durch einen durchgehenden Spalt (17) getrennt sind.

6. Eine teleskopische Ausrichtungs- und Halteeinheit für einen Stift (9) oder ein Stiftpaar (9, 10) oder einen Verlängerungsarm (11) gemäß Anspruch 5, die sich durch die Tatsache auszeichnet, dass sich auf dem unteren Teil (16) der Backe des Mechanismus zur Fixierung von Axialund Rotationsbewegungen (3) der Klemme (2) ein Loch (18) für die Aufnahme einer Schraube befindet, in welches eine Schraube (19) platziert wird, während sich auf dem oberen Teil der Backe (15) des Mechanismus zur Fixierung von Axial- und Rotationsbewegungen (3) der Klemme (2) ein Loch (21) für die Aufnahme einer Schraube (19) befindet, das über ein Innengewinde (22) verfügt und auf der gleichen Achse wie das Loch (18) für die Aufnahme der Schraube (19) des unteren Teils der Backe (16) liegt, sowie durch die Tatsache, dass das Festdrehen der Schraube (19) ein mechanisches Festziehen der beiden Teile der Backe, des oberen (15) und des unteren (16), bewirkt, was zu einer völligen Immobilisierung der Klemme (2) führt und ein Verschieben der Klemme (2) entlang der Stange (1) in Richtung von Pfeil A und ein Drehen um die Stange (1) in Richtung von Pfeil B verhindert.

7. Eine teleskopische Ausrichtungs- und Halteeinheit für einen Stift (9) oder ein Stiftpaar (9, 10) oder einen Verlängerungsarm (11) gemäß Anspruch 1, 3 und 4, die sich durch die Tatsache auszeichnet, dass, damit der Benutzer während einer chirurgischen Operation eine neue Teleskopeinheit zwischen zwei bereits platzierten Einheiten positionieren kann, ohne dazu diese zu entfernen und neu anbringen zu müssen, der Mechanismus zur Fixierung von Axial- und Rotationsbewegungen (3) der Klemme (2) aus zwei unabhängigen Backen bestehen kann, der oberen (15) und der unteren (16), welche aus zwei voneinander unabhängigen Teilen besteht, die an ihrem Ende, das sich diametrisch gegenüber dem Spalt (17) befindet, ein Verbindungsteil haben, (47) für die obere bzw. (48) für die untere, wobei sich in der Mitte beider Teile ein zentrales Loch befindet, (49) bzw. (50), und wobei (49) bzw. (50) zueinander koaxial sind, während durch ihre Mitte ein Achszapfen (46) verläuft, der zwischen den Verbindungsteilen (47) und (48) mittels eines beliebigen, den Fachleuten bekannten, Mittels fixiert wird (wie z.B. mittels Verkeilung, Verschraubung), wodurch eine Verbindung entsteht, welche die Rotation der unteren (16) Backe um den Achszapfen (46), in Richtung von Pfeil F erlaubt, sowie durch die Tatsache, dass das Festdrehen der Schraube (19) ein mechanisches Festziehen der beiden Teile der Backe, des oberen (15) und des unteren (16), bewirkt, was zu einer völligen Immobilisierung der Klemme (2) führt und ein Verschieben der Klemme (2) entlang der Stange (1) in Richtung von Pfeil A und ein Drehen um die Stange (1) in Richtung von Pfeil B verhindert.

8. Eine teleskopische Ausrichtungs- und Halteeinheit für einen Stift (9) oder ein Stiftpaar (9, 10) oder einen Verlängerungsarm (11) gemäß Anspruch 5, 6 oder 7, die sich durch die Tatsache auszeichnet, dass der obere (15) und untere (16) Teil der Backe der Festziehklemme (2) sowie die beiden unabhängigen Backen der Festziehklemme (2) an einer Stelle ihrer inneren Oberfläche ein reibungselastisches Medium (14) oder (53) und (54) tragen, welches dem Benutzer die Arbeit erleichtert, und zwar durch eine vorläufige Verhinderung eines unerwünschten Verrutschens der Festziehklemme (2) entlang der Stange (1) auf der Y-Y-Achse in Richtung Pfeil (A), sowie eines unerwünschten Drehens der Festziehklemme (2) um die Y-Y-Achse der Stange in Richtung Pfeil B, bis vom Benutzer mittels der Verwendung des Mechanismus zur Fixierung der Axial- und Rotationsbewegung (3) die endgültige und exakte Position der bleibenden Positionierung der Klemme (2) eingestellt wurde.

9. Eine teleskopische Ausrichtungs- und Halteeinheit für einen Stift (9) oder ein Stiftpaar (9, 10) oder einen Verlängerungsarm (11) gemäß Anspruch 1, die sich durch die Tatsache auszeichnet, dass der Teleskopmechanismus, bestehend aus einem axialen Immobilisierungsmechanismus (5), einem Rotationsregler (6) und einem Teleskoparm (7), sich innerhalb eines durchgehenden Lochs oder Öffnung (4) befindet und bewegt, welches quer durch die Klemme verläuft (2), durch deren Wand entweder auf ihrer gesamten Länge oder in einem Teilbereich eine Keilnut (32) verläuft, sowie durch die Tatsache, dass die Einführung des gesamten Teleskopmechanismus in das durchgehende Loch oder Öffnung (4) der Klemme (2) durch beide Eingänge des durchgehenden Lochs oder Öffnung möglich ist.

10. Eine teleskopische Ausrichtungs- und Halteeinheit für einen Stift (9) oder ein Stiftpaar (9, 10) oder einen Verlängerungsarm (11) gemäß Anspruch 1 und 9, die sich durch die Tatsache auszeichnet, dass der axiale Rotationsmechanismus (5) aus einer Schraube (25) und zwei Spannringen, einem inneren (26) und einem äußeren (27), besteht, sowie der Tatsache, dass das Paar aus dem inneren (26) und dem äußeren (27) Spannring möglicherweise nicht nur aus einem Paar besteht, sondern aus mehreren, die in Abfolge axial und paarweise verbunden angeordnet sind.

11. Eine teleskopische Ausrichtungs- und Halteeinheit für einen Stift (9) oder ein Stiftpaar (9, 10) oder einen Verlängerungsarm (11) gemäß Anspruch 10, die sich durch die Tatsache auszeichnet, dass die zwei Spannringe, der innere (26) und der äußere (27), des axialen Immobilisierungsmechanismus (5) jeweils eine Kegeligkeit aufweisen, und zwar der innere Spannring (26) eine nach außen weisende Kegeligkeit (28), die sich über seine ganze Länge erstreckt, und ein Loch (43) hat, durch das die Schraube (25) verläuft, während der äußere Spannring (27) eine nach innen weisende Kegeligkeit (29) hat, die sich über seine ganze Länge erstreckt und exakt der nach außen weisenden Kegeligkeit (28) des inneren Rings (26) entspricht, so dass bei der koaxialen Platzierung des inneren Spannrings (26) innerhalb des äußeren Spannrings (17) ein vollständiger Kontakt mindestens eines Teils der nach außen hin kegeligen Oberfläche (28) des inneren Rings (26) mit mindestens einem Teil der nach innen hin kegeligen Oberfläche (29) des äußeren Rings (27) erzielt wird, während der äußere Spannring (27) an einer Stelle seines Umfangs offen sein und einen Spalt (44) aufweisen kann.

12. Eine teleskopische Ausrichtungs- und Halteeinheit für einen Stift (9) oder ein Stiftpaar (9, 10) oder einen Verlängerungsarm (11) gemäß Anspruch 10 oder 11, die sich durch die Tatsache auszeichnet, dass dann, wenn die Schraube (25) in dass Innengewinde (38) des Teleskoparms (7) geschraubt ist, der Schraubenkopf (41) auf den inneren Spannring (26) drückt und ihn in das Innere des äußeren Spannrings (27) drückt, und es auf Grund der umgekehrt kegeligen Form der zwei Oberflächen (28) und (29) und dank des durchgehenden Spalts (44) zu einer Zunahme des Außendurchmessers des äußeren Rings (27) kommt, wodurch die äußere gekurvte Oberfläche (45) des äußeren Rings (27) an die Innenwand des durchgehenden Lochs (4) der Klemme (2) drückt und eine komplette axiale Immobilisierung entlang der X-X-Achse der zwei Spannringe des axialen Immobilisierungsmechanismus (5) und somit des Rotationsreglers (6), mit dem er verbunden ist, und des daran anschließenden Teleskoparms (7) bezüglich der Klemme erzielt.

13. Eine teleskopische Ausrichtungs- und Halteeinheit für einen Stift (9) oder ein Stiftpaar (9, 10) oder einen Verlängerungsarm (11) gemäß Anspruch 12, die sich durch die Tatsache auszeichnet, dass der Rotationsregler (6) eine zylindrische Form hat und sich in seiner Mitte ein durchgehendes Loch (33) befindet, das koaxial zum durchgehenden Loch (43) des inneren Spannrings (26) ist, sowie durch die Tatsache, dass er an seiner Oberfläche mit dem axialen Immobilisierungsmechanismus (5) verbunden ist, während er an seiner Vorderseite eine radial gezahnte Fläche (34) aufweist, in deren innerem Umfang sich eine Vertiefung (35) im Loch (33) befindet, das der Aufnahme eines elastischen Mediums (36) dient, welches dem Benutzer die Arbeit erleichtert, da es vorläufig das komplette Verzahnen der zwei vorderen gezahnten Flächen (39) und (34) des Teleskoparms (7) bzw. des Rotationsreglers (6) verhindert, bis der Benutzer die endgültige Position für ihre exakte definitive Positionierung eingestellt hat.

14. Eine teleskopische Ausrichtungs- und Halteeinheit für einen Stift (9) oder ein Stiftpaar (9, 10) oder einen Verlängerungsarm (11) gemäß Anspruch 1, 9 und 13, die sich durch die Tatsache auszeichnet, dass der Rotationsregler (6) auf einem Teil seiner gekurvten Oberfläche eine Keilnut (30), welche einen Keil (31 ) hält, aufweist, der, wenn mit einem Teil seiner Oberfläche in das Innere der Keilnut (30) eingelegt, innen mit irgendeinem, dem Fachmann bekannten Mittel, fixiert wird (wie Verschraubung, Verklebung, Verkeilung usw.) und so zu einem integrierten Teil von und einem neuen einheitlichen Körper mit dem Rotationsregler (6) wird, was die Rotation des Rotationsreglers (6) um die X-X-Achse des durchgehenden Lochs oder Öffnung der Klemme (2) verhindert.

15. Eine teleskopische Ausrichtungs- und Halteeinheit für einen Stift (9) oder ein Stiftpaar (9, 10) oder einen Verlängerungsarm (11) gemäß Anspruch 1, 9, 13 und 14, die sich durch die Tatsache auszeichnet, dass der Rotationsregler (6) und der Keil (31), die einen neuen, einheitlichen Körper bilden, aneinander anstoßen und entlang ihrer Oberflächen, entlang des durchgehenden Lochs oder Öffnung (4) und der Keilnut (32) gleiten können, welche quer über einen Teil oder die ganze Länge der Wand des durchgehenden Lochs (4) der Klemme (2) verläuft, sowie durch die Tatsache, dass es einen oder mehrere Keile (31) und Keilnuten (32) und Keilnuten (30) geben kann, wobei ihre Zahl jeweils gleich ist, so dass innerhalb jeder Keilnut (32) ein Keil (31) aufliegt und gleitet und innerhalb jeder Keilnut (30) ein Keil (31) fixiert ist.

16. Eine teleskopische Ausrichtungs- und Halteeinheit für einen Stift (9) oder ein Stiftpaar (9, 10) oder einen Verlängerungsarm (11) gemäß Anspruch 1, 9, 13, 14, 15 und 16, die sich durch die Tatsache auszeichnet, dass die Keilnut (30) des Rotationsreglers (6) einen Querschnitt von beliebiger Form, wie etwa kreisförmig, quadratisch usw. haben kann, jedoch immer gleich dem Querschnitt des Keilteils (31), das in sie gesteckt wird, und dass entsprechend die Keilnut (32) der Klemme (2), welche den anderen Teil des Keils (31) aufnimmt, einen Querschnitt von beliebiger Form, wie etwa kreisförmig, quadratisch usw. haben kann, jedoch immer gleich dem Querschnitt des Keilteils (31), das entlang seiner Oberfläche gleitet, so dass das Einstecken eines Teils des Keils (31 ) in die Keilnut (32) immer jegliche Rotationsbewegung der neuen, einheitlichen Komponente, die aus dem Rotationsregler (6) und dem Keil (31) besteht, um die X-X-Achse des durchgehenden Lochs oder Öffnung (4) der Klemme (2) verhindert.

17. Eine teleskopische Ausrichtungs- und Halteeinheit für einen Stift (9) oder ein Stiftpaar (9, 10) oder einen Verlängerungsarm (11) gemäß Anspruch 1, 9, 13, 14, 15 und 16, die sich durch die Tatsache auszeichnet, dass der Rotationsregler (6) und der Keil (31 ) von Beginn an eine einheitliche Komponente mit einem Querschnitt bilden, welcher demjenigen der Verbindung der zwei Teile, des Rotationsreglers (6) und des Keils (31), oder demjenigen der Verbindung des Rotationsreglers (6) mit mehreren Keilen (31) oder dem speziellen Querschnitt der Lücke, die durch das durchgehende Loch oder Öffnung (4) und die Keilnut (32) entsteht, ähnlich ist.

18. Eine teleskopische Ausrichtungs- und Halteeinheit für einen Stift (9) oder ein Stiftpaar (9, 10) oder einen Verlängerungsarm (11) gemäß Anspruch 1, 9, 13 und 14, die sich durch die Tatsache auszeichnet, dass der Querschnitt des Rotationsreglers (6) sowie der Querschnitt der durchgehenden Öffnung oder Lochs (4) der Klemme (2) möglicherweise nicht rund, sonder polygonal oder asymmetrisch usw. ist, und dass der Querschnitt des Rotationsreglers (6) und der Querschnitt der durchgehenden Öffnung oder Lochs (4) sich immer einander entsprechen.

19. Eine teleskopische Ausrichtungs- und Halteeinheit für einen Stift (9) oder ein Stiftpaar (9, 10) oder einen Verlängerungsarm (11) gemäß Anspruch 1, 9 und 13, die sich durch die Tatsache auszeichnet, dass die Keilnut (30) des Rotationsreglers (6), die einen Teil des Keils aufnimmt (31), einen trapezförmigen Querschnitt mit einer schräg verlaufenden Unterseite (55) hat, und zwar so, dass die Tiefe an der Einführseite (59) der Keilnut (30) größer als die Tiefe am Ende (60) der Keilnut (30) ist.

20. Eine teleskopische Ausrichtungs- und Halteeinheit für einen Stift (9) oder ein Stiftpaar (9, 10) oder einen Verlängerungsarm (11) gemäß Anspruch 1, 9, 13 und 19, die sich durch die Tatsache auszeichnet, dass der Keil (31) eine keilförmige Form mit einer schrägen Seite (61), deren Neigung der Neigung der Unterseite (55) der Keilnut (30) entspricht, und einer flachen Seite (62) hat, und dass seine Grundflächen, (58) und (56), ungleich sind, wobei die größere Grundfläche (58) größer oder gleich dem Querschnitt der Öffnung (57) ist, die durch die Keilnut (30) des Rotationsreglers (6) und die Keilnut (32) des durchgehenden Lochs oder Öffnung (4) der Klemme (2) gebildet wird, während die kleinere Grundfläche (56) kleiner als der Querschnitt der Öffnung (57) ist.

21. Eine teleskopische Ausrichtungs- und Halteeinheit für einen Stift (9) oder ein Stiftpaar (9, 10) oder einen Verlängerungsarm (11) gemäß Anspruch 13, 14, 15, 16, 17, 18, 19 und 20, die sich durch die Tatsache auszeichnet, dass das Hineinschrauben der Schraube (25) in das Innengewinde (38) des Teleskoparms (7) im ersten Stadium zu einer Annäherung der radial gezahnten Fläche (39) an die radial gezahnte Fläche (34) führt, und dass das elastische Medium (36), das sich zwischen diesen beiden Komponenten befindet, zusammen gedrückt wird, das eine komplette Verzahnung der zwei radial gezahnten Flächen vorläufig verhindert, sowie der Tatsache, dass, sobald der Benutzer den richtigen Winkel für die endgültige Position des Teleskoparms (7) zum Rotationsregler (6) und zur Klemme (2) eingestellt hat, er die Schraube (25) fester anzieht, was zu einer vollständigen Verzahnung der radial gezahnten Fläche (39) mit der radial gezahnten Fläche (34) führt, wobei gleichzeitig dank der Existenz des Keils (31) eine komplette Verzahnung der radial gezahnten Fläche (34) mit der radial gezahnten Fläche (39) dazu führt, dass die Rotation um die X-X-Achse des axialen Immobilisierungsmechanismus (5), des Rotationsreglers (6) und des Teleskoparms (7), mit dem er verzahnt ist, bezüglich der Klemme (2) verhindert wird.

22. Eine teleskopische Ausrichtungs- und Halteeinheit für einen Stift (9) oder ein Stiftpaar (9, 10) oder einen Verlängerungsarm (11) gemäß Anspruch 20, die sich durch die Tatsache auszeichnet, dass das Hineinschrauben der Schraube (25) den Keil (31), mit der schmaleren (56) seiner zwei Grundflächen zuerst, in die Öffnung (57) hineinschiebt, die durch die Keilnut (30) des Rotationsreglers (6) und die Keilnut (32) der durchgehenden Öffnung (4) gebildet wird, und dass das Festziehen der Schaube (25) zu einer kompletten Verkeilung des Keils (31) in der Öffnung (57) führt, sowie dazu, dass der Keil (31) auf Grund der entstehenden Reibungskräfte sowie seiner Form eine Fixierung der Axial- und Rotationsbewegung jeder einzelnen Komponente bewirkt, sowie in Bezug auf den Rest, d.h. des Rotationsreglers (6) und des Teleskoparms (7), bezüglich der Klemme (2).

23. Eine teleskopische Ausrichtungs- und Halteeinheit für einen Stift (9) oder ein Stiftpaar (9, 10) oder einen Verlängerungsarm (11) gemäß Anspruch 1 und 9, die sich durch die Tatsache auszeichnet, dass der Rotationsregler (6) außen zylinderförmig sein kann und in seiner Mitte eine Öffnung eines durchgehenden Lochs (33) mit Kegelform (66) aufweist und entlang der ganzen Länge seiner Oberfläche von einem länglichen, durchgehenden Spalt (63) durchzogen wird, sowie der Tatsache, dass der Teleskoparm (7) eine zylindrische Form hat und in der Mitte ein blindes Loch (37) für die Einsetzung der Schraube (25) mit einem Innengewinde (38) aufweist, das koaxial zum durchgehenden Loch (33) des Rotationsreglers (6) liegt, und dass der Teleskoparm (7) ein nach außen hin kegelförmiges Ende (64) hat, welches der Kegeligkeit nach innen hin (66) des durchgehenden Lochs (33) des Rotationsreglers (6) entspricht, sowie dass sich an der Spitze des kegeligen Endes (64), ein elastisches Medium (65) befinden kann, wie etwa eine Feder, ein elastischer Ring, usw.

24. Eine teleskopische Ausrichtungs- und Halteeinheit für einen Stift (9) oder ein Stiftpaar (9, 10) oder einen Verlängerungsarm (11) gemäß Anspruch 13, die sich durch die Tatsache auszeichnet, dass das Festziehen der Schraube (25) nacheinander das Zusammendrücken des elastischen Mediums (65), das komplette Ineinanderfügen der kegeligen Oberflächen (64) und (66), das komplette Einführen des kegeligen Endes (64) in das nach innen hin kegelige Ende (33) bewirkt und es wegen der umgekehrten Kegeligkeit der zwei Oberflächen (64) und (66) und dank des durchgehenden Spalts (63), der Spalt (44) des Rings (27) entspricht, zu einer Zunahme des Außendurchmessers des Rotationsregler (6) kommt, was dazu führt, dass seine außen gekurvte Oberfläche gegen die Innenwand des durchgehenden Lochs (4) drückt und, in Kombination mit der Existenz des Keils (31) im Rotationsregler (6), es zu einer kompletten Fixierung der Axial- und Rotationsbewegung entlang der und um die X-X-Achse des Rotationsreglers (6) und des Teleskoparms (7) kommt, mit der er sich verzahnt, und zwar sowohl untereinander als auch bezüglich der Klemme (2).

25. Eine teleskopische Ausrichtungs- und Halteeinheit für einen Stift (9) oder ein Stiftpaar (9, 10) oder einen Verlängerungsarm (11) gemäß Anspruch 1, 9, 13, 21, 22 und 24, die sich durch die Tatsache auszeichnet, dass der Teleskoparm (7) an dem einen Ende, das aus dem durchgehenden Loch oder Öffnung (4) herausragt, eine radial gezahnte Fläche (67) und einen Ausrichtungs- und Haltemechanismus (8) für einen Stift (9) oder auch einen zweiten Stift (10) oder sogar einen Verlängerungsarm (11) hat, während er am anderen Ende, das sich innerhalb des durchgehenden Lochs oder Öffnung (4) befindet, mit einem Rotationsregler (6) verbunden ist.

26. Eine teleskopische Ausrichtungs- und Halteeinheit für einen Stift (9) oder ein Stiftpaar (9, 10) oder einen Verlängerungsarm (11) gemäß Anspruch 1, 13, 21, 22, 24 oder 25, die sich durch die Tatsache auszeichnet, dass der Teleskoparm (7) eine zylindrische Form hat und sich in seiner Mitte ein blindes Loch (37) mit einem Innengewinne (38) für die Einführung der Schraube (25) befindet und dass das blinde Loch (37) koaxial zum durchgehenden Loch (33) des Rotationsreglers (6) liegt.

27. Eine teleskopische Ausrichtungs- und Halteeinheit für einen Stift (9) oder ein Stiftpaar (9, 10) oder einen Verlängerungsarm (11) gemäß Anspruch 1, 9, 21, 22, 25 und 26, die sich durch die Tatsache auszeichnet, dass der Teleskoparm (7), der mit dem Rotationsregler (6) verbunden ist, perimetrisch um das Loch (37) eine radial gezahnte Fläche (39) aufweist, mit der gleichen Anzahl an Zähnchen und einer Geometrie, welche jener der radial gezahnten Fläche (34) des Rotationsreglers (6) entspricht, und dass der innere Umfang der radial gezahnten Fläche (39) eine Vertiefung (40) im blinden Loch (37) aufweist, welche der Aufnahme eines elastischen Mediums (36) dient, das mit seiner einen Seite auf der Oberfläche der Vertiefung (40) des Lochs (37) der radial gezahnten Fläche (39) des Teleskoparms (7) aufliegt, und auf der anderen Seite auf der Oberfläche der Vertiefung (35) im Loch (33) der radial gezahnten Fläche (34) des Rotationsreglers (6) aufliegt.

28. Eine teleskopische Ausrichtungs- und Halteeinheit für einen Stift (9) oder ein Stiftpaar (9, 10) oder einen Verlängerungsarm (11) gemäß Anspruch 1, 9, 13, 14, 21, 22, 25, 26 und 27, die sich durch die Tatsache auszeichnet, dass die Zähnchen sowohl der radial gezahnten Fläche (34) des Rotationsreglers (6) und der radial gezahnten Fläche (39) des Teleskoparms (7) so gefertigt sind, dass entlang ihrer gesamten Oberfläche die Zähnchen jeder der gezahnten Flächen sich exakt zwischen die Zähnchen der jeweils gegenüber liegenden radial gezahnten Fläche einfügen und zu liegen kommen, so dass eine Rotationsbewegung des Teleskoparms bezüglich des Reglers (6) und der Klemme (2) verhindert wird.

29. Eine teleskopische Ausrichtungs- und Halteeinheit für einen Stift (9) oder ein Stiftpaar (9, 10) oder einen Verlängerungsarm (11) gemäß Anspruch 1, 9 und 25, die sich durch die Tatsache auszeichnet, dass der Ausrichtungs- und Haltemechanismus (8) für einen Stift (9) oder auch einen Verlängerungsarm (11) aus einer Schraube (68), zwei Backen, der oberen (72) und der unteren (73), einer Feder (74) zwischen den zwei Backen, einem elastischen Ring (75) und einer Festziehmutter (76) besteht, und dass der Ausrichtungs- und Haltemechanismus einen Mechanismus zur vorläufigen Stabilisierung der Stange tragen kann (9).

30. Eine teleskopische Ausrichtungs- und Halteeinheit für einen Stift (9) oder ein Stiftpaar (9, 10) oder einen Verlängerungsarm (11) gemäß Anspruch 1, 9, 25 und 29, die sich durch die Tatsache auszeichnet, dass die radial gezahnte Fläche des Teleskoparms (7) auf einer Ebene liegt, die zur Ebene der radial gezahnten Fläche (39), die der Teleskoparm (7) auf seinem anderen Ende aufweist, also dem Ende innerhalb des durchgehenden Lochs oder Öffnung (4) der Klemme (2), einen Winkel bildet, und dass sich unter der radial gezahnten Fläche (67) ein Loch (128) mit einer Verengungsstelle für das Halten des vorläufigen Stabilisierungsmechanismus befindet.

31. Eine teleskopische Ausrichtungs- und Halteeinheit für einen Stift (9) oder ein Stiftpaar (9, 10) oder einen Verlängerungsarm (11) gemäß Anspruch 1 und 30, die sich durch die Tatsache auszeichnet, dass die Schraube (68) durch ein durchgehendes Loch (98) geht, welches durch die Mitte der radial gezahnten Fläche (67) des Teleskoparms (7) verläuft und ein Gewinde (107) aufweist, und auf einem Teil ihres Kopfes (69) eine perimetrische Rille (70) aufweist, in welche ein elastischer Ring (71) platziert wird, während sie auf einem Teil ihres Körpers vor dem Gewinde eine perimetrische Rille (127) für die vorläufige Stabilisierung der Schraube (68) aufweisen kann, und während sich am Ende der Schraube ein Abschraub-Limiter (79) wie etwa eine Halteschraube, eine kleine Schraube usw. befinden kann, der in einer geeigneten Weise, wie etwa Verschraubung oder Verklebung etc. auf das Ende (106) der Schraube (68) angebracht wird.

32. Eine teleskopische Ausrichtungs- und Halteeinheit für einen Stift (9) oder ein Stiftpaar (9, 10) oder einen Verlängerungsarm (11) gemäß Anspruch 30 oder 31, die sich durch die Tatsache auszeichnet, dass die obere Backe (72) auf einem Teil ihrer Oberfläche ein durchgehendes Loch (80) aufweist, an dessen Öffnungsstelle sich eine Vertiefung (82) für den Kopf (69) der Schraube (68) befinden kann, und dass sie auf ihrer unteren Seite (84) eine Halte-Rille (86) für einen Stift (9) und eine Vertiefung (88) aufweist und außerdem eine Vertiefung (85) im Loch (80) für die Aufnahme der Feder (74) aufweisen kann, mit deren Hilfe die zwei Backen (72) und (73) auf Distanz gehalten werden, so dass das Einlegen des Stiftes (9) zwischen die Rillen (86) und (91 ) der oberen bzw. der unteren Backe erleichtert wird.

33. Eine teleskopische Ausrichtungs- und Halteeinheit für einen Stift (9) oder ein Stiftpaar (9, 10) oder einen Verlängerungsarm (11) gemäß Anspruch 31 oder 32, die sich durch die Tatsache auszeichnet, dass die untere Backe (73) auf ihrer Oberseite (89) eine Rille (91) für einen Stift (9) aufweist, und zwar gegenüber der Rille (86) der oberen Backe (72), sowie an einer Stelle ihrer Oberfläche ein durchgehendes Loch (81), und dass sie auf ihrer oberen Seite (89) eine Vertiefung (90) im Loch (81) für die Aufnahme der Feder (74) aufweisen kann, während sie auf ihrer unteren Seite eine Vertiefung (97) aufweisen kann, in welche ein elastischer Körper (75) platziert wird, welcher vorläufig ein komplettes Verzahnen der beiden radial gezahnten Flächen (67) und (96) verhindert, während beim Anziehen der Schraube (68) der elastische Körper (75) zusammen gedrückt wird, so dass ein kompletter Kontakt der beiden radial gezahnten Flächen hergestellt wird.

34. Eine teleskopische Ausrichtungs- und Halteeinheit für einen Stift (9) oder ein Stiftpaar (9, 10) oder einen Verlängerungsarm (11) gemäß Anspruch 30, 31, 32 oder 33, die sich durch die Tatsache auszeichnet, dass die untere Backe (73) auf ihrer oberen Seite (89) einen Vorsprung (93) aufweist, welcher der Vertiefung (88) in der unteren Seite (84) der oberen Backe (72) entspricht, und dass die Höhe des Vorsprungs (93) und die Tiefe der Vertiefung (88) derartig sein können, dass zwischen der unteren Seite (84) der oberen Backe (72) und der oberen Seite (89) der unteren Backe (73) eine Lücke (94) entsteht, sowie dass die Vertiefung (88) und der Vorsprung (93) sich in umgekehrter Position auf der unteren bzw. der oberen Backe befinden können.

35. Eine teleskopische Ausrichtungs- und Halteeinheit für einen Stift (9) oder ein Stiftpaar (9, 10) oder einen Verlängerungsarm (11) gemäß Anspruch 30, 31, 32, 33 oder 34, die sich durch die Tatsache auszeichnet, dass die untere Backe (73) auf ihrer Unterseite (95) eine radial gezahnte Fläche (96) aufweist, welche perimetrisch zum Loch (81 ) liegt, mit einer gleichen Anzahl an Zähnchen und einer Geometrie, welche derjenigen der radial gezahnten Fläche (67) des Teleskoparms (7) entspricht, und dass die beiden radial gezahnten Flächen, also die radial gezahnte Fläche (96) der unteren Backe (73) und die radial gezahnte Fläche (67) des Teleskoparms (7) so gefertigt sind, dass auf der ganzen Länge ihrer Oberflächen ihre Zähnchen sich jeweils zwischen die Zähnchen der gegenüber liegenden radial gezahnten Fläche einfügen und komplett an diesen aufliegen.

36. Eine teleskopische Ausrichtungs- und Halteeinheit für einen Stift (9) oder ein Stiftpaar (9, 10) oder einen Verlängerungsarm (11) gemäß Anspruch 30 oder 35, die sich durch die Tatsache auszeichnet, dass die Festziehmutter (76) einen Kopf (101) trägt, der an seiner Unterseite eine Fassung oder einen Griff (102) aufweist, geeignet für ein Festschraub- bzw. Aufschraubwerkzeug oder auch für ein anfängliches Festziehen per Hand, sowie eine perimetrische Rille (77), in die ein elastischer Ring (78) gelegt wird, dank dessen vorläufig die freie Rotation der Festziehmutter (76) auf Grund der zwischen dem elastischen Ring (78) und der inneren Wand der Oberfläche der Vertiefung (100) des Lochs (98) entstehenden Reibung verhindert wird, und dass sich in der Mitte der Festziehmutter (76) ein durchgehendes Loch (103) befindet, das die Festziehungsmutter (76) in ihrer ganzen Länge durchdringt, während an einer Stelle des durchgehenden Lochs (103) ein Innengewinde (104) beginnt, welches sich bis zum Ende des durchgehenden Lochs (103) erstreckt und dessen Durchmesser kürzer als der Durchmesser des Aufschraub-Limiters (79) ist, so dass ein Durchgehen und folglich ein Durchrutschen der Schraube (68) durch die Mutter (76) hinaus verhindert wird.

37. Eine teleskopische Ausrichtungs- und Halteeinheit für einen Stift (9) oder ein Stiftpaar (9, 10) oder einen Verlängerungsarm (11) gemäß Anspruch 29, die sich durch die Tatsache auszeichnet, dass der Mechanismus zur vorläufigen Stabilisierung aus einer Spitze (124), welche eine Verbreiterung (129) aufweisen kann, während sich an ihrem Ende, das in das Loch (128) eingeführt wird, eine kegel- oder keilförmige oder sonst wie gekurvte Konfiguration (130) befindet, welche der einer Rille (127) entspricht, in welche sie eindringt, wenn sich die Schraube (68) in einer geeigneten Position befindet, aus einem elastischen Druckmittel (125) wie einer Feder, einer elastischen Kugel usw., welches einen ständigen Druck auf die Spitze (124) ausübt, die dazu tendiert, in Richtung Schraube zu gleiten, sowie aus einem Stopper (126) bestehen kann, der von Beginn an mit einem dem Fachmann bekannten Mittel (wie z.B. Verschraubung, Verkeilung) an einer festen Position fixiert ist, und dass die Spitze (124), das elastische Druckmittel (125) und der Stopper (126) in das Loch (128) des Teleskoparms (7) platziert werden.

38. Eine teleskopische Ausrichtungs- und Halteeinheit für einen Stift (9) oder ein Stiftpaar (9,10) oder einen Verlängerungsarm (11) gemäß Anspruch 37, die sich durch die Tatsache auszeichnet, dass der Benutzer sobald er den Stift (9) in den wieder herzustellenden Knochen implantiert hat, er den Stift, um ihn innerhalb der Teleskopeinheit zu platzieren, zwischen der Rille (91) und der gegenüber liegenden Rille (86) einpasst, und dass der Benutzer, wenn in das System ein Mechanismus für die vorläufige Stabilisierung eines Stifts (9) platziert wurde, in diesem Stadium eine vorläufige Immobilisierung des Stifts (9) erreichen kann, indem er einfach Druck auf die Schraube (68) ausübt, so dass die Spitze (124) in die Rille (127) der Schraube (68) hinein geschoben wird, während sich das Aushaken des Stifts (9) aus dem Haltemechanismus (8) durch die Anwendung einer umgekehrten Kraft erreichen lässt.

39. Eine teleskopische Ausrichtungs- und Halteeinheit für einen Stift (9) oder ein Stiftpaar (9, 10) oder einen Verlängerungsarm (11) gemäß Anspruch 36, 37 oder 38, die sich durch die Tatsache auszeichnet, dass der Benutzer, um zu erreichen, dass die Teleskopeinheit den Stift (9) festhält, entweder die Schraube (68) in der Festziehmutter (76) auswählt und festzieht, oder umgekehrt - da die Einheit dank des elastischen Rings der Mutter (76) und der Schraube (68) es dem Benutzer freistellt zu entscheiden, nur eine der beiden Komponenten festzuziehen, zu denen er Zugang hat, ohne dazu gleichzeitig auch die andere immobilisieren zu müssen, wobei jeweils das gleiche Ergebnis erzielt wird - so dass die Feder (74) zusammen gedrückt wird und sich somit die beiden Backen, die untere (72) und die obere (73), einander annähern, sowie dass der Benutzer, sobald er den richtigen Winkel für die Endposition des Mechanismus (8) in Bezug zum Teleskoparm (7) und der Klemme (2) eingestellt hat, er die Schraube (68) in der Festziehmutter (76) fester zieht, so dass die zwei radial gezahnten Flächen, (67) und (96), sich nach und nach komplett verzahnen und gleichzeitig die zwei Backen (71 ) und (73) ganz angezogen werden, und zwar nicht nur um den Stift (9) und gegeneinander, sondern auch gegen den Teleskoparm (7), wodurch eine komplette Immobilisierung des Stifts (9) in Bezug auf den Teleskoparm (7) und somit die Klemme (2) erreicht wird.

40. Eine teleskopische Ausrichtungs- und Halteeinheit für einen Stift (9) oder ein Stiftpaar (9, 10) oder einen Verlängerungsarm (11) gemäß Anspruch 1, 9, 25, 29, 30, 31, 32, 33, 34, 35, 36 und 37, die sich durch die Tatsache auszeichnet, dass die Länge des Teleskoparms (7) durch Anbringung des Verlängerungsarm (11)-Teils verlängert werden kann, an dessen beiden Enden sich Oberflächen, (108) und (109), mit einer radial gezahnten Fläche befinden, und zwar auf einer solchen Ebene, dass die Achse des Arms auf einer Ebene mit den Oberflächen liegt, welche zueinander eine gegenteilige oder eine gleiche oder eine andere beliebige Ausrichtung haben.

41. Eine teleskopische Ausrichtungs- und Halteeinheit für einen Stift (9) oder ein Stiftpaar (9, 10) oder einen Verlängerungsarm (11) gemäß Anspruch 40, die sich durch die Tatsache auszeichnet, dass die zwei radial gezahnten Flächen (108) und (109) des Verlängerungsarms (11) in ihrer Mitte ein Loch, (110) bzw. (111), sowie jeweils eine Vertiefung, (112) bzw. (113), für zwei elastische Ringe, (75) bzw. (114), haben, und sich am Ausgang der durchgehenden Löcher (110) und (111) eine Vertiefung, (131) bzw. (115), für die Aufnahme der Köpfe (101) und (101) der zwei Festziehmuttern (76) und (76) befindet, sowie dass die Verwendung von zwei elastischen Ringen (75) und (114), bis der Benutzer die Schraube (68) komplett angezogen hat, vorläufig ein komplettes Verzahnen der zwei radial gezahnten Oberflächen (67) und (108) sowie der zwei radial gezahnten Oberflächen (109) und (96) verhindert.

42. Eine teleskopische Ausrichtungs- und Halteeinheit für einen Stift (9) oder ein Stiftpaar (9, 10) oder einen Verlängerungsarm (11) gemäß Anspruch 40 oder 41, die sich durch die Tatsache auszeichnet, dass für die Anbringung des Verlängerungsarms (11) am Teleskoparm (7) sich die radial gezahnte Fläche (108) des Verlängerungsarms (11) mit der radial gezahnten Fläche (67) des Teleskoparms (7) verzahnt, wobei in der gleichen Art und Weise die gleichen Teile wie für die Anbringung des Ausrichtungs- und Haltemechanismus (8) für den Stift (9) auf den Teleskoparm (7) verwendet werden, d.h. eine Schraube (68) mit einem Gewinde (107) und einer perimetrischen Rille (70), ein elastischer Ring (71), eine Festziehmutter (76) mit einem elastischen Ring (78) und ein Mechanismus für die vorläufige Stabilisierung.

43. Eine teleskopische Ausrichtungs- und Halteeinheit für einen Stift (9) oder ein Stiftpaar (9, 10) oder einen Verlängerungsarm (11) gemäß 40, 41 oder 42, die sich durch die Tatsache auszeichnet, dass dann, wenn der Verlängerungsarm (11) auf den Teleskoparm (7) angebracht ist, der Ausrichtungs- und Haltemechanismus (8) für einen Stift (9) auf einer zweiten radial gezahnten Fläche (109) des Verlängerungsarms (11) angebracht wird, sowie dass während der Verwendung des Verlängerungsarms (11) die Immobilisierung des Stifts (9) in Bezug auf die Komponente des Verlängerungsarms (11) und folglich die Klemme (2) durch die Verwendung eines Haltemechanismus (8) in exakt der gleichen Art und Weise erreicht wird, wie der Stift (9) in dem Fall immobilisiert wird, dass der Verlängerungsarm (11) nicht benutzt wird, und auch die Immobilisierung des Verlängerungsarms (11) in Bezug auf den Teleskoparm (7) und folglich die Klemme (2) in exakt der gleichen Art und Weise erreicht wird, wie der Ausrichtungs- und Haltemechanismus (8) in Bezug auf den Teleskoparm (7) immobilisiert wird

44. Eine teleskopische Ausrichtungs- und Halteeinheit für einen Stift (9) oder ein Stiftpaar (9, 10) oder einen Verlängerungsarm (11) gemäß Anspruch 1, 9, 25, 29, 30, 31, 32, 33, 34, 35, 36, 37, 40, 41, 42 und 43, die sich durch die Tatsache auszeichnet, dass der Teleskoparm (7) an dem Ende, das aus der durchgehenden Öffnung oder Loch (4) herausragt und das den Ausrichtungs- und Haltemechanismus für einen Stift (9) oder auch einen Verlängerungsarm (11) trägt, eine zweite radial gezahnte Fläche (116) auf einer zur ersten radial gezahnten Fläche (67) parallelen Ebene aufweisen kann, so dass er ein identisches Paar an Ausrichtungs- und Haltebacken (120) für einen zweiten Stift (10) aufnehmen kann, das aus genau den gleichen Komponenten wie das erste Paar an Ausrichtungs- und Haltebacken (8) für einen Stift (9) besteht.

45. Eine teleskopische Ausrichtungs- und Halteeinheit für einen Stift (9) oder ein Stiftpaar (9, 10) oder einen Verlängerungsarm (11) gemäß Anspruch 44, die sich durch die Tatsache auszeichnet, dass die Anbringung eines zweiten, identischen Paars an Ausrichtungs- und Haltebacken (120) für einen zweiten Stift (10) auf der zweiten radial gezahnten Fläche (116) in genau der gleichen Art und Weise erreicht wird, wie das erste Paar an Ausrichtungs- und Haltebacken (8) für den ersten Stift (9) auf der ersten radial gezahnten Fläche (67) platziert wird.

46. Eine teleskopische Ausrichtungs- und Halteeinheit für einen Stift (9) oder ein Stiftpaar (9, 10) oder einen Verlängerungsarm (11) gemäß Anspruch 1, 44 oder 45, die sich durch die Tatsache auszeichnet, dass der Benutzer, um zu erreichen, dass die zwei Haltemechanismen (8) bzw. (120) die zwei Stifte (9) und (10) festhalten, sobald der Benutzer die zwei Stifte (9) und (10) in den wieder herzustellenden Knochen implantiert hat, er diese, um sie in die Teleskopeinheit zu platzieren, zwischen die Rillen (91) und (86) des ersten, sowie die Rillen (121) und (122) des zweiten Haltemechanismus positioniert, und dass der Benutzer entweder die Schraube (68) in der Festziehmutter (76) auswählt und festzieht, oder umgekehrt die Festziehmutter (76) um die Schraube (68) festzieht - da die Einheit dank des elastischen Rings in der Mutter (76) und der Schraube (68) es dem Benutzer freistellt zu entscheiden, nur eine der beiden Komponenten festzuziehen, zu denen er Zugang hat, ohne dazu gleichzeitig auch die andere immobilisieren zu müssen, wobei jeweils das gleiche Ergebnis erzielt wird - so dass die zwei Federn (74) und (117) der zwei Haltemechanismen (8) bzw. (120) gleichzeitig zusammen gedrückt werden und sich somit die beiden Backen, die obere (72) und die untere (73), des ersten Haltemechanismus (8) sowie gleichzeitig die zwei Backen, die obere (118) und die untere (119) des zweiten Mechanismus (120), einander annähern, und zwar nicht nur untereinander, sondern auch in Bezug auf den Teleskoparm (7), wodurch nicht nur der erste Stift (9) zwischen den Rillen (86) und (91 ) des ersten Mechanismus (8), sondern auch der zweite Stift (10) zwischen den Rillen (121) und (122) des zweiten Mechanismus (120) festgezogen wird.

47. Eine teleskopische Ausrichtungs- und Halteeinheit für einen Stift (9) oder ein Stiftpaar (9, 10) oder einen Verlängerungsarm (11) gemäß Anspruch 1, 44, 45 oder 46, die sich durch die Tatsache auszeichnet, dass, damit die zwei Stifte (9) und (10) komplett von den zwei Haltemechanismen (8) und (120) gehalten werden, wenn der Benutzer die Schraube (68) fester in die Festziehmutter (76) hineinschraubt, die zwei elastischen Ringe (75) und (92), die sich an je einer Seite des Teleskoparms (7) befinden und zwischen den Teleskoparm und die zwei Haltemechanismen (8) bzw. (120) platziert werden, zusammen gedrückt werden, die vorläufig ein komplettes Verzahnen der zwei Paare an radial gezahnten Flächen, (67) und (69) des ersten Mechanismus, und (116) und (123) des zweiten Mechanismus (120) verhindern, sowie dass der Benutzer, sobald er den korrekten Winkel der Endposition der zwei Mechanismen (8) und (120) zum Teleskoparm (7) eingestellt hat, die Schraube (68) in der Mutter (76) fester anzieht, und durch den durch das Festziehen der Schraube (68) zur Mutter (76) des ersten Backenpaars (72) und (73) ausgeübten Druck dieses Backenpaar festgezogen wird, sowie gleichzeitig auch das zweite Backenpaar (118) und (119) festgezogen wird, und zwar nicht nur jeweils zueinander, sondern auch gegen den Teleskoparm (7), was zu einer kompletten Verzahnung der zwei Paare radial gezahnter Flächen, also einerseits (96) und (67) und andererseits (123) und (116), führt. Als Ergebnis des Festziehens der vier Backen gegen den Teleskoparm kommt es zu einer kompletten Immobilisierung nicht nur der zwei Backenpaare, sondern auch der zwei Stifte (9) und (10) in Bezug auf den Teleskoparm (7) und die Klemme (2).

48. Eine teleskopische Ausrichtungs- und Halteeinheit für einen Stift (9) oder ein Stiftpaar (9, 10) oder einen Verlängerungsarm (11) gemäß Anspruch 1, 44 und 45, die sich durch die Tatsache auszeichnet, dass auf dem Ende des Teleskoparms (7), der aus dem durchgehenden Loch (4) herausragt und an welches die zwei Ausrichtungsmechanismen für einen Stift angeschlossen werden, in der gleichen Art und Weise auf der einen Seite (67) des Teleskoparms (7) ein Ausrichtungs- und Haltemechanismus (8) für einen Stift (9), und auf der anderen Seite (116) des Teleskoparms (7) ein Verlängerungsarm (11) angebracht und festgezogen werden können, oder umgekehrt.

49. Eine teleskopische Ausrichtungs- und Halteeinheit für einen Stift (9) oder ein Stiftpaar (9, 10) oder einen Verlängerungsarm (11) gemäß Anspruch 32, 33, 34, 35, 36 oder 39, 40, 41, 42, 43, 44, 45, 46, 47, 48, die sich durch die Tatsache auszeichnet, dass bei allen Anwendungsmöglichkeiten der vorliegenden Erfindung die Rillen (86) und (91) einen runden oder einen dreieckigen Querschnitt haben können, und zwar entweder eine oder einige oder alle Rillen, da eine solche Variation es dem Benutzer erlaubt, Stifte von verschiedenem Durchmesser einzulegen, sowie dass in dem Fall, dass eine von ihnen einen dreieckigen Querschnitt hat, die Druck-Oberfläche (87) des Kopfs (69) der Schraube (68) Teil einer Kugel sein kann und die Oberfläche der Vertiefung (82) eine entsprechende Form haben muss, so dass eine gleichmäßige Belastung der Druck-Oberfläche (87) des Kopfes (69) der Schraube (68) bei jedem Winkel, den die obere Backe (72) zur unteren Backe (73) einnehmen kann, erreicht wird.

50. Ein externes Fixierungssystem gemäß Anspruch 2, das sich durch die Tatsache auszeichnet, dass in dem Fall, dass das System auch eine zweite Stange (132) umfasst, diese ebenfalls zwei oder mehr teleskopische Ausrichtungs- und Halteeinheiten (8) für einen Stift (9) oder ein Stiftpaar (9, 10) oder einen Verlängerungsarm (11) trägt, und mit der ersten Stange (1) mittels einer paarweisen Direktkopplung der radial gezahnten Flächen von zwei oder mehr Teleskoparmen (7) verbunden werden kann, d.h. eine der ersten Stange (1) mit jeweils einer der zweiten Stange (2), so dass eine maximale Stabilität des Systems gewährleistet wird.

51. Ein externes Fixierungssystem gemäß Anspruch 2 oder 50, das sich durch die Tatsache auszeichnet, dass das System für die direkte Verbindung eines jeden Paars der Teleskoparme (7), d.h. einen der ersten Stange (1) mit einem der zweiten Stange (132), die radial gezahnte Fläche (67), die jeder Teleskoparm an seinem Ende aufweist, mittels einer Schraube (68) direkt gegen die radial gezahnte Fläche (67) des anderen Teleskoparms (7) angezogen wird, was außerdem zu einem Zusammendrücken des elastischen Mediums (75) führt, das sich möglicherweise zwischen den zwei radial gezahnten Flächen (67) der zwei Teleskoparme (7) befindet, das auf ihrer Seite auf die Oberfläche einer Vertiefung (99) in ein Loch (98) gelegt wird, welches die radial gezahnte Fläche (67) eines jeden Teleskoparms (7) aufweist.

52. Ein externes Fixierungssystem gemäß Anspruch 2 oder 50, das sich durch die Tatsache auszeichnet, dass das System eine zweite Stange (132) umfassen kann, welche ebenfalls zwei oder mehr teleskopische Ausrichtungs- und Halteeinheiten für einen Stift (9) oder ein Stiftpaar (9, 10) oder einen Verlängerungsarm (11) trägt, deren Verbindung mit der ersten Stange (1) durch einen Verlängerungsarm (11) erfolgen kann, der einen oder mehrere Teleskoparme (7) trägt, von dem das eine herausragende Ende mit dem Teleskoparm (7) und das andere herausragende Ende mit dem Teleskoparm (7) einer anderen, auf einer zweiten Stange (132) angebrachten Teleskopeinheit verbunden wird, so dass eine maximale Stabilität des Systems gewährleistet wird.

53. Ein externes Fixierungssystem gemäß Anspruch 2, 50 oder 52, das sich durch die Tatsache auszeichnet, dass die Verbindung von zwei Stangen durch die Verwendung eines Teleskoparms (7) erreicht werden kann, der an seinen Enden außer der ersten radial gezahnten Fläche (67) noch eine zweite radial gezahnte Fläche (116) aufweist, wobei an eine der radial gezahnten Flächen ein Ausrichtungs- und Haltemechanismus (8) für einen Stift (9) und an die andere gezahnte Fläche ein Verlängerungsarm (11) angebracht wird, und zwar in einer ähnlichen Art und Weise, wie wenn an das Ende des Teleskoparms (7) zwei identische Ausrichtungsund Haltemechanismen (8) für einen Stift angebracht werden.

54. Ein externes Fixierungssystem gemäß Anspruch 2, 50 oder 53, das sich durch die Tatsache auszeichnet, dass, falls am Ende des Teleskoparms (7) ein Mechanismus (8) und ein Verlängerungsarm (11) angebracht werden, das Ende des Verlängerungsarms (11) entweder einen Haltemechanismus (8) mit einem Stift (9) tragen kann oder an das Ende eines Teleskoparms (7) eine Teleskopeinheit einer zweiten Stange (132) angeschlossen werden kann, so dass auch auf diese Art und Weise die Verbindung von zwei Stange erreicht wird, was eine maximale Stabilität des Systems gewährleistet.

55. Ein externes Fixierungssystem gemäß Anspruch 2 oder 50, 51, 52, 53, 54, das sich durch die Tatsache auszeichnet, dass in dem Fall, dass das System nur eine Stange (1) hat, an diese zwei oder mehr Teleskopeinheiten angebracht werden können, die jeweils einen Stift (9) halten, oder an diese zwei oder mehr Einheiten angebracht werden können, die einen Verlängerungsarm (11) halten, der wiederum einen Haltemechanismus (8) für einen Stift (9) hält, oder dass an dieser zwei oder mehr Einheiten angebracht werden können, die jeweils zwei Stifte (9) und (10) halten, oder an dieser zwei oder mehr Halteeinheiten für einen Stift (9) und ein Verlängerungsarm angebracht werden können, der wiederum einen Haltemechanismus für einen weiteren Stift hält, so dass dieses System flexibler wird und sich besser an die Bedürfnisse des Benutzers anpassen lässt.

## Revendications

1. Une unité télescopique d'orientation et de maintien pour une broche (9) ou même une paire de broches ou même un bras extensible, la dite unité étant montable sur une barre (1), la dite unité étant telle que la broche ou la paire de broches ou le bras extensible dispose de cinq degrés de liberté de mouvement dans l'espace par rapport à la barre (1) suivant les flèches A, B, C, D, E où A indique la translation le long de l'axe de la barre, B indique la rotation autour de l'axe de la barre, C indique la translation télescopique perpendiculaire à l'axe de la barre, le long de l'axe du bras télescopique, D indique la rotation autour de l'axe du bras télescopique, qui est perpendiculaire à l'axe de la barre, E indique la rotation autour de l'axe de la vis, qui est perpendiculaire à l'axe du bras télescopique et conforme au fait qu'elle est constituée de :
a. une pince (2) à placer le long et autour de la barre (1), avec un mécanisme de fixation axial et pivotant autour (B) de la barre (1), percé d'un trou traversant (4) dont l'axe est perpendiculaire à l'axe de la barre (1) lorsque l'unité est montée sur la barre.
b. un mécanisme télescopique, constitué d'un mécanisme d'immobilisation axiale (5) et d'un régulateur de rotation (6), tous deux situés à l'intérieur d'un trou traversant ou ouverture (4) de la pince (2), et un bras télescopique (7) connecté au mécanisme d'immobilisation axiale (5) pour l'immobilisation du bras télescopique (7) le long (C) de l'axe de l'ouverture (4), et connecté au régulateur de rotation (6) pour l'immobilisation du bras télescopique (7) autour (D) de l'axe de l'ouverture (4).
c. un mécanisme d'orientation et de maintien (8) de la broche (9) ou du bras extensible (11) et, en option, un second mécanisme d'orientation et de maintien (120) pour une deuxième broche (10), le dit mécanisme (8) ou les dits mécanismes (8, 120) étant montés sur le bras télescopique (7) de façon à pouvoir pivoter autour de l'axe (E) qui est perpendiculaire à l'axe du bras télescopique (7) ; le dit mécanisme (8) ou les dits mécanismes (8, 120) d'orientation et de maintien peuvent être solidement immobilisés, lorsque la position définitive est déterminée, au moyen d'un couplage rigide (96, 97),
Avec les caractéristiques suivantes :
il y a un couplage rigide avec clavette (31) - rainure (32) entre l'ouverture (4) et le régulateur de rotation (6) ; le bras télescopique (7) est connecté au régulateur de rotation (6) et au mécanisme d'immobilisation axiale via une paire de faces dentées et radiales (34, 39) et une vis (25), afin de permettre l'immobilisation rotative et axiale du bras télescopique (7) et par conséquent du mécanisme d'orientation et de maintien (8), et chaque mécanisme d'orientation et de maintien (8, 120) est monté sur le bras télescopique (7) via une paire de faces dentées et radiales (96, 97) et une vis (68), pour empêcher la rotation (E) du dit mécanisme d'orientation et de maintien (8, 120) par rapport au dit bras télescopique (7) et par conséquent fixer solidement la broche (9) ou paire de broches (9, 10) ou le bras extensible (11).

2. Un mécanisme de fixation externe, constitué de deux unités télescopiques d'orientation et de maintien ou davantage, conformes à la revendication 1, positionné sur une barre (1) facultativement connectée à une deuxième barre (132) au moyen d'un couplage entre les bras télescopiques (7) appartenant à des unités différentes.

3. Une unité télescopique d'orientation et de maintien pour une broche (9) ou une paire de broches (9, 10) ou un bras extensible (11), conforme à la revendication 1, **caractérisée par le fait que**, la pince (2) est percée d'un trou traversant ou ouverture (4) et **par le fait que**, à travers un trou traversant ou ouverture (12) d'une section située à certain point de son corps, elle a la possibilité de coulisser le long de la barre (1) sur l'axe Y-Y' indiqué par la flèche A et de pivoter autour de la barre (1), sur l'axe Y-Y' indiqué par la flèche B.

4. Une unité télescopique d'orientation et de maintien pour une broche (9) ou une paire de broches (9, 10) ou un bras extensible (11), conforme à la revendication 2, **caractérisée par le fait que**, le trou traversant ou ouverture (12) et la barre (1) peuvent tous deux avoir une section circulaire ou non circulaire, par exemple polygonale, etc., qui doit cependant être telle qu'elle permettra à la pince (2) de coulisser le long de la barre (1) sur l'axe Y-Y' indiqué par la flèche A et de pivoter autour de la barre (1), sur l'axe Y-Y' indiqué par la flèche B, tout en étant également **caractérisée par le fait que** la barre (1) peut être pleine ou non, elle peut par exemple être tubulaire ou même comporter des éléments télescopiques, ou des joints ou des agents élastiques ou un amortisseur, etc., ou une combinaison de ces éléments.

5. Une unité télescopique d'orientation et de maintien pour une broche (9) ou une paire de broches (9, 10) ou un bras extensible (11), conforme aux revendications 1, 3 et 4, **caractérisée par le fait que**, la pince (2) est constituée d'un mécanisme de fixation du mouvement axial et pivotant (3) le long de et autour de la barre (1) qui comprend une mâchoire, dont les parties supérieure (15) et inférieure (16) sont séparées par une fente transversale (17).

6. Une unité télescopique d'orientation et de maintien pour une broche (9) ou une paire de broches (9, 10) ou un bras extensible (11), conforme à la revendication 5, **caractérisée par le fait que**, la partie inférieure (16) de la mâchoire du mécanisme de fixation du mouvement axial et pivotant (3) de la pince (2) comporte un trou (18) destiné au maintien d'une vis, à l'intérieur duquel une vis (19) est placée, tandis que la partie supérieure de la mâchoire (15) du mécanisme de fixation du mouvement axial et pivotant (3) de la pince (2) comporte un trou (21) destiné au maintien d'une vis (19), comportant un filetage interne (22) et située sur le même axe que le trou (18) destiné au maintien de la vis (19) de la partie inférieure de la mâchoire (16), ainsi que **par le fait que** le vissage de la vis (19) produit un serrement mécanique des deux parties de la mâchoire, la partie supérieure (15) et la partie inférieure (16), causant l'immobilisation complète de la pince (2) et l'empêchant de coulisser le long de la barre (1), comme indiqué par la flèche A, et autour de la barre (1), comme indiqué par la flèche B.

7. Une unité télescopique d'orientation et de maintien pour une broche (9) ou une paire de broches (9, 10) ou un bras extensible (11), conforme aux revendications 1, 3 et 4, **caractérisée par le fait qu'**afin de permettre à l'utilisateur de positionner, durant l'opération chirurgicale, une nouvelle unité télescopique entre deux unités déjà placées, sans avoir à les retirer et à les réinstaller, le mécanisme de fixation du mouvement axial et pivotant (3) de la pince (2) peut être constitué de deux mâchoires indépendantes, la mâchoire supérieure (15) et la mâchoire inférieure (16), comprenant deux éléments indépendants, chacun d'entre eux comportant à son extrémité qui est diamétralement opposée à la fente (17) une partie jointe, (47) pour la mâchoire supérieure et (48) pour la mâchoire inférieure, respectivement, avec un trou central au centre de chaque partie, (49) et (50) respectivement, qui (49) et (50) sont coaxiaux l'un par rapport à l'autre, tandis qu'en leur centre passe un pivot (46) qui est fixé entre les parties jointes (47) et (48) par tout moyen connu de ceux qui sont versés dans cet art (par exemple, par calage, vissage, etc.), formant ainsi un joint qui permet la rotation de la mâchoire inférieure (16) autour du pivot (46), comme indiqué par la flèche F, ainsi que **par le fait que** le vissage de la vis (19) produit un serrement mécanique des deux parties de la mâchoire, la partie supérieure (15) et la partie inférieure (16), causant l'immobilisation complète de la pince (2) et l'empêchant de coulisser le long de la barre (1), comme indiqué par la flèche A, et autour de la barre (1), comme indiqué par la flèche B.

8. Une unité télescopique d'orientation et de maintien pour une broche (9) ou une paire de broches (9, 10) ou un bras extensible (11), conforme aux revendications 5, 6 ou 7, **caractérisée par le fait que**, les parties supérieure (15) et inférieure (16) de la mâchoire de la pince de serrage (2), ainsi que les deux mâchoires indépendantes, de la pince de serrage (2), comportent sur un point de leur surface interne un agent élastique de friction (14) ou (53) et (54), qui aide l'utilisateur, en empêchant temporairement un coulissage indésirable de la pince de serrage (2) le long de la barre (1) sur l'axe Y-Y', comme indiqué par la flèche A, le long de l'axe de la barre, et une rotation indésirable de la pince de serrage (2) autour de l'axe Y-Y', autour de l'axe de la barre, comme indiqué par la flèche B, jusqu'à ce que la position définitive et précise du positionnement de la pince (2) ait été sélectionnée par l'utilisateur au moyen du mécanisme de fixation du mouvement axial et pivotant (3).

9. Une unité télescopique d'orientation et de maintien pour une broche (9) ou une paire de broches (9, 10) ou un bras extensible (11), conforme à la revendication 1, **caractérisée par le fait que**, le mécanisme télescopique, constitué d'un mécanisme d'immobilisation axial (5), d'un régulateur de rotation (6) et d'un bras télescopique (7), est situé et se déplace dans un trou traversant ou ouverture (4) qui traverse la pince (2), dont la paroi, soit sur toute sa longueur soit partiellement, est traversée par une rainure (32), et **par le fait que** l'insertion de l'ensemble du mécanisme télescopique dans le trou traversant ou ouverture (4) de la pince (2) est possible par les deux entrées du trou traversant ou ouverture (4).

10. Une unité télescopique d'orientation et de maintien pour une broche (9) ou une paire de broches (9, 10) ou un bras extensible (11), conforme aux revendications 1 et 9, **caractérisée par le fait que**, le mécanisme d'immobilisation axiale (5) est constitué d'une vis (25) et de deux anneaux de serrage, l'un interne (26) et l'autre externe (27) et **par le fait qu'**il peut y avoir non pas une mais plusieurs paires d'anneaux de serrage interne (26) et externe (27), disposées en séquence, axialement et jointes par paires.

11. Une unité télescopique d'orientation et de maintien pour une broche (9) ou une paire de broches (9, 10) ou un bras extensible (11), conforme à la revendication 10, **caractérisée par le fait que**, les deux anneaux de serrage, l'anneau interne (26) et externe (27) du mécanisme d'immobilisation axial (5) comportent, quant à l'anneau de serrage interne (26) une conicité externe (28) se continuant sur toute sa longueur, avec un trou (43) pour le passage de la vis (25), tandis que de son côté, l'anneau de serrage externe (27) comporte une conicité interne (29) se continuant sur toute sa longueur, correspondant à la conicité externe (28) de l'anneau interne (26), assurant ainsi un contact complet d'au moins une partie de la surface conique externe (28) de l'anneau interne (26) avec au moins une partie de la surface conique interne (29) de l'anneau externe (27) grâce au placement coaxial de l'anneau de serrage interne (26) à l'intérieur de l'anneau de serrage externe (27), l'anneau de serrage externe (27) pouvant aussi être ouvert sur un point de son périmètre, par une fente (44).

12. Une unité télescopique d'orientation et de maintien pour une broche (9) ou une paire de broches (9, 10) ou un bras extensible (11), conforme aux revendications 10 ou 11, **caractérisée par le fait que**, lorsque la vis (25) est vissée à l'intérieur du filetage interne (38) du bras télescopique (7), sa tête (41) appuie sur l'anneau de serrage interne (26), forçant celui-ci à entrer dans l'anneau de serrage externe (27), et à cause de la forme conique inversée des deux surfaces (28) et (29), et grâce à la fente transversale (44), il se produit une augmentation du diamètre externe de l'anneau externe (27), faisant ainsi appuyer la surface externe incurvée (45) de l'anneau externe (27) sur la paroi interne du trou traversant (4) de la pince (2) et causant une immobilisation axiale complète le long de l'axe X-X' des deux anneaux de serrage du mécanisme d'immobilisation axiale (5) et par conséquent du régulateur de rotation (6) qui lui est contigu, puis du bras télescopique (7), par rapport à la pince (2).

13. Une unité télescopique d'orientation et de maintien pour une broche (9) ou une paire de broches (9, 10) ou un bras extensible (11), conforme à la revendication 12, **caractérisée par le fait que**, le régulateur de rotation (6) est de forme cylindrique et comporte en son centre un trou traversant (33), coaxial au trou traversant (43) de l'anneau de serrage interne (26), et **par le fait que** sur l'une de ses surfaces il est contigu au mécanisme d'immobilisation axial (5) tandis que sur son autre surface frontale, il comporte une face dentée radiale (34), dans le périmètre interne de laquelle figure une niche (35) dans le trou (33), destinée à un agent élastique (36), pour aider l'utilisateur, en empêchant temporairement l'engrenage complet des deux surfaces à face frontale dentée, (39) et (34), du bras télescopique (7) et du régulateur de rotation (6), respectivement, jusqu'à ce que l'utilisateur ait choisi la position finale de leur positionnement exact et définitif.

14. Une unité télescopique d'orientation et de maintien pour une broche (9) ou une paire de broches (9, 10) ou un bras extensible (11), conforme aux revendications 1, 9 et 13, **caractérisée par le fait que**, le régulateur de rotation (6) comporte sur une partie de la surface courbée une rainure (30), destinée à maintenir une clavette (31), qui lorsqu'elle est adjointe, sur une partie de sa surface, à l'intérieur de la rainure (30), est fixée à l'intérieur par tout moyen connu de ceux qui sont versés dans cet art (vissage, collage, calage, etc.) de façon à faire partie intégrale du régulateur de rotation (6) et à former avec lui un nouveau corps unifié, empêchant ainsi la rotation du régulateur de rotation (6) autour de l'axe X-X' du trou traversant ou ouverture de la pince (2).

15. Une unité télescopique d'orientation et de maintien pour une broche (9) ou une paire de broches (9, 10) ou un bras extensible (11), conforme aux revendications 1, 9, 13 et 14, **caractérisée par le fait que**, le régulateur de rotation (6), réuni à la clavette (31), constituant un nouveau corps unifié, sont contigus et capables de coulisser le long de leurs surfaces, le long du trou traversant ou ouverture (4) et de la rainure (32), qui traverse une partie de la longueur totale de la paroi du trou traversant ou trou (4) de la pince (2), ainsi que **par le fait qu'**il peut y avoir plus d'une clavette (31) et plus d'une rainure (32), leur nombre étant cependant toujours égal, de telle sorte qu'à chaque rainure (32) une clavette (31) est adjointe et coulisse et qu'à l'intérieur de chaque rainure (30) une clavette (31) est fixée.

16. Une unité télescopique d'orientation et de maintien pour une broche (9) ou une paire de broches (9, 10) ou un bras extensible (11), conforme aux revendications 1, 9, 13, 14, 15 et 16, **caractérisée par le fait que**, la rainure (30) du régulateur de rotation (6), peut avoir une section de n'importe quelle forme, par exemple circulaire, carrée, etc., toujours semblable à la section de la partie de la clavette (31) qui y est insérée et que, respectivement, la rainure (32) de la pince (2), qui contient l'autre partie de la clavette (31) peut avoir une section de n'importe quelle forme, par exemple circulaire, carrée, etc., toujours semblable à la section de la partie de la clavette (31) coulissant sur sa surface, de telle sorte que l'insertion d'une partie de la clavette (31) dans la rainure (32) empêche toujours tout mouvement pivotant de la totalité du nouvel élément unifié, constitué du régulateur de rotation (6) réuni à la clavette (31) autour de l'axe X-X' du trou traversant ou ouverture de la pince (2).

17. Une unité télescopique d'orientation et de maintien pour une broche (9) ou une paire de broches (9, 10) ou un bras extensible (11), conforme aux revendications 1, 9, 13, 14, 15 et 16, **caractérisée par le fait que**, le régulateur de rotation (6) et la clavette (31), peuvent constituer dès le départ un élément unifié, avec une section similaire à celle qui résulte de l'union de deux pièces, du régulateur de rotation (6) et de la clavette (31), ou semblable à celle résultant de l'union du régulateur de rotation (6) et d'un plus grand nombre de clavettes (31), ou similaire à la section particulière du vide créé par le trou traversant ou trou (4) et la rainure (32).

18. Une unité télescopique d'orientation et de maintien pour une broche (9) ou une paire de broches (9, 10) ou un bras extensible (11), conforme aux revendications 1, 9, 13 et 14, **caractérisée par le fait que**, la section du régulateur de rotation (6), ainsi que la section du trou traversant ou trou (4) de la pince (2), peut ne pas être circulaire mais polygonale, ou symétrique, etc., et que les sections du régulateur de rotation (6) et du trou traversant ou trou (4) correspondront toujours entre elles.

19. Une unité télescopique d'orientation et de maintien pour une broche (9) ou une paire de broches (9, 10) ou un bras extensible (11), conforme aux revendications 1, 9 et 13, **caractérisée par le fait que**, la rainure (30) du régulateur de rotation (6), qui contient une partie de la clavette (31), peut avoir un section trapézoïdale avec une surface de fond inclinée (55), de telle sorte que la profondeur d'insertion (59) de la rainure (30) soit supérieure à la profondeur de fond (60) de la rainure (30).

20. Une unité télescopique d'orientation et de maintien pour une broche (9) ou une paire de broches (9, 10) ou un bras extensible (11), conforme aux revendications 1, 9, 13 et 19, **caractérisée par le fait que**, la clavette (31) est de forme sphénoïdale, l'un de ses côtés étant incliné (61 ), suivant une inclination correspondant à celle de la surface de fond (55) de la rainure (30), l'autre côté étant plat (62), et que ses bases, (58) et (56) sont inégales, la plus longue (58) étant plus longue que ou égale à la section de l'ouverture (57) créée par la rainure (30) du régulateur de rotation (6) et la rainure (32) du trou traversant ou ouverture (4) de la pince (2), la plus courte (56) étant plus courte que la section de l'ouverture (57).

21. Une unité télescopique d'orientation et de maintien pour une broche (9) ou une paire de broches (9, 10) ou un bras extensible (11), conforme aux revendications 13, 14, 15, 16, 17, 18, 19 et 20, **caractérisée par le fait que**, lorsque la vis (25) est vissée à l'intérieur du filetage interne (38) du bras télescopique (7), cela provoque dans un premier stade le rapprochement de la face dentée radiale (39) et de la face dentée radiale (34), et que l'agent élastique (36), qui est situé entre ces deux éléments, subit une torsion, empêchant ainsi temporairement l'engrenage complet des deux faces dentées radiales, et qu'une fois que l'utilisateur a choisi l'angle adéquat de la position finale du bras télescopique (7), par rapport au régulateur de rotation (6) et à la pince (2), il intensifie le serrage de la vis (25), provoquant ainsi l'engrenage complet de la face dentée radiale (39) avec la face dentée radiale (34), tandis que, grâce à l'existence de la clé (31), l'engrenage complet de la face dentée radiale (34) avec la face dentée radiale (39) a pour résultat d'empêcher la rotation autour de l'axe X-X' du mécanisme d'immobilisation axiale (5), du régulateur de rotation (6) et du bras télescopique (7) avec lequel il est engrené, par rapport à la pince (2).

22. Une unité télescopique d'orientation et de maintien pour une broche (9) ou une paire de broches (9, 10) ou un bras extensible (11), conforme à la revendication 20, **caractérisée par le fait que**, le vissage de la vis (25) insère la clavette (31), la plus courte (56) des bases de celles-ci d'abord, dans l'ouverture (57) formée par la rainure (30) du régulateur de rotation (6) et la rainure (32) de l'ouverture traversante (4), et que le serrage de la vis (25) provoque le calage complet de la clavette (31) dans l'ouverture (57) et que la clavette (31), en raison des forces de friction qui se développent et de sa forme, provoque une fixation du mouvement axial et pivotant de chaque élément individuellement et, également, par rapport au reste, c'est-à-dire au régulateur de rotation (6) et au bras télescopique (7), par rapport à la pince (2).

23. Une unité télescopique d'orientation et de maintien pour une broche (9) ou une paire de broches (9, 10) ou un bras extensible (11), conforme aux revendications 1 et 9, **caractérisée par le fait que**, le régulateur de rotation (6) peut avoir extérieurement une forme cylindrique, et comporter en son centre une ouverture ou trou traversant (33), avec conicité (66), et être traversé sur toute la longueur de sa surface par une fente traversante longitudinale (63), et que le bras télescopique (7) est de forme cylindrique et comporte en son centre un trou non débouchant (37) pour l'insertion de la vis (25) avec filetage interne (38), coaxial avec le trou traversant (33) du régulateur de rotation (6) et que le bras télescopique (7) a une extrémité externe conique (64), correspondant à celle à conicité interne (66) du trou traversant (33) du régulateur de rotation (6), et qu'au sommet de l'extrémité conique (64) peut être placé un agent élastique (65), tel qu'un ressort, un anneau élastique, etc.

24. Une unité télescopique d'orientation et de maintien pour une broche (9) ou une paire de broches (9, 10) ou un bras extensible (11), conforme à la revendication 13, **caractérisée par le fait que**, le vissage de la vis (25) provoque, successivement, la torsion de l'agent élastique (65), la correspondance complète des surfaces coniques (64) et (66), l'insertion progressive de l'extrémité conique (64) à l'intérieur du trou à conicité interne (33) et en raison de la conicité inversée des deux surfaces (64) et (66), et grâce à le fente traversante (63), correspondant à la fente (44) de l'anneau (27), il se produit une augmentation du diamètre externe du régulateur de rotation (6), faisant ainsi appuyer sa surface externe incurvée sur la paroi interne du trou traversant (4) et, en combinaison avec l'existence de la clé (31) dans le régulateur de rotation (6), il se produit une immobilisation axiale et rotative complète le long et autour de l'axe X-X' du régulateur de rotation (6) et du bras télescopique (7), avec lequel il est engrené, entre eux et également par rapport à la pince (2).

25. Une unité télescopique d'orientation et de maintien pour une broche (9) ou une paire de broches (9, 10) ou un bras extensible (11), conforme aux revendications 1, 9, 13, 21, 22 et 24, **caractérisée par le fait que**, le bras télescopique (7), à l'une de ses extrémités, qui dépasse du trou traversant ou ouverture (4), comporte une face dentée radiale (67) et un mécanisme d'orientation et de maintien (8) d'une broche (9) ou même d'une seconde broche (10) ou même d'un bras extensible (11), tandis qu'à son autre extrémité, qui se trouve à l'intérieur du trou traversant ou ouverture (4), il est adjacent à un régulateur de rotation (6).

26. Une unité télescopique d'orientation et de maintien pour une broche (9) ou une paire de broches (9, 10) ou un bras extensible (11), conforme aux revendications 1, 13, 21, 22 , 24 ou 25, **caractérisée par le fait que**, le bras télescopique (7) est de forme cylindrique et comporte en son centre un trou non débouchant (37) destiné à l'insertion de la vis (25) avec filetage interne (38) et que le trou non débouchant (37) est coaxial au trou traversant (33) du régulateur de rotation (6).

27. Une unité télescopique d'orientation et de maintien pour une broche (9) ou une paire de broches (9, 10) ou un bras extensible (11), conforme aux revendications 1, 9, 21, 22, 25 et 26, **caractérisée par le fait que**, l'extrémité du bras télescopique (7), qui est adjacente au régulateur de rotation (6), comporte une face dentée radiale (39), sur le périmètre du trou (37), avec un nombre égal de dents et une géométrie correspondant à la face dentée radiale (34) du régulateur de rotation (6), et que le périmètre interne de la face dentée radiale (39) comporte une niche (40) dans le trou non débouchant (37) afin de recevoir un agent élastique (36), qui est sur l'un de ses côtés contigu à la surface de la niche (40) du trou (37) de la face dentée radiale (39) du bras télescopique (7), et sur son autre côté est contigu à la surface de la niche (35) du trou (33) de la face dentée radiale (34) du régulateur de rotation (6).

28. Une unité télescopique d'orientation et de maintien pour une broche (9) ou une paire de broches (9, 10) ou un bras extensible (11), conforme aux revendications 1, 9, 13, 14, 21, 22, 25, 26 et 27, **caractérisée par le fait que**, les dents de la face dentée radiale (34), ainsi que du celles du régulateur de rotation (6) et celles de la face dentée radiale (39) du bras télescopique (7) sont conçues de telle façon que les dents de chaque face dentée sont insérées dans et correspondent totalement aux dents de la face dentée radiale opposée sur toute la longueur de leur surface, empêchant ainsi le mouvement pivotant du bras télescopique (7), par rapport au régulateur de rotation (6) et à la pince (2).

29. Une unité télescopique d'orientation et de maintien pour une broche (9) ou une paire de broches (9, 10) ou un bras extensible (11), conforme aux revendications 1, 9 et 25, **caractérisée par le fait que**, le mécanisme d'orientation et de maintien (8) pour une broche (9) ou même un bras extensible (11), est constitué d'une vis (68), de deux mâchoires, la mâchoire supérieure (72) et la mâchoire inférieure (73), d'un ressort (74), placé entre les mâchoires, d'un anneau élastique (75) et d'un écrou de serrage (76), et que le mécanisme d'orientation et de maintien peut comporter un mécanisme de stabilisation temporaire de la broche (9).

30. Une unité télescopique d'orientation et de maintien pour une broche (9) ou une paire de broches (9, 10) ou un bras extensible (11), conforme aux revendications 1, 9, 25, 29, **caractérisée par le fait que**, la face dentée radiale (67) du bras télescopique (7) est sur un plan formant un angle avec le plan de la face dentée radiale (39) située à l'autre extrémité du bras télescopique (7), à savoir celle qui se trouve à l'intérieur du trou traversant ou ouverture (4) de la pince (2) et que sous la face dentée radiale (67) se trouve un trou (128) avec un point rétrécissant destiné au maintien du mécanisme de stabilisation temporaire.

31. Une unité télescopique d'orientation et de maintien pour une broche (9) ou une paire de broches (9, 10) ou un bras extensible (11), conforme aux revendications 1 et 30, **caractérisée par le fait que**, la vis (68) passe dans un trou traversant (98) qui traverse le centre de la face dentée radiale (67) du bras télescopique (7) et comporte un filetage (107), et sur une partie de sa tête (69) peut comporter une cannelure périmétrique (70), dans laquelle est placé un anneau élastique (71), tandis que sur une partie de son corps située avant le filetage elle peut comporter une cannelure périmétrique (127) destinée à la stabilisation temporaire de la vis (68), tandis qu'à son extrémité, elle peut comporter un limiteur de dévissage (79), tel qu'une sécurité, une petite vis, etc., qui est placée à l'extrémité (106) de la vis (68) d'une manière adéquate, par exemple par vissage ou collage, etc.

32. Une unité télescopique d'orientation et de maintien pour une broche (9) ou une paire de broches (9, 10) ou un bras extensible (11), conforme aux revendications 30 et 31, **caractérisée par le fait que**, la mâchoire supérieure (72) comporte sur une partie de sa surface un trou traversant (80), pouvant comporter à son point d'ouverture une niche (82) destinée à la tête (69) de la vis (68), et que son côté inférieur (84) comporte une encoche (86) destinée à une broche (9) et une niche (88) et peut aussi comporter une niche (85) située dans le trou (80) et destinée au ressort (74) dont l'utilisation provoque l'écartement des deux mâchoires, (72) et (73) afin de faciliter le positionnement de la broche (9) entre les encoches (86) et (91) des mâchoires supérieure et inférieure, respectivement.

33. Une unité télescopique d'orientation et de maintien pour une broche (9) ou une paire de broches (9, 10) ou un bras extensible (11), conforme aux revendications 31 ou 32, **caractérisée par le fait que**, la mâchoire inférieure (73) comporte sur son côté supérieur (89) une encoche (91) d'une broche (9), en face de l'encoche (86) de la mâchoire supérieure (72) et à un certain point de sa surface un trou traversant (81), et que sur son côté supérieur (89) elle peut comporter une niche (90) située dans le trou (81), destinée au ressort (74), tandis que sur son côté inférieur, elle peut comporter une niche (97), dans laquelle est placé l'agent élastique (75) par lequel est temporairement empêché l'engrenage complet des deux faces dentées radiales, (67) et (96), tandis que durant le serrage de la vis (68), l'agent élastique (75) subit une torsion afin de réaliser un contact complet des deux faces dentées radiales.

34. Une unité télescopique d'orientation et de maintien pour une broche (9) ou une paire de broches (9, 10) ou un bras extensible (11), conforme aux revendications 30, 31, 32 ou 33, **caractérisée par le fait que**, la mâchoire inférieure (73) comporte sur son côté supérieur (89) une projection (93), correspondant à la niche (88) que comporte la mâchoire supérieure (72) sur son côté inférieur (84), et que la hauteur de la projection (93) et la profondeur de la niche (88) peuvent être telles entre le côté inférieur (84) de la mâchoire supérieure (72) et le côté supérieur (89) de la mâchoire inférieure (73) qu'un écart (94) est créé, et que la niche (88) et la projection (93) peuvent être dans une position inverse, sur la mâchoire inférieure et mâchoire supérieure respectivement.

35. Une unité télescopique d'orientation et de maintien pour une broche (9) ou une paire de broches (9, 10) ou un bras extensible (11), conforme aux revendications 30, 31, 32, 33 ou 34, **caractérisée par le fait que**, la mâchoire inférieure (73) comporte sur son côté inférieur (95) une face dentée radiale (96) sur le périmètre du trou (81 ), avec un nombre égal de dents et une géométrie correspondante à la face dentée radiale (67) du bras télescopique (7), et que les dents de la face dentée radiale (96) de la mâchoire inférieure (73) comme celles de la face dentée radiale (67) du bras télescopique (7) sont conçues de telle sorte que les dents de chaque face dentée radiale sont insérées dans et correspondent totalement aux dents de la face dentée radiale opposée, sur toute la longueur de leurs surfaces.

36. Une unité télescopique d'orientation et de maintien pour une broche (9) ou une paire de broches (9, 10) ou un bras extensible (11), conforme aux revendications 30 ou 35, **caractérisée par le fait que**, l'écrou de serrage (76) comporte une tête (101) qui à son extrémité inférieure comporte un support ou prise (102), adapté à l'outil de vissage et de dévissage ou même à un premier serrage à la main, ainsi qu'une cannelure périmétrique (77), dans laquelle est placée un anneau élastique (78), grâce auquel la rotation libre de l'écrou de serrage (76) est temporairement empêchée, en raison de la friction générée entre l'anneau élastique (78) et la paroi interne de la surface de la niche (100) du trou (98), et qu'au centre de l'écrou de serrage (76) se trouve un trou traversant (103) qui traverse l'écrou de serrage (76) sur toute sa longueur, tandis que d'un point du trou traversant (103) part un filetage interne (104) qui s'étend jusqu'à l'extrémité du trou traversant (103) et dont le diamètre est plus court que celui du limiteur de dévissage (79) de façon à empêcher son passage et par conséquent le passage de la vis (68), hors de l'écrou (76).

37. Une unité télescopique d'orientation et de maintien pour une broche (9) ou une paire de broches (9, 10) ou un bras extensible (11), conforme à la revendication 29, **caractérisée par le fait que**, le mécanisme de stabilisation temporaire est constitué d'une pointe (124), pouvant comporter une partie qui s'élargit (129), tandis que l'extrémité qui s'insère dans le trou (128) a une configuration conique ou sphénoïdale ou tout autre type de configuration incurvée (130), correspondant à celle de la rainure (127) dans laquelle elle s'insère lorsque la vis (68) est dans une position adéquate, d'un moyen de pression élastique (125) tel qu'un ressort, une sphère élastique, etc., qui exerce une pression permanente sur la pointe (124), qui tend à glisser vers la vis, et d'une pièce d'arrêt (126), qui se trouve en position fixe dès le départ grâce à tout moyen connu de ceux qui sont versés dans cet art (par exemple, par vissage, calage, etc.), et que la pointe (124), le moyen de pression élastique (125) et la pièce d'arrêt (126) sont placés dans le trou (128) du bras télescopique (7).

38. Une unité télescopique d'orientation et de maintien pour une broche (9) ou une paire de broches (9, 10) ou un bras extensible (11), conforme à la revendication 37, **caractérisée par le fait que**, lorsque l'utilisateur a placé la broche (9) dans l'os à réparer, afin de le placer dans l'unité télescopique, il l'ajuste entre l'encoche (91) et l'encoche (86), et que si dans le système a également été placé un mécanisme temporaire de stabilisation de la broche (9), l'utilisateur peut obtenir, à ce stade, une immobilisation temporaire de la broche (9), simplement en exerçant une pression sur la vis (68), de telle sorte que la pointe (124) soit insérée dans la rainure (127) de la vis (68), tandis que la broche (9) peut être décrochée du mécanisme de maintien (8) en exerçant une force inverse.

39. Une unité télescopique d'orientation et de maintien pour une broche (9) ou une paire de broches (9, 10) ou un bras extensible (11), conforme aux revendications 36, 37 ou 38, **caractérisée par le fait que**, pour obtenir la maintien de la broche (9) par l'unité télescopique, l'utilisateur sélectionne et serre soit la vis (68) dans l'écrou de serrage (76) soit l'inverse - étant donné que l'unité, grâce aux anneaux élastiques que comporte l'écrou (76) et la vis (68), permet à l'utilisateur de choisir de ne visser que l'un des deux éléments auxquels il a accès, sans être en même temps obligé d'immobiliser également l'autre, avec le même résultat - provoquant ainsi la torsion du ressort (74) et donc le rapprochement des deux mâchoires, la mâchoire supérieure (72) et la mâchoire inférieure (73), et que lorsque l'utilisateur a sélectionné l'angle adéquat de la position finale du mécanisme (8), par rapport au bras télescopique (7) et à la pince (2), il intensifie le serrage de la vis (68) contre l'écrou de serrage (76), causant ainsi progressivement l'engrenage complet des deux faces dentées radiales, (67) et (96), et en même temps, le serrage complet des deux mâchoires (71) et (73), non seulement autour de la broche (9) et entre elles mais aussi contre le bras télescopique (7), obtenant ainsi l'immobilisation complète de la broche (9), par rapport au bras télescopique (7) et donc à la pince (2).

40. Une unité télescopique d'orientation et de maintien pour une broche (9) ou une paire de broches (9, 10) ou un bras extensible (11), conforme aux revendications 1, 9, 25, 29, 30, 31, 32, 33, 34, 35, 36 et 37, **caractérisée par le fait que**, la longueur du bras télescopique (7) peut être augmentée en y ajustant l'élément de bras extensible (11), qui comporte à ses deux extrémités des surfaces, (108) et (109), avec une face dentée radiale, sur un plan tel que l'axe du bras est contenu dans le plan des surfaces, qui peuvent avoir une orientation inverse l'une par rapport à l'autre, une orientation similaire ou de tout autre type.

41. Une unité télescopique d'orientation et de maintien pour une broche (9) ou une paire de broches (9, 10) ou un bras extensible (11), conforme à la revendication 40, **caractérisée par le fait que**, les deux faces dentées radiales, (108) et (109), du bras extensible (11) comportent en leur centre des trous traversants (110) et (111) respectivement, et une niche chacun, (112) et (113), pour deux anneaux élastiques (75) et (114) respectivement, et qu'à la sortie des trous traversants, (110) et (111), se trouve une niche (131) et (115) respectivement, destinée à contenir la tête, (101) et (101), des deux écrous de serrage (76) et (76), ainsi que **par le fait que** l'utilisation des deux anneaux élastiques (75) et (114), jusqu'à ce que l'utilisateur serre complètement deux vis (68), empêche temporairement l'engrenage complet des surfaces des faces dentées radiales, (67) et (108), ainsi que des deux faces dentées radiales (109) et (96).

42. Une unité télescopique d'orientation et de maintien pour une broche (9) ou une paire de broches (9, 10) ou un bras extensible (11), conforme aux revendications 40 ou 41, **caractérisée par le fait que**, pour adapter le bras télescopique (11) sur le bras télescopique (7), la face dentée radiale (108) du bras extensible (11) s'engrène avec la face dentée radiale (67) du bras télescopique (11), en utilisant de la même façon les mêmes pièces que celle qui sont utilisées pour fixer le mécanisme de maintien et d'orientation (8) d'une broche (9) sur le bras télescopique (7), à savoir une vis (68) à filetage (107) et cannelure périmétrique (70), un anneau élastique (71 ), un écrou de serrage (76), avec anneau élastique (78) et un mécanisme de stabilisation temporaire.

43. Une unité télescopique d'orientation et de maintien pour une broche (9) ou une paire de broches (9, 10) ou un bras extensible (11), conforme aux revendications 40, 41 ou 42, **caractérisée par le fait que**, lorsque le bras extensible (11) est fixé sur le bras télescopique (7), le mécanisme de maintien et d'orientation (8) d'une broche (9) est fixé sur la seconde face dentée radiale (109) du bras extensible (11), de sorte que durant l'utilisation du bras extensible (11) l'immobilisation de la broche (9), par rapport à l'élément du bras extensible (11) et donc par rapport à la pince (2), est obtenue au moyen du mécanisme de maintien (8), exactement de la façon dont la broche (9) est immobilisée lorsque le bras extensible (11) n'est pas utilisé, et l'immobilisation du bras extensible (11), par rapport au bras télescopique (7) et donc par rapport à la pince (2) est obtenue exactement de la façon dont le mécanisme d'orientation et de maintien (8) est immobilisé par rapport au bras télescopique (7).

44. Une unité télescopique d'orientation et de maintien pour une broche (9) ou une paire de broches (9, 10) ou un bras extensible (11), conforme aux revendications 1, 9, 25, 29, 30, 31, 32, 33, 34, 35, 36, 37, 40, 41, 42 et 43, **caractérisée par le fait que**, l'extrémité du bras télescopique (7) qui s'étend hors du trou traversant ou ouverture (4) et porte le mécanisme d'orientation et de maintien d'une broche (9) ou même d'un bras extensible (11), peut comporter une seconde face dentée radiale (116) sur un plan parallèle à la première face dentée radiale (67) de façon à avoir une paire identique de mâchoires d'orientation et de maintien (120) d'une seconde broche (10), constituée des même éléments que la première paire de mâchoires d'orientation et de maintien (8) d'une broche (9).

45. Une unité télescopique d'orientation et de maintien pour une broche (9) ou une paire de broches (9, 10) ou un bras extensible (11), conforme à la revendication 44, **caractérisée par le fait que**, le placement de la seconde paire de mâchoires d'orientation et de maintien (120) d'une seconde broche (10) est effectué sur la seconde face dentée radiale (116) exactement de la façon dont la première paire de mâchoires d'orientation et de maintien (8) de la première broche (9) est placée sur la première face dentée radiale (67).

46. Une unité télescopique d'orientation et de maintien pour une broche (9) ou une paire de broches (9, 10) ou un bras extensible (11), conforme aux revendications 1, 44 ou 45, **caractérisée par le fait que**, pour obtenir le maintien des deux broches, (9) et (10), par les deux mécanismes de maintien, (8) et (120) respectivement, une fois que l'utilisateur a implanté les deux broches, (9) et (10), dans l'os à réparer, afin de les placer dans l'unité télescopique, il dispose chacune d'elles entre les encoches (91) et (86) du premier (121) et du second (122) mécanisme de maintien, et que l'utilisateur sélectionne et serre la vis (68) dans l'écrou de serrage (76) ou, inversement, l'écrou de serrage (76) autour de la vis (68) - étant donné que l'unité, grâce aux anneaux élastiques que comporte la vis (68) et l'écrou (76), permet à l'utilisateur de choisir de visser l'une des deux pièces auxquelles il a accès, sans être en même temps obligé d'immobiliser l'autre, avec le même résultat - provoquant ainsi la torsion simultanée des deux ressorts (74) et (117) des deux mécanismes de maintien, (8) et (120) respectivement, et par conséquent le rapprochement des deux mâchoires, la mâchoire supérieure (72) et la mâchoire inférieure (73), du premier mécanisme (8) et le rapprochement simultané des deux mâchoires, la mâchoire supérieure (118) et la mâchoire inférieure (119), du second mécanisme (120), non seulement l'une par rapport à l'autre, mais aussi par rapport au bras télescopique (7), serrant ainsi non seulement la première broche (9) entre les encoches (86) et (91) du premier mécanisme (8) mais aussi la seconde broche (10) entre les encoches (121) et (122) du second mécanisme (120).

47. Une unité télescopique d'orientation et de maintien pour une broche (9) ou une paire de broches (9, 10) ou un bras extensible (11), conforme aux revendications 1, 44, 45 ou 46, **caractérisée par le fait que**, pour obtenir le maintien complet des deux broches, (9) et (10), par les deux mécanismes de maintien, (8) et (120), comme l'utilisateur intensifie le serrage de la vis (68) dans l'écrou de serrage (76), les deux anneaux élastiques (75) et (92), situés de chaque côté du bras télescopique (7) et placés entre celui-ci et les deux mécanismes de maintien, (8) et (120) respectivement, subissent une torsion, empêchant temporairement l'engrenage complet des deux paires de faces dentées radiales, (67) et (96), du premier mécanisme (8), et (116) et (123) du second mécanisme (120), et que lorsque l'utilisateur a sélectionné l'angle adéquat de la position finale des deux mécanismes (8) et (120) par rapport au bras télescopique (7), il intensifie le serrage de la vis (68) dans l'écrou (76), et sous l'effet de la pression exercée par le resserrement de l'assemblage de la vis (68) contre l'écrou (76) de la première paire de mâchoires (72) et (73) celle-ci est resserrée, ainsi qu'en même temps la seconde paire de mâchoires, (118) et (119), non seulement l'une par rapport à l'autre, mais aussi contre le bras télescopique (7), causant ainsi l'engrenage complet des deux paires de faces dentées radiales, (96) et (67), d'une part, et (123) et (116) de l'autre. Le resserrement des quatre mâchoires contre le bras télescopique (7) a pour résultat une immobilisation complète, non seulement des deux paires de mâchoires mais aussi des deux broches, (9) et (10), par rapport au bras télescopique (7) et à la pince (2).

48. Une unité télescopique d'orientation et de maintien pour une broche (9) ou une paire de broches (9, 10) ou un bras extensible (11), conforme aux revendications 1, 44 et 45, **caractérisée par le fait que**, à l'extrémité du bras télescopique (7) qui s'étend hors du trou traversant (4) et à laquelle sont adjoints les deux mécanismes d'orientation d'une broche, peut être fixé et assuré de la même manière, sur l'un des côtés (67) du bras télescopique (7), un mécanisme d'orientation et de maintien (8) d'une broche (9), et de l'autre côté (116) du bras télescopique (7) peut être fixé un bras extensible (11), ou inversement.

49. Une unité télescopique d'orientation et de maintien pour une broche (9) ou une paire de broches (9, 10) ou un bras extensible (11), conforme aux revendications 32, 33, 34, 35, 36 ou 39, 40, 41, 42, 43, 44, 45, 46, 47, 48, **caractérisée par le fait que**, dans tous les modes d'application de la présente invention, les encoches (86) et (91) peuvent être de section circulaire ou triangulaire, ceci pouvant s'appliquer à l'une d'entre elle, ou à plusieurs, ou à toutes, cette variation permettant à l'utilisateur de placer des broches de divers diamètres, et que dans le cas où seule l'une d'entre elles est de section triangulaire, la surface de pression (87) de la tête (69) de la vis (68) peut être partiellement sphérique et la surface de la niche (82) doit avoir une forme correspondante afin d'obtenir une charge uniforme de la surface de pression (87) de la tête (69) de la vis (68), dans n'importe quelle position angulaire possible de la mâchoire supérieure (72) par rapport à la mâchoire inférieure (73).

50. Un système de fixation externe, conforme à la revendication 2, **caractérisé par le fait que**, dans le cas où le système comprend également une deuxième barre (132), celle-ci comporte également deux unités télescopique d'orientation ou de maintien (8) ou davantage pour une broche (9) ou une paire de broches (9, 10) ou un bras extensible (11), et peut être connectée à la première barre (1) par le couplage direct des deux faces dentées radiales de deux bras télescopiques (7) ou davantage par paires, c'est-à-dire, l'une de celle de la première barre (1) avec à chaque fois l'une de celle de la seconde barre (132), ce qui permet d'obtenir une stabilité maximale du système.

51. Un système de fixation externe, conforme aux revendications 2 ou 50, **caractérisé par le fait que**, pour la connexion directe de chaque paire de bras télescopiques (7), à savoir, de l'un de ceux de la première barre (1) avec l'un de ceux de la seconde barre (132), la face dentée radiale (67), de chaque bras télescopique (7) située à son extrémité, est fixée au moyen d'une vis (68), directement contre la face dentée radiale (67) de l'autre bras télescopique (7), ce qui provoque également la torsion d'un agent élastique (75), susceptible d'intervenir entre les deux faces dentées radiales (67) des deux bras télescopiques (7), contigus sur son côté à la surface de la niche (99) dans le trou (98) comporté par la face dentée radiale (67) de chaque bras télescopique (7).

52. Un système de fixation externe, conforme aux revendications 2 ou 50, **caractérisé par le fait que**, le système peut comprendre une seconde barre (132), qui peut aussi comporter deux unités télescopiques d'orientation et de maintien ou davantage pour une broche (9) ou une paire de broches (9, 10) ou un bras extensible (11), dont la connexion à la première barre (1) peut être réalisée au moyen d'un bras extensible (11), doté d'un ou de plusieurs bras télescopiques (7), dont l'une des extrémités extensibles est connectée au bras télescopique (7) et l'autre extrémité extensible est connectée au bras télescopique (7) d'une autre unité télescopique placée sur la seconde barre (132), ce qui permet d'obtenir une stabilité maximale du système.

53. Un système de fixation externe, conforme aux revendications 2, 50 ou 52, **caractérisé par le fait que**, la connexion des deux barres peut être réalisée au moyen d'un bras télescopique (7) qui comporte à son extrémité, outre la première face dentée radiale (67), une seconde face dentée radiale (116), attachant un mécanisme d'orientation et de maintien (8) d'une broche (9) à une face dentée radiale et un bras extensible (11) à l'autre face dentée radiale, d'une façon similaire à celle qui est utilisée pour placer à l'extrémité du bras télescopique (7) deux mécanismes identiques d'orientation et de maintien (8) d'une broche (9).

54. Un système de fixation externe, conforme aux revendications 2, 50 ou 53, **caractérisé par le fait que**, au cas où à l'extrémité du bras télescopique (7) sont attachés un mécanisme (8) et un bras extensible (11), l'extrémité du bras extensible (11) peut comporter un mécanisme de maintien (8) avec une broche (9) ou peut être adjointe à l'extrémité du bras télescopique (7) une unité télescopique d'une seconde barre (132), causant ainsi, de cette manière également, la connexion des deux barres, ce qui permet d'obtenir une stabilité maximale du système.

55. Un système de fixation externe, conforme aux revendications 2, ou 50, 51, 52, 53, 54, **caractérisé par le fait que**, dans le cas où le système ne comporte qu'une barre (1), peuvent y être attachées deux unités télescopiques ou davantage, portant une broche (9) chacune, ou peuvent y être attachées deux unités ou davantage, portant une extension (11), qui porte à son tour un mécanisme de maintien (8) d'une broche (9), ou peuvent y être attachées deux unités ou davantage, portant chacune deux broches, (9) et (10), ou peuvent y être attachées deux unités ou davantage d'une broche (9) et une extension qui, à son tour, porte un mécanisme de maintien d'une broche supplémentaire, rendant ainsi le système plus flexible et ajustable aux besoins de l'utilisateur.
